# EUROPÄISCHE PATENTANMELDUNG

(11) **EP 0 600 830 A1**
(43) Veröffentlichungstag der Anmeldung: **08.06.1994**
(21) Anmeldenummer: 93810800.8
(22) Anmeldetag: 18.11.1993
(51) Int. Cl.: C07D 487/04, A61K 31/40, C07D 209/48, C07D 241/42, C07C 229/62, C07C 235/18, C07D 307/89

(54) **Substituierte Derivate von Diaminophthalimid als Protein-Tyrosin-Kinase-Hemmer**

(30) Priorität: 27.11.1992 CH 3649/92
(71) Anmelder: CIBA-GEIGY AG, CH-4002 Basel (CH)
(72) Erfinder: Trinks, Uwe, Dr., CH-4142 Münchenstein (CH); Traxler, Peter, Dr., CH-4124 Schönenbuch (CH)

(57) **Zusammenfassung**

Beschrieben werden Verbindungen der Formel I, 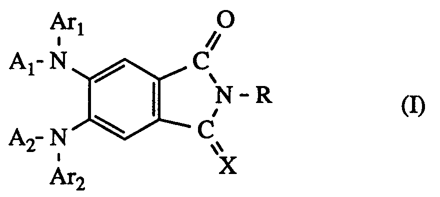
worin A₁ und A₂ unabhängig voneinander substituiertes Niederalkyl, substituiertes Niederalkenyl, substituiertes Niederalkinyl oder Wasserstoff bedeuten, wobei maximal einer der Reste A₁ und A₂ Wasserstoff bedeuten kann, oder A₁ und A₂ zusammen durch Substituenten ausser Niederalkyl und ausser Hydroxy substituiertes Niederalkylen bedeuten; Ar₁ und Ar₂ unabhängig voneinander Aryl, Heteroaryl oder unsubstituiertes oder substituiertes Cycloalkyl bedeuten, die Gruppe -C(=X)- für -C(=O)-, -C(=S)-, -CH₂-oder -C(=CR₁R₂)- steht, wobei R₁ und R₂ unabhängig voneinander Wasserstoff oder Niederalkyl bedeuten, und R Wasserstoff, Niederalkyl, Arylniederalkyl, Aryl, Amino, Hydroxy oder Niederalkoxy bedeutet, Salze davon, sofern salzbildende Gruppen vorliegen, und/oder Tautomere davon, sofern tautomerisierbare Reste vorliegen, Verfahren zur Herstellung dieser Verbindungen, pharmazeutische Präparate, die diese Verbindungen enthalten, und die Verwendung dieser Verbindungen zur therapeutischen Behandlung des menschlichen oder tierischen Körpers oder zur Herstellung pharmazeutischer Präparate.

## Beschreibung

Die Erfindung betrifft Verbindungen der Formel I, 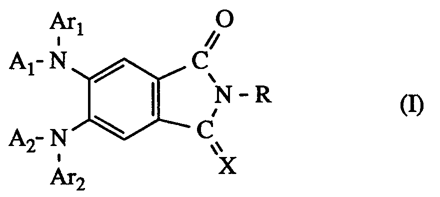
worin A₁ und A₂ unabhängig voneinander substituiertes Niederalkyl, substituiertes Niederalkenyl, substituiertes Niederalkinyl oder Wasserstoff bedeuten, wobei maximal einer der Reste A₁ und A₂ Wasserstoff bedeuten kann, oder A₁ und A₂ zusammen durch Substituenten ausser Niederalkyl und ausser Hydroxy substituiertes Niederalkylen bedeuten; Ar₁ und Ar₂ unabhängig voneinander Aryl, Heteroaryl oder unsubstituiertes oder substituiertes Cycloalkyl bedeuten, die Gruppe -C(=X)- für -C(=O)-, -C(=S)-, -CH₂- oder -C(=CR₁R₂)- steht, wobei R₁ und R₂ unabhängig voneinander Wasserstoff oder Niederalkyl bedeuten, und R Wasserstoff, Niederalkyl, Arylniederalkyl, Aryl, Amino, Hydroxy oder Niederalkoxy bedeutet, Salze davon, sofern salzbildende Gruppen vorliegen, und/oder Tautomere davon, sofern tautomerisierbare Reste vorliegen, Verfahren zur Herstellung dieser Verbindungen, pharmazeutische Präparate, die diese Verbindungen enthalten, und die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7, insbesondere bis und mit 4, und vor allen Dingen mit 1 bis 3 Kohlenstoffatomen, sofern nichts anderes angegeben ist.

Niederalkyl ist vorzugsweise n-Propyl, Isopropyl, n-Butyl, Isobutyl, sek.-Butyl, tert.-Butyl, n-Pentyl, Neopentyl, n-Hexyl oder n-Heptyl, vorzugsweise Methyl, Ethyl oder n-Propyl.

Niederalkenyl hat 2 bis 7, vorzugsweise 3 bis 7, insbesondere 3 oder 4 Kohlenstoffatome, und ist z. B. Allyl oder Crotyl.

Niederalkinyl hat 2 bis 7, vorzugsweise 3 bis 7, insbesondere 3 oder 4 Kohlenstoffatome, und ist z. B. Propin-l- oder Propin-2-yl oder 2-Butin-l-yl.

Substituiertes Niederalkyl ist vorzugsweise wie oben definiertes Niederalkyl, welches durch bis zu 4, vorzugsweise bis zu 2 Reste ausgewählt aus Amino, wie in Aminomethyl, -ethyl, -propyl oder -butyl, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, wie Aminomethyl, Aminoethyl, Aminopropyl oder Aminobutyl, Cycloalkylamino, Phenylniederalkylamino oder Phenylamino, Acylamino, z.B. Niederalkanoylamino, wie Acetylamino, Phenylniederalkanoylamino oder Phenylcarbonylamino (≙ Benzoylamino), Hydroxy, wie in Hydroxymethyl, Hydroxyethyl oder Hydroxypropyl, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkoxy, Acyloxy, insbesondere Niederalkanoyloxy, Mercapto, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkylthio, Acylthio, insbesondere Niederalkanoylthio, Carboxy, wie in Carboxymethyl, -ethyl oder -propyl, verestertes Carboxy, z.B. Niederalkoxycarbonyl, wie Methoxycarbonyl, Ethoxycarbonyl oder tert-Butoxycarbonyl, vor allem in Methoxy- oder Ethoxycarbonyl-methyl, -ethyl oder -proypl, oder Phenylniederalkoxycarbonyl, wie Benzyloxycarbonyl, Cyano, Carbamoyl, wie in Carbamoylmethyl, Carbamoylethyl oder Carbamoylpropyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, insbesondere 1- oder 3-Mono-, 1,3- oder 3,3-Di- oder 1,3,3-Triniederalkylureido, 1- oder 3-Phenylureido, 1- oder 3-Mono-, 1,3- oder 3,3-Di- oder 1,3,3-Triphenylniederalkylureido, z. B. 3-Niederalkylureido, wie 3-Methyl- oder 3-Ethylureido, vor allem in **Ureido-**, 3-Methyl- oder 3-Ethylureido-methyl, -ethyl oder -propyl, Thioureido, an einem oder beiden Stickstoffatomen durch Niederalkyl, Aryl oder Arylniederalkyl substituiertes Thioureido, insbesondere 1- oder 3-Mono-, 1,3- oder 3,3-Di- oder 1,3,3-Triniederalkylthioureido, 1- oder 3-Phenylthioureido, 1- oder 3-Mono-, 1,3- oder 3,3-Di- oder 1,3,3-Triphenylniederalkylthioureido, z.B. 3-Niederalkylthioureido, wie 3-Methyl- oder 3-Ethylthioureido, vor allem in **Thioureido-**, 3-Methyl- oder 3-Ethyl-thioureido-methyl, -ethyl oder -propyl, Hydrazino, an einem oder beiden Stickstoffatomen durch Niederalkyl, Aryl oder Arylniederalkyl substituiertes Hydrazino, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, Amidino, wie in Amidinomethyl, -ethyl oder -propyl, an einem oder beiden Stickstoffatomen durch Niederalkyl, Aryl oder Arylniederalkyl substituiertes Amidino, insbesondere N¹- oder N²-Mono-, N¹,N²- oder N¹,N¹-Di- oder N¹,N¹,N²-Triniederalkylamidino, N¹- oder N²-Phenylamidino, N¹- oder N²-Mono-, N¹,N²-, N¹,N¹-Di- oder N¹,N¹,N²-Triphenylniederalkylamidino, z. B. N¹,N¹-Diniederalkylamidino, wie N¹,N¹-Dimethyl- oder N¹,N¹-Diethylamidino, Guanidino, wie in Guanidinomethyl, -ethyl oder -propyl, an einem, zwei oder allen drei Stickstoffatomen durch Niederalkyl, Aryl oder Arylniederalkyl substituiertes Guanidino, insbesondere 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3-Pentaniederalkyl- oder -Phenylniederalkylguanidino, insbesondere 3,3-Diniederalkylguanidino, wie 3,3-Dimethylguanidino oder 3,3-Diethylguanidino, Oxo, welches nicht am Kohlenstoff gebunden ist, welcher an den A₁ oder A₂ tragenden Stickstoff gebunden ist, wie in 2-Oxopropyl oder 3-Oxo-n-butyl, Thioxo, Imino, Niederalkylimino, Acylimino, insbesondere Niederalkanoylimino, wie Acetylimino, Hydroxyimino (HO-N=), wie in Hydroxyiminomethyl (HO-N=CH-), Hydroxyimino-ethyl oder -propyl, Niederalkoxyimino, wie Methoxyimino, Hydrazono, wie in Hydrazonomethyl, -ethyl oder -propyl, N-Mono- oder N,N-Diniederalkylhydrazono, N-Acylhydrazono, insbesondere N-Niederalkanoylhydrazono, wie Acetylhydrazono oder Niederalkoxycarbonylhydrazono, wie tert-Butoxycarbonylhydrazono, Niederalkylthioimino, wie Methyl- oder Ethylthioimino, vor allem in Methylthioimino- oder Ethylthioimino-methyl, -ethyl oder -propyl, und Heterocyclyl, worin Heterocyclyl wie unten für Heterocyclylniederalkyl definiert ist, substituiert ist. Vorzugsweise ist Niederalkyl linear und endständig substituiert mit einem der genannten Substituenten.

Substituiertes Niederalkenyl ist vorzugsweise wie oben definiertes Niederalkenyl, insbesondere mit 3 bis 7, vor allem 3 oder 4 Kohlenstoffatomen, welches durch bis zu 4 Reste, vorzugsweise einen Rest ausgewählt aus den bei der Definition von substituiertem Niederalkyl genannten Substituenten substituiert ist. Bei bestimmten Substituenten sind durch Wechselwirkung mit der Doppelbindung Tautomere möglich. So können Hydroxy, Mercapto oder N-gebundene Substituenten, die noch einen freien Wasserstoff am bindenden Stickstoff haben, bei Bindung an ein Kohlenstoffatom einer Dreifachbindung tautomerisieren zu Oxo-, Thioxo- oder Iminoverbindungen; mit einer Doppelbindung über Stickstoff gebundene Substituenten wie Hydroxyimino oder Hydrazono können bei Vorliegen in Konjugation zu einer Doppelbindung im Niederalkenylrest ebenfalls tautomerisieren; derartige Verbindungen können auch in Tautomeriegleichgewichten vorliegen. Bevorzugt als substituiertes Niederalkenyl sind solche Reste, bei denen keine Tautomerie auftritt, d. h. wo z.B. Hydroxy oder N-gebundene Substituenten, die noch einen freien Wasserstoff am bindenden Stickstoff haben, nicht an ein Kohlenstoffatom im Niederalkenylrest gebunden sind, von dem eine Doppelbindung ausgeht, und/oder wo mit einer Doppelbindung über Stickstoff gebundene Substituenten wie Hydroxyimino oder Hydrazono nicht in Konjugation mit einer Doppelbindung im Niederalkenylrest stehen. Bevorzugt gegenüber substituiertem Niederalkenyl ist unsubstituiertes Niederalkenyl A₁ und/oder A₂, wie 3-Allyl.

Substituiertes Niederalkinyl ist vorzugsweise wie oben definiertes Niederalkinyl, insbesondere mit 3 bis 7, vor allem 3 oder 4 Kohlenstoffatomen, welches durch bis zu 4 Reste, vorzugsweise einen Rest ausgewählt aus den bei der Definition von substituiertem Niederalkyl genannten Substituenten substituiert ist. Analog der Definition von Tautomeren und Tautomeriegleichgewichten für substituiertes Niederalkenyl können auch für substituiertes Niederalkinyl entsprechende Tautomere und Tautomeriegleichgewichte vorliegen. Bevorzugt gegenüber substituiertem Niederalkinyl ist unsubstituiertes Niederalkinyl A₁ und/oder A₂.

Heterocyclylniederalkyl ist einer der oben genannten Niederalkylreste, vorzugsweise Methyl, Ethyl oder n-Propyl, welcher, vorzugsweise am endständigen Kohlenstoffatom, substituiert ist durch Heterocyclyl, welches in erster Linie einen gesättigten, teilweise gesättigten oder ungesättigten einfachen Ring bedeutet, der über ein Ringstickstoffatom gebunden ist und 3 bis 7 , in erster Linie 5 - 7 Ringatome enthält, wobei ausser dem bindenden Stickstoffatom noch bis zu zwei weitere Heteroatome ausgewählt aus Stickstoff, Schwefel und/oder Sauerstoff vorliegen können; als einfacher Ring vorliegt oder bis zu zweifach, vorzugsweise einfach benzanneliert, cyclopenta-, cyclohexa- oder cycloheptaanneliert sein kann; und unsubstituiert oder insbesondere durch Niederalkyl, Niederalkanoyl, Hydroxy, Niederalkoxy, Halogen, Cyano und/oder Trifluormethyl substituiert sein kann, z. B. Pyrrolyl, 2,5-Dihydropyrrolyl, Pyrrolinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, oder durch Niederalkanoyl, z. B. in 4-Niederalkanoyl-piperazin-1-yl, wie 4-Acetyl-piperazin-1-yl.

Durch Substituenten ausser Niederalkyl oder Hydroxy substituiertes Niederalkylen, welches aus A₁ und A₂ zusammen gebildet wird, ist unverzweigt, hat insbesondere 1 bis 4, bevorzugt 1 bis 3, Kohlenstoffatome und ist durch einen oder mehrere, vorzugsweise bis zu 3, insbesondere einen Substituenten ausgewählt aus Amino, Aminoniederalkyl, wie Aminomethyl, -ethyl oder -propyl, Mono- oder Diniederalkylamino, Mono- oder Diniederalkylaminoniederalkyl, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Cycloalkylamino, Cycloalkylaminoniederalkyl, Phenylniederalkylamino, Phenylniederalkylaminoniederalkyl, Phenylamino, Phenylaminoniederalkyl, Acylamino, z.B. Niederalkanoylamino, Phenylniederalkanoylamino oder Phenylcarbonylamino (≙ Benzoylamino), Acylaminoniederalkyl, z.B. Niederalkanoylaminoniederalkyl, Phenylniederalkanoylaminoniederalkyl oder Phenylcarbonylaminoniederalkyl (≙ Benzoylaminoniederalkyl), Hydroxyniederalkyl, z. B. Hydroxymethyl, Hydroxyethyl oder Hydroxypropyl, Niederalkoxy oder Niederalkoxyniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, z. B. 2-Methoxy- oder 2-Ethoxyethoxyniederalkyl, Phenylniederalkoxy, wie Benzyloxy, Phenylniederalkoxyniederalkyl, wie 2-Benzyloxy-ethyl, Acyloxy, insbesondere Niederalkanoyloxy, Acyloxyniederalkyl, insbesondere Niederalkanoyloxyniederalkyl, wie 2-Acetoxy-ethyl, Mercapto, Mercaptoniederalkyl, z. B. Mercaptomethyl oder Mercaptoethyl, Niederalkylthio oder Niederalkylthioniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, z.B. 2-Methylthio- oder 2-Ethylthio-ethylthioniederalkyl, Phenylniederalkylthio, wie Benzylthio, Phenylniederalkylthioniederalkyl, wie 2-Benzylthio-ethyl, Acylthio, insbesondere Niederalkanoylthio, Acylthioniederalkyl, insbesondere Niederalkanoylthioniederalkyl, wie 2-Acetylthio-ethyl, Carboxy, Carboxyniederalkyl, wie Carboxymethyl, verestertes Carboxy, z.B. Niederalkoxycarbonyl, wie Methoxycarbonyl, Ethoxycarbonyl oder tert-Butoxycarbonyl, verestertes Carboxyniederalkyl, z.B. Niederalkoxycarbonylniederalkyl, wie Methoxycarbonylmethyl, Ethoxycarbonylmethyl oder tert-Butoxycarbonylmethyl, oder Phenylniederalkoxycarbonylniederalkyl, wie Benzyloxycarbonylmethyl, Cyano, Cyanoniederalkyl, Carbamoyl, Carbamoylniederalkyl, wie Carbamoylmethyl, -ethyl oder -propyl, N-Niederalkylcarbamoyl, wie N-Methyl- oder N-Ethylcarbamoyl-methyl, ethyl oder -propyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylnieder-alkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl, Aryl oder Arylniederalkyl substituiertes Ureido oder Ureidoniederalkyl, insbesondere 1- oder 3-Mono-, 1,3- oder 3,3-Di- oder 1,3,3-Triniederalkylureido, 1- oder 3-Phenylureido, 1- oder 3-Mono-, 1,3- oder 3,3-Di- oder 1,3,3-Triphenylniederalkylureido, z. B. 3-Niederalkylureido, wie 3-Methyl- oder 3-Ethylureido, 1- oder 3-Mono-, 1,3- oder 3,3-Di- oder 1,3,3-Triniederalkylureidoniederalkyl, 1- oder 3-Phenylureidoniederalkyl, 1- oder 3-Mono-, 1,3- oder 3,3-Di- oder 1,3,3-Triphenylniederalkylureidoniederalkyl, z. B. 3-Niederalkylureidoniederalkyl, wie 3-Methyl- oder 3-Ethylureidoniederalkyl, vor allem 3-Methyl- oder 3-Ethylureidomethyl, -ethyl oder -propyl, Thioureido, Thioureidoniederalkyl, an einem oder beiden Stickstoffatomen durch Niederalkyl, Aryl oder Arylniederalkyl substituiertes Thioureido oder Thioureidoniederalkyl, insbesondere 1- oder 3-Mono-, 1,3- oder 3,3-Di- oder 1,3,3-Triniederalkylthioureido, 1- oder 3-Phenylthioureido, 1- oder 3-Mono-, 1,3- oder 3,3-Di- oder 1,3,3-Triphenylniederalkylthioureido, z. B. 3-Niederalkylthioureido, wie 3-Methyl- oder 3-Ethylthioureido, 1- oder 3-Mono-, 1,3- oder 3,3-Di- oder 1,3,3-Triniederalkylthioureidoniederalkyl, 1- oder 3-Phenylthioureidoniederalkyl, 1- oder 3-Mono-, 1,3- oder 3,3-Di- oder 1,3,3-Triphenylniederalkylthioureidoniederalkyl, z. B. 3-Niederalkylthioureidoniederalkyl, wie 3-Methyl- oder 3-Ethylthioureidoniederalkyl, vor allem 3-Methyl- oder 3-Ethylthioureidomethyl, -ethyl oder -propyl, Hydrazino, Hydrazinoniederalkyl, wie Hydrazinomethyl, -ethyl oder -propyl, an einem oder beiden Stickstoffatomen durch Niederalkyl, Aryl oder Arylniederalkyl substituiertes Hydrazino oder Hydrazinoniederalkyl, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, 1- oder 2-Mono-, 1,2- oder 2,2-Di- oder 1,2,2-Triniederalkylhydrazinoniederalkyl, 1- oder 2-Phenylhydrazinoniederalkyl, 1- oder 2-Mono-, 1,2- oder 2,2-Di- oder 1,2,2-Triphenylniederalkylhydrazinoniederalkyl, z. B. 2,2-Diniederalkylhydrazinoniederalkyl, wie 2,2-Dimethyl- oder 2,2-Diethylhydrazinoniederalkyl, Amidino, Amidinoniederalkyl, wie Amidinomethyl, -ethyl oder -propyl, an einem oder beiden Stickstoffatomen durch Niederalkyl, Aryl oder Arylniederalkyl substituiertes Amidino oder Amidinoniederalkyl, insbesondere N¹- oder N²-Mono-, N¹,N²- oder N¹,N¹-Di- oder N¹,N¹,N²-Triniederalkylamidino, N¹- oder N²-Phenylamidino, N¹- oder N²-Mono-, N¹,N²-, N¹,N¹-Di- oder N¹,N¹,N²-Triphenylniederalkylamidino, z. B. N¹,N¹-Diniederalkylamidino, wie N¹,N¹-Dimethyl- oder N¹,N¹-Diethylamidino, N¹- oder N²-Mono-, N¹,N²- oder N¹,N¹-Di- oder N¹,N¹,N²-Triniederalkylamidinoniederalkyl, N¹- oder N²-Phenylamidinoniederalkyl, N¹- oder N²-Mono-, N¹,N²-, N¹,N¹-Di- oder N¹,N¹,N²-Triphenylniederalkylamidinoniederalkyl, z. B. N¹,N¹-Diniederalkylamidinoniederalkyl, wie N¹,N¹-Dimethyl- oder N¹,N¹-Diethylamidinoniederalkyl, Guanidino, Guanidinoniederalkyl, wie Guanidinomethyl, -ethyl oder -propyl, an einem, zwei oder allen drei Stickstoffatomen durch Niederalkyl, Aryl oder Arylniederalkyl substituiertes Guanidino oder Guanidinoniederalkyl, insbesondere 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3-Pentaniederalkyl- oder -phenylniederalkylguanidino, insbesondere 3,3-Diniederalkylguanidino, wie 3,3-Dimethylguanidino oder 3,3-Diethylguanidino, 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3-Pentaniederalkyl- oder -Phenylniederalkylguanidinoniederalkyl, insbesondere 3,3-Diniederalkylguanidinoniederalkyl, wie 3,3-Dimethylguanidinoniederalkyl oder 3,3-Diethylguanidinoniederalkyl, Oxo, Oxoniederalkyl, insbesondere Niederalkanoyl, wie Formyl, Acetyl oder Propionyl, Thioxo, Thioxoniederalkyl, Imino, Iminoniederalkyl, Niederalkylimino, Niederalkyliminoniederalkyl, Acylimino, insbesondere Niederalkanoylimino, wie Acetylimino, Acyliminoniederalkyl, insbesondere Niederalkanoyliminoniederalkyl, wie Acetyliminoniederalkyl, Hydroxyimino, Hydroxyiminoniederalkyl, wie Hydroxyiminomethyl, Hydroxyiminoethyl oder Hydroxyiminopropyl, Niederalkoxyimino oder Niederalkoxyiminoniederalkyl, wie Methoxyimino oder Methoxyiminoniederalkyl, Hydrazono, Hydrazononiederalkyl, wie Hydrazonomethyl, Hydrazonoethyl oder Hydrazonopropyl, N-Mono- oder N,N-Diniederalkylhydrazono, N-Mono- oder N,N-Diniederalkylhydrazononiederalkyl, N-Acylhydrazono, insbesondere N-Niederalkanoylhydrazono, wie Acetylhydrazono, oder Niederalkoxycarbonylhydrazono, wie tert-Butoxycarbonylhydrazono, N-Acylhydrazononiederalkyl, insbesondere N-Niederalkanoylhydrazononiederalkyl, wie Acetylhydrazononiederalkyl, oder Niederalkoxycarbonylhydrazononiederalkyl, wie tert-Butoxycarbonylhydrazononiederalkyl, Niederalkylthioimino, wie Methyl- oder Ethylthioimino, und Niederalkylthioiminoniederalkyl, wie Methyl- oder Ethylthioiminoniederalkyl, vor allem Methyl- oder Ethylthioiminomethyl, ethyl oder -propyl, substituiert.

Bei der Definition der Gruppe -(C=X)- sind -(C=O)-, -(C=S)- und -CH₂- bevorzugt, insbesondere -(C=O)- und -(C=S)-, vor allem -(C=O)-.

Aryl ist vorzugsweise Phenyl oder Naphthyl, wie 1- oder 2-Naphthyl. Die Phenyl- und Naphthylreste können unsubstituiert oder substituiert sein, insbesondere wie nachfolgend für Phenyl angegeben. Aryl ist bevorzugt Phenyl, das unsubstituiert oder durch einen oder mehrere, vorzugsweise bis zu 5, insbesondere einen oder zwei, vor allem einen insbesondere in p-Stellung vorliegenden oder im Falle von Halogen, vor allem Fluor, als Substituenten bis zu 5, Substituenten aus der Gruppe bestehend aus Hydrocarbyl, z.B. Niederalkyl, Niederalkenyl, Niederalkinyl, Niederalkylen (angeknüpft an zwei benachbarten C-Atomen), Cycloalkyl, Phenylniederalkyl oder Phenyl; substituiertes Hydrocarbyl, z.B. Niederalkyl, das z.B. durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; verethertes Hydroxy, z.B. Niederalkoxy, Halogenniederalkoxy, Phenylniederalkoxy, Phenyloxy, Niederalkenyloxy, Halogenniederalkenyloxy, Niederalkinyloxy oder Niederalkylendioxy (angeknüpft an zwei benachbarten C-Atomen); verestertes Hydroxy, z.B. Niederalkanoyloxy, Phenylniederalkanoyloxy oder Phenylcarbonyloxy (≙ Benzoyloxy); Mercapto; verethertes Mercapto, das gegebenenfalls oxidiert ist, z.B. Niederalkylthio, Phenylniederalkylthio, Phenylthio, Niederalkylsulfinyl [-S(=O)-Niederalkyl], Phenylniederalkylsulfinyl, Phenylsulfinyl, Niederalkylsulfonyl [-S(O₂)-Niederalkyl], Phenylniederalkylsulfonyl oder Phenylsulfonyl; Halogen, Nitro, Amino; Monohydrocarbylamino, z.B. Niederalkylamino, Cycloalkylamino, Phenylniederalkylamino oder Phenylamino; Dihydrocarbylamino, z.B. Diniederalkylamino, N-Niederalkyl-N-phenylamino, N-Niederalkyl-N-phenylniederalkylamino, Niederalkylenamino oder durch -O-, -S- oder -NR'' unterbrochenes Niederalkylenamino (worin R'' für Wasserstoff, Niederalkyl oder Acyl, z.B. Niederalkanoyl, steht); Acylamino, z.B. Niederalkanoylamino, Phenylniederalkanoylamino oder Phenylcarbonylamino (≙ Benzoylamino); Acyl, z.B. Niederalkanoyl, Phenylniederalkanoyl oder Phenylcarbonyl (= Benzoyl); Carboxy; verestertes Carboxy, z.B. Niederalkoxycarbonyl; amidiertes Carboxy, z.B. Carbamoyl, N-Niederalkylcarbamoyl, N,N-Diniederalkylcarbamoyl, N-Hydroxycarbamoyl oder N-Phenylcarbamoyl; Cyano, durch zwei unabhängig voneinander aus Hydroxy, Niederalkoxy oder Phenylniederalkoxy, wie Benzyloxy, ausgewählte Reste am Phosphor substituiertes Phosphoryloxy oder durch Phenylen-1,2-dioxy am Phosphor substituiertes Phosphoryloxy (d. h. einen Rest der Formel 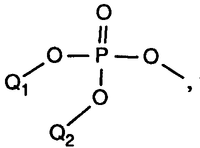
worin Q₁ und Q₂ unabhängig voneinander Wasserstoff, Niederalkyl oder Phenylniederalkyl bedeuten, oder worin Q₁ und Q₂ zusammen ortho-Phenylen bedeuten), Sulfo (SO₃H); verestertes Sulfo, z.B. Niederalkoxysulfonyl; und amidiertes Sulfo, z.B. Sulfamoyl (SO₂NH₂), N-Niederalkylsulfamoyl, N,N-Diniederalkylsulfamoyl oder N-Phenylsulfamoyl, substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; in erster Linie ist Aryl unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl oder Pentafluorphenyl.

Niederalkylen, angeknüpft an zwei benachbarten C-Atomen eines Benzolrings, ist vorzugsweise C₃-C₄-Alkylen, z.B. 1,3-Propylen oder 1,4-Butylen.

Niederalkylendioxy, angeknüpft an zwei benachbarten C-Atomen, bedeutet vorzugsweise C₁-C₂-Alkylendioxy, z.B. Methylen- oder 1,2-Ethylendioxy.

Niederalkylenamino ist vorzugsweise C₄-C₇- und insbesondere C₄-C₅-Alkylenamino, z.B. Piperidino. Durch -O-, -S- oder -NR''- unterbrochenes Niederalkylenamino steht vorzugsweise für solches C₄-C₇- und insbesondere C₄-C₅-Alkylenamino, bei dem ein Ringkohlenstoffatom durch die entsprechende Heterogruppe ersetzt ist, und ist insbesondere Morpholino, Thiomorpholino, Piperazino oder 4-Niederalkyl- oder 4-Niederalkanoylpiperazino.

Acyl bedeutet vorzugsweise Niederalkanoyl, Halogenniederalkanoyl, wie Trifluor- oder Trichloracetyl, Arylniederalkanoyl, z.B. Phenylniederalkanoyl, wie Phenylacetyl, Arylcarbonyl, wie Phenylcarbonyl, wobei Aryl in den beiden zuletzt genannten Fällen wie oben definiert ist mit der Ausnahme, dass in den zuletzt genannten Arylresten vorkommende Acylsubstituenten vorzugsweise aus Niederalkanoyl, Phenylniederalkanoyl und Phenylcarbonyl ausgewählt sind, Niederalkoxycarbonyl, wie tert-Butoxycarbonyl, oder Phenylniederalkoxycarbonyl, wie Benzyloxycarbonyl. Acyl ist in erster Linie Niederalkanoyl.

Niederalkanoyl ist vorzugsweise Formyl, Acetyl, Propionyl, n-Butyryl, Pivaloyl oder Valeroyl, insbesondere Formyl, Acetyl oder Propionyl.

Verestertes Carboxy ist vorzugsweise Niederalkoxycarbonyl, wie Methoxycarbonyl, Ethoxycarbonyl oder tert-Butoxycarbonyl, Halogenniederalkyloxycarbonyl, Fluorenyl-, Naphthyl- oder Phenylniederalkoxycarbonyl, wie Fluorenylmethoxycarbonyl oder Benzyloxycarbonyl.

Arylniederalkyl enthält vorzugsweise Aryl, wie oben definiert, und ist insbesondere Phenylniederalkyl, wie Benzyl.

Cycloalkyl ist vorzugsweise C₃-C₈- und insbesondere C₅-C₇-Cycloalkyl, was bedeuten soll, dass es 3 bis 8 bzw. 5 bis 7 Ringkohlenstoffe enthält, und ist z. B. Cyclopentyl, Cyclohexyl oder Cycloheptyl. Die genannten Cycloalkylreste können auch substituiert sein, z.B. durch Niederalkyl oder Hydroxy.

Halogen steht insbesondere für Fluor, Chlor und/oder Brom, kann aber auch Iod bedeuten.

R ist vorzugsweise aus allen vorgenannten Bedeutungen ausser Aryl ausgewählt und ist insbesondere Wasserstoff.

Heteroaryl steht für einen ungesättigten heterocyclischen Rest und ist vorzugsweise über ein Ringkohlenstoffatom verknüpft. Es handelt sich insbesondere um einen 5- oder 6-gliedrigen Ring mit bis zu drei Heteroatomen ausgewählt aus N, O und S, vor allem N, z.B. Imidazolyl, Triazolyl, Pyridyl, Pyrimidinyl oder Triazinyl, und vor allen Dingen um Pyridyl. Diese Reste können unsubstituiert oder z.B. durch Niederalkyl, Hydroxy, Niederalkoxy, Halogen, Cyano und/oder Trifluormethyl substituiert sein.

Pyridyl ist z.B. 2-, 3- oder 4-Pyridyl.

Imidazolyl ist z.B. 2- oder 4(5)-Imidazolyl.

Triazolyl ist z.B. 1,2,4-Triazol-3- oder -4-yl oder 1,2,3-Triazol-4-yl.

Pyrimidinyl ist z.B. 2-, 4- oder 5-Pyrimidinyl.

Triazinyl ist z.B. 1,3,5-Triazin-2-yl.

Soweit vorstehend oder nachstehend Thioxo als Substituent genannt ist, liegt dieses nicht an endständiges Methyl gebunden vor (d.h. nicht in Thioaldehyden).

Salze von erfindungsgemässen Verbindungen mit salzbildenden Gruppen sind in erster Linie pharmazeutisch verwendbare, nicht-toxische Salze. Beispielsweise können Verbindungen der Formel I mit basischen Gruppen, wie primären, sekundaren oder tertiären Aminogruppen Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. Carboxy, Sulfo oder Phospho, bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder Benzyl-β-phenethylamin. Verbindungen der Formel I mit einer sauren und einer basischen Gruppe können auch in Form von inneren Salzen, d.h. in zwitterionischer Form, vorliegen.

Unter die Salze der erfindungsgemässen Verbindungen fallen auch Komplexe von Verbindungen der Formel I mit Übergangsmetallionen, z.B. Kupfer, Kobalt, Platin oder Mangan.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die Verbindungen der Formel I der vorliegenden Erfindung können als einzelne Isomere oder Isomerengemische vorliegen, sofern mehrere Isomeren möglich sind. Bei Vorliegen unsymmetrisch substituierter Doppelbindungen oder Ringe, z. B. bei substituiertem Niederalkenyl A₁ oder A₂, können die cis- und/oder die trans-Form vorliegen, bei doppelt gebundenem Stickstoff an Ringsystemen, wie in Hydroxyimino, beispielsweise die syn- oder die anti-Form. Asymmetrisch substituierte Kohlenstoffatome können in der (S)-, (R)- oder (R,S)-Form vorliegen. Es können mithin bei Vorliegen entsprechender struktureller Voraussetzungen Isomerengemische (wie Racemate oder Diastereomerengemische), reine Diastereomeren oder reine Enantiomeren vorliegen.

Soweit von den Verbindungen der vorliegenden Erfindung Tautomere vorliegen können, sei es in reiner Form oder im Gleichgewicht mit anderen Tautomeren, sind diese bei den vor- und nachstehenden Definitionen ebenfalls umfasst. Tautomerisierbare Gruppen liegen z.B. vor, wenn an O oder N, welche an ein Kohlenstoffatom gebunden sind, von welchem eine Doppelbindung ausgeht, Wasserstoff gebunden ist, was Keto/Enol- bzw. Imin/Enamin-Tautomerie ermöglicht. Dies ist beispielsweise der Fall bei solchen Verbindungen der Formel I, die Thioureido- oder Guanidinoreste oder deren wie oben definierte substituierte Derivate mit mindestens einem Wasserstoffatom an einem Stickstoffatom enthalten. Es ist auch möglich, dass die tautomeren Formen, beispielsweise in Lösungen, im Gleichgewicht vorliegen. Alle derartigen Tautomeren, deren Auftreten dem Fachmann geläufig ist, sind in der vorliegenden Anmeldung ebenfalls umfasst. Bevorzugt sind Verbindungen der Formel I oder deren Salze, die in nur einer tautomeren Form vorliegen oder bei denen eine tautomere Form stark überwiegt.

Die erfindungsgemässen Verbindungen weisen wertvolle, insbesondere pharmakologisch verwendbare, Eigenschaften auf. Insbesondere zeigen sie spezifische Hemmwirkungen, die von pharmakologischem Interesse sind. In erster Linie wirken sie als Protein-Tyrosinkinase-Hemmer und zeigen z.B. eine potente Hemmung der Tyrosinkinaseaktivität des Rezeptors für den epidermalen Wachstumsfaktor (EGF) und der c-erbB2-Kinase. Diese rezeptorspezifischen Enzymaktivitäten spielen eine Schlüsselrolle in der Signalübertragung in einer Vielzahl von Säugetierzellen einschliesslich Humanzellen, insbesondere von Epithelialzellen, Zellen des Immunsystems und Zellen des zentralen und peripheren Nervensystems. Die EGF-induzierte Aktivierung der rezeptorassoziierten Protein-Tyrosinkinase (EGF-R-PTK) ist bei verschiedenen Zelltypen eine Voraussetzung für die Zellteilung und damit für die Proliferation einer Zellpopulation. Durch Zugabe von EGF-Rezeptorspezifischen Tyrosinkinasehemmern wird somit die Vermehrung dieser Zellen gehemmt.

Die Hemmung der EGF-Rezeptor-spezifischen Protein-Tyrosinkinase (EGF-R-PTK) kann z.B. mit der Methode von E. McGlynn et al. (siehe Europ. J. Biochem. 207, 265-275 (1992)) nachgewiesen werden. Die erfindungsgemässen Verbindungen hemmen die Enzymaktivität zu 50 % (IC₅₀) vorzugsweise in Konzentrationen ab etwa 0,01 µM oder höher, insbesondere bei 10⁻⁸ bis 10⁻³M, vor allem bei 10⁻⁶ bis 10⁻⁴M. Sie zeigen ferner ebenfalls im mikromolaren Bereich, vorzugsweise bei 5 x 10⁻⁷ bis 10⁻³M, insbesondere bei 10⁻⁵ bis 10⁻³M, z.B. auch eine Hemmung des Zellwachstums einer EGF-abhängigen Zellinie, etwa der epidermoiden Maus-Keratinocyten-Zellinie. Um diese Hemmung des Zellwachstums zu messen, wird die EGF-stimulierte Zellproliferation von epidermalen BALB/MK-Keratinocyten verwendet (Beschreibung des Verfahrens in Meyer, T., et al., Int. J. Cancer 43, 851 (1989). Diese Zellen sind zur Proliferation in hohem Masse auf die Gegenwart von EGF angewiesen (Weissmann, B. E., Aaronson, S. A, Cell 32, 599 (1983)). Zur Durchführung des Tests werden BALB/MK-Zellen (10 000/Vertiefung) auf 96-Loch-Mikrotiterplatten übertragen und über Nacht inkubiert. Die Testsubstanzen (gelöst in DMSO) werden in in verschiedenen Konzentrationen (in Verdünnungsreihen) zugegeben, so dass die Endkonzentration an DMSO nicht grösser als 1 % ist. Nach der Zugabe werden die Platten für drei Tage inkubiert, während denen die Kontrollkulturen ohne Testsubstanz wenigstens drei Zellteilungscyclen durchlaufen können. Das Wachstum der MK-Zellen wird gemessen mittels Methylenblau-Färbung. IC₅₀-Werte sind definiert als die Konzentration der jeweiligen Testsubstanz, die zu einer 50 %-igen Abnahme relativ zu Kontrollkulturen ohne Inhibitor führt.

Die erfindungsgemässen Verbindungen hemmen neben oder anstelle der EGF-R-PTK auch andere Tyrosinkinasen, die in die durch trophische Faktoren vermittelte Signalübertragung involviert sind, z.B. die abl-Kinase, Kinasen aus der Familie der src-Kinasen und die c-erbB2-Kinase (HER-2), sowie Serin/Threonin-Kinasen, z.B. die Proteinkinase C, welche alle in der Wachstumsregulation und Transformation bei Säugetierzellen einschliesslich Humanzellen eine Rolle spielen.

Die Hemmung der c-erbB2-Tyrosinkinase (HER-2) kann z.B. analog der für EGF-R-PTK verwendeten Methode von E. McGlynn et al. (siehe Europ. J. Biochem. 207, 265-275 (1992)) nachgewiesen werden. Die c-erbB2-Kinase kann nach bekannten Protokollen isoliert und ihre Aktivität bestimmt werden, z.B. nach T. Akiyama et al., Science 232, 1644 (1986) oder insbesondere nach P. M. Guy et al. (see J. Biol. Chem. 267, 13851-13856 (1992)).

Somit sind die erfindungsgemässen Verbindungen auch zur Hemmung der durch diese und verwandte Tyrosinkinasen vermittelten Prozesse geeignet.

Die Antitumoraktivität in vivo wird beispielsweise getestet unter Verwendung des menschlichen Epithelzellkaninoms A431 (ATCC No. CRL 1555), welches in weibliche BALB/c-Nacktmäuse (Bomholtgard, Dänemark) transplantiert wird. Für die Experimente werden in vivo auftretende Tumoren von etwa 1 cm³ Durchmesser unter sterilen Bedingungen aus den Tieren durch Excision gewonnen. Diese Tumoren werden homogenisiert, in 10 Volumina (w/v) Phosphat-gepufferter Saline suspendiert und s.c. (0,2 ml/Maus, z.B. 106 Zellen/Maus) in die linke Flanke der Tiere injiziert. Die Behandlung mit einer Testsubstanz wird 5 bis 8 Tage nach der Transplantation eingeleitet, wenn der Durchmesser der Tumoren 4 bis 5 mm beträgt. Die jeweilige Testsubstanz (beispielsweise gelöst in ®Lauroglykol (1,2-Propylenglykol-monolaurat, Gemisch der beiden Konstitutionsisomeren, Gattefossé S.A., Saint Priest, Frankreich), ®Gelucire (Glyceride und Teilpolyglyceride von Fettsäure, Gattefossé S.A., Saint Priest, Frankreich) oder Sesamöl, wird p.o. täglich während 15 aufeinanderfolgenden Tagen verabreicht. Das Tumorwachstum wird durch Überwachung des senkrechten Tumordurchmessers bestimmt und das Tumorvolumen nach der Formel π x L x D²/6 berechnet (L = Länge, D = Durchmesser des Tumors senkrecht zur Tumorachse). Die Ergebnisse können als Behandlung/Kontrolle (T/C) in Prozent ausgedrückt werden.

Die erfindungsgemässen Verbindungen sind daher z.B. bei der Behandlung benigner oder maligner Tumoren nützlich. Sie sind in der Lage, Tumorregression zu bewirken und Tumormetastasierung und das Wachstum von Mikrometastasen zu verhindern. Insbesondere sind sie bei epidermaler Hyperproliferation (Psoriasis), bei der Behandlung von Neoplasien epithelialen Charakters, z.B. Mammakarzinomen, und bei Leukämien anwendbar. Ferner sind die Verbindungen bei der Behandlung von Erkrankungen des Immunsystems und von Entzündungen einzusetzen, soweit in diese Erkrankungen Proteinkinasen involviert sind. Auch bei der Behandlung von Erkrankungen des zentralen oder peripheren Nervensystems sind die Verbindungen einzusetzen, soweit Signalübertragung durch Proteinkinasen involviert ist. Schliesslich haben die Verbindungen der vorliegenden Erfindung auch antimikrobielle Eigenschaften, die sie z. B. zur Behandlung von Erkrankungen geeignet machen, die durch Bakterien, wie Salmonella typhimurium, Viren, wie Vacciniavirus, und weitere Mikroben, welche mit auf Wachstumsfaktoren reagierenden Proteinkinasen interagieren, hervorgerufen werden.

Erfindungsgemässe Verbindungen können sowohl alleine als auch in Kombination mit anderen pharmakologisch wirksamen Substanzen angewandt werden, z.B. zusammen mit (a) Inhibitoren der Enzyme der Polyaminsynthese, (b) Inhibitoren der Proteinkinase C, (c) Inhibitoren anderer Tyrosinkinasen, (d) Cytokinen, (e) negativen Wachstumsregulatoren, z.B. TGF-β oder IFN-β, (f) Aromatasehemmern, (g) Antiöstrogenen oder (h) Cytostatika.

Bei den nachfolgend genannten Gruppen von Verbindungen der Formel I können allgemeine Definitionen, z.B. von Substituenten, unabhängig voneinander durch oben bei den allgemeinen Definitionen genannte spezifischere Definitionen ersetzt werden.

Die Erfindung betrifft insbesondere Verbindungen der Formel I, worin A₁ und A₂ unabhängig voneinander Wasserstoff, verzweigtes oder vorzugsweise lineares Niederalkyl, welches durch bis zu 2 Reste, vorzugsweise einen Rest ausgewählt aus Amino, Mono- oder Diniederalkylamino, Cycloalkylamino, Phenylniederalkylamino, Phenylamino, Niederalkanoylamino, Phenylniederalkanoylamino, Phenylcarbonylamino, Hydroxy, Niederalkoxy, Phenylniederalkoxy, Niederalkanoyloxy, Mercapto, Niederalkylthio, Phenylniederalkylthio, Niederalkanoylthio, Carboxy, Niederalkoxycarbonyl, Phenylniederalkoxycarbonyl, Cyano, Carbamoyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, Thioureido, an einem oder beiden Stickstoffatomen durch Niederalkyl, Aryl oder Arylniederalkyl substituiertes Thioureido, Hydrazino, an einem oder beiden Stickstoffatomen durch Niederalkyl, Aryl oder Arylniederalkyl substituiertes Hydrazino, Amidino, an einem oder beiden Stickstoffatomen durch Niederalkyl, Aryl oder Arylniederalkyl substituiertes Amidino, Guanidino, an einem, zwei oder allen drei Stickstoffatomen durch Niederalkyl, Aryl oder Arylniederalkyl substituiertes Guanidino, Oxo, welches nicht am Kohlenstoff gebunden ist, welcher an den A₁ oder A₂ tragenden Stickstoff gebunden ist, Thioxo, Imino, Niederalkylimino, Niederalkanoylimino, Hydroxyimino, Niederalkoxyimino, Hydrazono, N-Mono- oder N,N-Diniederalkylhydrazono, N-Niederalkanoylhydrazono, Niederalkoxycarbonylhydrazono und Niederalkylthioimino substituiert ist, vorzugsweise endständig, oder Heterocyclylniederalkyl, worin Heterocyclyl unsubstituiertes oder durch Niederalkyl, Niederalkanyol, Hydroxy, Niederalkoxy, Halogen, Cyano und/oder Trifluormethyl substituiertes Pyrrolyl, 2,5-Dihydropyrrolyl, Pyrrolinyl, Imidazolyl, Imidazolidinyl, Pyrazolinyl, Pyrazolidinyl, Triazolyl, Tetrazolyl, Tetrahydro-oxazolyl, Tetrahydro-isoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, welches endständig an den Niederalkylrest gebunden ist, bedeutet, wobei maximal einer der Reste A₁ und A₂ Wasserstoff bedeutet, oder worin A₁ und A₂ gemeinsam Niederalkylen, welches durch bis zu 3 Substituenten ausgewählt aus Amino, Aminoniederalkyl, Mono- oder Diniederalkylamino, Mono- oder Diniederalkylaminoniederalkyl, Cycloalkylamino, Cycloalkylaminoniederalkyl, Phenylniederalkylamino, Phenylniederalkylaminoniederalkyl, Phenylamino, Phenylaminoniederalkyl, Niederalkanoylamino, Phenylniederalkanoylamino, Phenylcarbonylamino, Niederalkanoylaminoniederalkyl, Phenylniederalkanoylaminoniederalkyl, Phenylcarbonylaminoniederalkyl, Hydroxyniederalkyl, Niederalkoxy, Niederalkoxyniederalkyl, Niederalkoxyniederalkoxy, Niederalkoxyniederalkoxyniederalkyl, Phenylniederalkoxy, Phenylniederalkoxyniederalkyl, Niederalkanoyloxy, Niederalkanoyloxyniederalkyl, Mercapto, Mercaptoniederalkyl, Niederalkylthio, Niederalkylthioniederalkyl, Niederalkylthioniederalkylthioniederalkyl, Phenylniederalkylthio, Phenylniederalkylthioniederalkyl, Niederalkanoylthio, Niederalkanoylthioniederalkyl, Carboxy, Carboxyniederalkyl, Niederalkoxycarbonyl, Niederalkoxycarbonylniederalkyl, Phenylniederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylnieder-alkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl, Aryl oder Arylniederalkyl substituiertes Ureido oder Ureidoniederalkyl, Thioureido, Thioureidoniederalkyl, an einem oder beiden Stickstoffatomen durch Niederalkyl, Aryl oder Arylniederalkyl substituiertes Thioureido oder Thioureidoniederalkyl, Hydrazino, Hydrazinoniederalkyl, an einem oder beiden Stickstoffatomen durch Niederalkyl, Aryl oder Arylniederalkyl substituiertes Hydrazino oder Hydrazinoniederalkyl, Amidino, Amidinoniederalkyl, an einem oder beiden Stickstoffatomen durch Niederalkyl, Aryl oder Arylniederalkyl substituiertes Amidino oder Amidinoniederalkyl, Guanidino, Guanidinoniederalkyl, an einem, zwei oder allen drei Stickstoffatomen durch Niederalkyl, Aryl oder Arylniederalkyl substituiertes Guanidino oder Guanidinoniederalkyl, Oxo, Oxoniederalkyl, Thioxo, Thioxoniederalkyl, Imino, Iminoniederalkyl, Niederalkylimino, Niederalkyliminoniederalkyl, Niederalkanoylimino, Niederalkanoyliminoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederalkanoylhydrazononiederalkyl, Niederalkoxycarbonylhydrazononiederalkyl, Niederalkylthioimino und Niederalkylthioiminoniederalkyl substituiert ist, bedeuten; Ar₁ und Ar₂ unabhängig voneinander Aryl, unsubstituiertes oder durch Niederalkyl, Hydroxy, Niederalkoxy, Halogen, Cyano und/oder Trifluormethyl substituiertes Imidazolyl, Triazolyl, Pyridyl, Pyrimidinyl oder Triazinyl oder unsubstituiertes oder durch Niederalkyl oder Hydroxy substituiertes Cycloalkyl bedeuten; die Gruppe -C(=X)- für -(C=O)-, -C(=S)- oder -CH₂- steht; und R Wasserstoff, Niederalkyl, Amino, Hydroxy oder Niederalkoxy bedeutet; wobei (soweit möglich, unabhängig von anderen Resten, die einem dieser Oberbegriffe entsprechen)

Aryl unsubstituiertes oder substituiertes Phenyl oder Naphthyl bedeutet, worin die Substituenten aus einem oder mehreren Substituenten aus der Gruppe bestehend aus Niederalkyl, Niederalkenyl, Niederalkinyl, Niederalkylen (angeknüpft an zwei benachbarten C-Atomen), Cycloalkyl, Phenylniederalkyl oder Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; Niederalkoxy, Halogenniederalkoxy, Phenylniederalkoxy, Phenyloxy, Niederalkenyloxy, Halogenniederalkenyloxy, Niederalkinyloxy oder Niederalkylendioxy (angeknüpft an zwei benachbarten C-Atomen); Niederalkanoyloxy, Phenylniederalkanoyloxy, Phenylcarbonyloxy; Mercapto; Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl; Halogen, Nitro, Amino; Niederalkylamino, Cycloalkylamino, Phenylniederalkylamino, Phenylamino; Diniederalkylamino, N-Niederalkyl-N-phenylamino, N-Niederalkyl-N-phenylniederalkylamino, Niederalkylenamino, Niederalkanoylamino, Phenylniederalkanoylamino, Phenylcarbonylamino, Niederalkanoyl, Phenylniederalkanoyl; Phenylcarbonyl; Carboxy; Niederalkoxycarbonyl; Carbamoyl, N-Niederalkylcarbamoyl, N,N-Diniederalkylcarbamoyl, N-Hydroxycarbamoyl, N-Phenylcarbamoyl; Cyano, durch zwei unabhängig voneinander aus Hydroxy, Niederalkoxy oder Phenylniederalkoxy, wie Benzyloxy, ausgewählte Reste am Phosphor substituiertes Phosphoryloxy oder durch Phenylen- 1 ,2-dioxy am Phosphor substituiertes Phosphoryloxy, Sulfo; Niederalkoxysulfonyl; Sulfamoyl, N-Niederalkylsulfamoyl, N,N-Diniederalkylsulfamoyl und N-Phenylsulfamoyl, wobei in den Substituenten vorkommende Phenylgruppen jeweils unsubstituiert oder durch Niederalkyl, Niederalkoxy, Hydroxy, Halogen und/oder Trifluormethyl substituiert sind, ausgewählt sind; und worin
Cycloalkyl C₃-C₈-Cycloalkyl, insbesondere Cyclopentyl, Cyclohexyl oder Cycloheptyl bedeutet;
und pharmazeutisch verwendbare Salze dieser Verbindungen, sofern salzbildende Gruppen vorliegen.

Bevorzugt sind die Verbindungen der Formel I, worin A₁ und A₂ unabhängig voneinander aus Wasserstoff und Niederalkyl, welches durch bis zu 2 Reste, vorzugsweise einen Rest ausgewählt aus Amino, Mono- oder Diniederalkylamino, Niederalkylamino, wie Acetylamino, Hydroxy, Carboxy, Niederalkoxycarbonyl, Phenylniederalkoxycarbonyl, Carbamoyl, N-Niederalkylcarbamoyl, N,N-Diniederalkylcarbamoyl, Ureido, an einem oder beiden Stickstoffatomen durch Niederalkyl substituiertes Ureido, Thioureido, an einem oder beiden Stickstoffatomen durch Niederalkyl substituiertes Thioureido, Amidino, an einem oder beiden Stickstoffatomen durch Niederalkyl substituiertes Amidino, Guanidino, an einem, zwei oder allen drei Stickstoffatomen durch Niederalkyl substituiertes Guanidino, Oxo, welches nicht am Kohlenstoff gebunden ist, welcher an den A₁ oder A₂ tragenden Stickstoff gebunden ist, Hydroxyimino, Niederalkoxyimino, Hydrazono, N-Mono- oder N,N-Diniederalkylhydrazono, N-Niederalkanoylhydrazono, Niederalkoxycarbonylhydrazono und Niederalkylthioimino ausgewählt sind, wobei maximal einer der beiden Reste A₁ und A₂ Wasserstoff bedeuten kann, oder A₁ und A₂ zusammen Niederalkylen, welches durch einen oder bis zu 3, vorzugsweise einen Substituenten ausgewählt aus Amino, Aminoniederalkyl, Mono- oder Diniederalkylamino, Mono- oder Diniederalkylaminoniederalkyl, Hydroxyniederalkyl, Carboxy, Carboxyniederalkyl, Niederalkoxycarbonyl, Niederalkoxycarbonylniederalkyl, Phenylniederalkoxycarbonylniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, Ureido, Ureidoniederalkyl, an einem oder beiden Stickstoffatomen durch Niederalkyl substituiertes Ureido oder Ureidoniederalkyl, Thioureido, Thioureidoniederalkyl, an einem oder beiden Stickstoffatomen durch Niederalkyl substituiertes Thioureido oder Thioureidoniederalkyl, Amidino, Amidinoniederalkyl, an einem oder beiden Stickstoffatomen durch Niederalkyl substituiertes Amidino oder Amidinoniederalkyl, Guanidino, Guanidinoniederalkyl, an einem, zwei oder allen drei Stickstoffatomen durch Niederalkyl substituiertes Guanidino oder Guanidinoniederalkyl, Oxo, Oxoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederalkanoylhydrazononiederalkyl, Niederalkoxycarbonylhydrazononiederalkyl, Niederalkylthioimino und Niederalkylthioiminoniederalkyl substituiert ist, bedeutet; Ar₁ und Ar₂ Phenyl, das unsubstituiert oder durch einen Substituenten ausgewählt aus Trifluormethyl, Hydroxy, Niederalkoxy, Halogenniederalkoxy, Phenylniederalkoxy, Phenyloxy, Niederalkanoyloxy, Phenylniederalkanoyloxy, Phenylcarbonyloxy, Cyano, Sulfo und Sulfamoyl substituiert oder bis zu fünfmal durch Halogen substituiert ist, die Gruppe -(C=X)- für -(C=O)- oder -(C=S)- steht, und R Wasserstoff oder Niederalkyl bedeutet, und pharmazeutisch verwendbare Salze davon, sofern salzbildende Gruppen vorliegen.

Stärker bevorzugt sind Verbindungen der Formel I, worin A₁ und A₂ unabhängig voneinander Wasserstoff oder Niederalkyl, welches durch einen Rest ausgewählt aus Amino, wie in Aminomethyl, 2-Aminoethyl, 3-Aminopropyl oder 4-Aminobutyl, Niederalkanoylamino, wie in 3-Acetylaminopropyl, Carbamoyl, wie in Carbamoylmethyl, 2-Carbamoylethyl oder 3-Carbamoylpropyl, Ureido, wie in 3-Ureidopropyl, an einem oder beiden Stickstoffatomen durch Niederalkyl substituiertes Ureido, z.B. 3-Niederalkylureido, wie in 3-Methyl-ureido- oder 3-Ethylureido-methyl, -ethyl oder -propyl, Thioureido, wie in 3-Thioureidopropyl, an einem oder beiden Stickstoffatomen durch Niederalkyl substituiertes Thioureido, z.B. 3-Niederalkylthioureido, wie in 3-Methyl- oder 3-Ethyl-thioureido-methyl, -ethyl oder -propyl, Amidino, wie in Amidinomethyl, -ethyl oder -propyl, Guanidino, wie in Guanidinomethyl, -ethyl oder -propyl, und Niederalkylthioimino, wie in Methyltioimino- oder Ethylthioimino-methyl, -ethyl oder -propyl, substituiert ist, bedeuten, wobei maximal einer der Reste A₁ und A₂ Wasserstoff bedeuten kann, oder worin A₁ und A₂ zusammen Niederalkylen, welches durch einen Substituenten ausgewählt aus Amino, Aminoniederalkyl, wie Aminomethyl, 2-Aminoethyl oder 3-Aminopropyl, Hydroxyniederalkyl, wie Hydroxymethyl, 2-Hydroxyethyl oder 3-Hydroxypropyl, Carboxy, Carboxyniederalkyl, wie Carboxymethyl, Niederalkoxycarbonyl, wie Methoxycarbonyl, Ethoxycarbonyl oder tert-Butoxycarbonyl, Niederalkoxycarbonylniederalkyl, wie Methoxycarbonylmethyl, Ethoxycarbonylmethyl oder tert-Butoxycarbonylmethyl, Niederalkanoyl, wie Formyl, Acetyl oder Propionyl, Hydroxyimino, Hydroxyiminoniederalkyl, wie Hydroxyiminomethyl, 1- oder 2-Hydroxymethyliminoethyl oder 1-, 2- oder 3-Hydroxyiminopropyl, Hydrazono und Hydrazononiederalkyl, wie Hydrazonomethyl, 1- oder 2-Hydrazonoethyl oder 1-, 2- oder 3-Hydrazonopropyl, substituiert ist, vor allem Ethylen oder (alternativ oder ergänzend zu Ethylen) Methylen, welches in 1-Stellung durch einen der genannten Reste Aminoniederalkyl, Hydroxyniederalkyl, Carboxy, Niederalkoxycarbonyl, Niederalkanoyl, Hydroxyiminoniederalkyl oder Hydrazononiederalkyl substituiert ist, bedeuten; Ar₁ und Ar₂ Phenyl oder 4-Halogenphenyl, z.B. 4-Fluorphenyl, bedeuten; die Gruppe -(C=X)- für -(C=O)- steht; und R Wasserstoff bedeutet, und pharmazeutisch verwendbare Salze davon, sofern salzbildende Gruppen vorliegen.

Noch stärker bevorzugt sind die Verbindungen der Formel I, worin A₁ und A₂ unabhängig voneinander 2-Aminoethyl, 3-Aminopropyl, 4-Aminobutyl, 3-Acetylaminopropyl, 2-Carbamoylethyl, 3-Carbamoylpropyl, 2-(3-Methylureido)-ethyl, 2-(3-Ethylureido)-ethyl, 3-Ureidopropyl, 3-Thioureidopropyl, 3-(3-Methylureido)-propyl, 3-(3-Ethylureido)-propyl, 2-(3-Methylthioureido)-ethyl, 2-(3-Ethylthioureido)-ethyl, 3-(3-Methylthioureido)-propyl, 3-(3-Ethylthioureido)-propyl, 2-Amidinoethyl, 3-Amidinopropyl, 2-Guanidinoethyl, 3-Guanidinopropyl, 3-(N-Methylthioimino)-propyl oder Wasserstoff bedeuten, wobei maximal einer der Reste A₁ und A₂ Wasserstoff bedeuten kann, oder worin A₁ und A₂ zusammen in 1-Stellung durch Aminomethyl, 3-Aminopropyl, Hydroxymethyl, Carboxy, Methoxycarbonyl, Ethoxycarbonyl, Propionyl, Hydroxyiminomethyl oder Hydrazonomethyl substituiertes Ethylen oder (alternativ oder ergänzend zu Ethylen) Methylen bedeuten; Ar₁ und Ar₂ unabhängig voneinander Phenyl oder 4-Fluorphenyl bedeuten, wobei Ar₁ und Ar₂ vorzugsweise die gleiche Bedeutung haben; die Gruppe -(C=X)- für -(C=O)- steht; und R Wasserstoff bedeutet, und pharmazeutisch verwendbare Salze davon, sofern salzbildende Gruppen vorliegen.

Sehr bevorzugt sind die Verbindungen der Formel I, worin A₁ und A₂ unabhängig voneinander 2-Aminoethyl, 3-Aminopropyl, 2-Carbamoylethyl, 3-Ureidopropyl, 3-(3-Methylureido)-propyl, 3-Thioureidopropyl oder Wasserstoff bedeuten, wobei maximal einer der Reste A₁ und A₂ Wasserstoff bedeuten kann, oder worin A₁ und A₂ zusammen in 1-Stellung durch Aminomethyl, Hydroxymethyl, Carboxy, Methoxycarbonyl, Propionyl, Hydroxyiminomethyl oder Hydrazonomethyl substituiertes Ethylen oder (alternativ oder ergänzend zu Ethylen) Methylen bedeuten; Ar₁ und Ar₂ Phenyl bedeuten; die Gruppe -(C=X)- für -(C=O)- steht; und R Wasserstoff bedeutet, und pharmazeutisch verwendbare Salze davon, sofern salzbildende Gruppen vorliegen.

Von allen bisher genannten Verbindungen der Formel I sind jeweils solche in allererster Linie bevorzugt, worin worin einer der beiden Reste A₁ und A₂ Wasserstoff bedeutet, der andere unsubstituiertes oder durch einen der jeweils genannten Substituenten substituiertes Niederalkyl bedeutet; oder worin A₁ und A₂ zusammen durch einen der jeweils genannten Substituenten substituiertes Niederalkylen bedeuten; während die übrigen Reste die genannten Bedeutungen haben, pharmazeutisch verwendbare Salze davon, sofern salzbildende Gruppen vorliegen, und Tautomere davon, sofern tautomerisierbare Gruppen vorliegen.

Die Erfindung betrifft vor allem die in den Beispielen beschriebenen spezifischen Verbindungen und pharmazeutisch anwendbare Salze davon.

Die Verbindungen der Formel I können in an sich bekannter Weise hergestellt werden, indem man z.B.
a) eine Carbonsäure der Formel II, 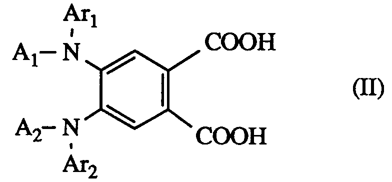 worin Ar₁, Ar₂, A₁ und A₂ die unter Formel I angegebene Bedeutung haben, oder ein reaktionsfähiges Säurederivat davon mit einer Aminoverbindung der Formel III,

   H₂N-R (III)

   worin R die unter Formel I angegebene Bedeutung hat, umsetzt, wobei in den Ausgangsmaterialien funktionelle Gruppen, die nicht an der Reaktion teilnehmen sollen, erforderlichenfalls in geschützter Form vorliegen, und vorhandene Schutzgruppen abspaltet, oder
b) zur Herstellung von Verbindungen der Formel I, worin A₁ und A₂ zusammen durch verestertes Carboxy oder Niederalkanoyl substituiertes Methylen bedeuten, eine Verbindung der Formel XI, 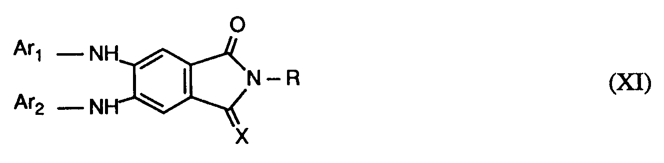 worin Ar₁, Ar₂, X und R die für Verbindungen der Formel I genannten Bedeutungen haben, mit einem Aldehyd der Formel XII,

   R₀-CHO (XII)

   worin R₀ verestertes Carboxy oder Niederalkanoyl bedeutet, oder einem reaktionsfähigen Derivat davon, umsetzt, wobei in den Ausgangsmaterialien funktionelle Gruppen, die nicht an der Reaktion teilnehmen sollen, erforderlichenfalls in geschützter Form vorliegen, und vorhandene Schutzgruppen abspaltet,

und gewünschtenfalls eine erhältliche Verbindung der Formel I in eine andere Verbindung der Formel I umwandelt, und/oder ein erhältliches Salz in die freie Verbindung oder in ein anderes Salz umwandelt, und/oder eine erhältliche freie Verbindung der Formel I in ein Salz umwandelt und/oder ein erhältliches Gemisch von isomeren Verbindungen der Formel I in die einzelnen Isomeren auftrennt.

In der folgenden näheren Beschreibung der Verfahren haben die Symbole Ar₁, Ar₂, A₁, A₂, X, R, R₁ und R₂ jeweils die unter Formeln I und II angegebene Bedeutung, sofern nichts anderes angegeben ist, die Reste R₃ und R₄ bedeuten Aryl, Arylniederalkyl oder Niederalkyl.

### Verfahren a): Bildung eines Imids

Die Carbonsäuren der Formel II liegen entweder mit freien Carboxygruppen vor, oder als reaktionsfähige Derivate (wobei eine oder vorzugsweise beide Carboxygruppen derivatisiert sein können), beispielsweise als von der freien Carboxyverbindung abgeleiteter Ester, z. B. als aktivierter Ester, als reaktionsfähiges Anhydrid, oder ferner als reaktionsfähiges cyclisches Amid. Die reaktionsfähigen Derivate können auch in situ gebildet werden.

Ester von Verbindungen der Formel II sind vorzugsweise solche der Formel II', 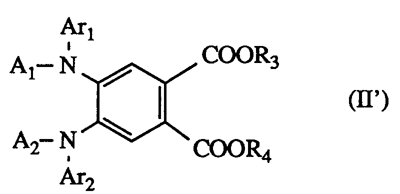
worin Ar₁, Ar₂, A₁ und A₂ die unter Formel I angegebene Bedeutung haben und R₃ und R₄ unabhängig voneinander Aryl, z.B. 4-Nitrophenyl, Arylniederalkyl, z.B. Benzyl, oder Niederalkyl, wie Methyl, bedeuten.

Aktivierte Ester von Verbindungen der Formel II sind insbesondere am Verknüpfungskohlenstoffatom des veresternden Restes ungesättigte Ester, z. B. vom Vinylester-Typ, wie Vinylester (erhältlich z. B. durch Umesterung eines entsprechenden Esters mit Vinylacetat; Methode des aktivierten Vinylesters), Carbamoylester (erhältlich z. B. durch Behandeln der entsprechenden Säure mit einem Isoxazoliumreagens; 1,2-Oxazolium- oder Woodward-Methode), oder 1-Niederalkoxyvinylester (erhältlich z. B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z. B. durch Behandeln der entsprechenden Säure mit einem geeigneten N,N'-disubstituierten Carbodiimid, z. B. N,N'-Dicyclohexylcarbodiimid; Carbodiimid-Methode), oder N,N-di-substituierte Amidinoester (erhältlich z. B. durch Behandeln der entsprechenden Säure mit einem N,N-disubstituierten Cyanamid; Cyanamid-Methode), geeignete Arylester, insbesondere durch elektronenanziehende Substituenten substituierte Phenylester (erhältlich z. B. durch Behandeln der entsprechenden Säure mit einem geeignet substituierten Phenol, z. B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z. B. durch Behandeln der entsprechenden Säure mit Chloracetonitril in Gegenwart einer Base; Cyanmethylester-Methode), Thioester, insbesondere gegebenenfalls, z. B. durch Nitro, substituierte Phenylthioester (erhältlich z. B. durch Behandeln der entsprechenden Säure mit gegebenenfalls, z. B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z. B. durch Behandeln der entsprechenden Säure mit einer N-Hydroxyamino- bzw. N-Hydroxyamidoverbindung, z. B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benztriazin-4-on, z. B. nach der Anhydrid- oder Carbodiimid-Methode; Methode der aktivierten N-Hydroxyester).

Anhydride von Säuren der Formel II können innere Anhydride oder gemischte Anhydride dieser Säuren sein, z. B. Anhydride mit anorganischen Säuren, wie Säurehalogenide, insbesondere Säurechloride (erhältlich z. B. durch Behandeln der entsprechenden Säure mit Thionylchlorid, Phosphorpentachlorid oder Oxalylchlorid; Säurechloridmethode), Azide (erhältlich z. B. aus einem entsprechenden Säureester über das entsprechende Hydrazid und dessen Behandlung mit salpetriger Säure; Azidmethode), Anhydride mit Kohlensäurehalbestern, z. B. Kohlensäureniederalkylhalbestern (erhältlich z. B. durch Behandeln der entsprechenden Säure mit Chlorameisensäureniederalkylestern oder mit einem 1-Niederalkoxycarbonyl-2-niederalkoxy-1,2-dihydrochinolin; Methode der gemischten O-Alkylkohlensäureanhydride), oder Anhydride mit dihalogenierter, insbesondere dichlorierter Phosphorsäure (erhältlich z. B. durch Behandeln der entsprechenden Säure mit Phosphoroxychlorid; Phosphoroxychloridmethode), Anhydride mit anderen Phosphorsäurederivaten (z. B. solchen, die man mit Phenyl-N-phenylphosphoramidochloridat erhalten kann oder durch Umsetzung von Alkylphosphorsäureamiden in Gegenwart von Sulfonsäureanhydriden und/oder racemisierungssenkenden Additiven, wie N-Hydroxybenztriazol, oder in Gegenwart von Cyanphosphonsäurediethylester) oder mit Phosphorigsäurederivaten, oder Anhydride mit organischen Säuren, wie gemischte Anhydride mit organischen Carbonsäuren (erhältlich z. B. durch Behandeln der entsprechenden Säure mit einem gegebenenfalls substituierten Niederalkan- oder Phenylniederalkancarbonsäurehalogenid, z. B. Phenylessigsäure-, Pivalinsäure- oder Trifluoressigsäurechlorid; Methode der gemischten Carbonsäureanhydride) oder mit organischen Sulfonsäuren (erhältlich z. B. durch Behandeln eines Salzes, wie eines Alkalimetallsalzes, der entsprechenden Säure mit einem geeigneten organischen Sulfonsäurehalogenid, wie Niederalkan- oder Aryl-, z. B. Methan- oder p-Toluolsulfonsäurechlorid; Methode der gemischten Sulfonsäureanhydride) sowie symmetrische Anhydride (erhältlich z. B. durch Kondensation der entsprechenden Säure in Gegenwart eines Carbodiimides oder von 1-Diethylaminopropin; Methode der symmetrischen Anhydride). Innere Anhydride lassen sich vorzugsweise aus einer freien Dicarbonsäure der Formel II durch Umsetzung mit Säureanhydriden der Formel (X), R₅-(C=O)-O-(C=O)-R₅', worin R₅ und R₅' unabhängig voneinander für Wasserstoff oder Niederalkyl, nicht jedoch beide für Wasserstoff, stehen, insbesondere Acetanhydrid, herstellen.

Anhydride von Carbonsäuren der Formel II sind vorzugsweise innere Anhydride der Formel IV, 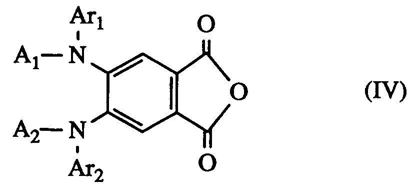
worin A₁, A₂, Ar₁und Ar₂ die unter Formel I angegebene Bedeutung haben.

Geeignete cyclische Amide sind insbesondere Amide mit fünfgliedrigen Diazacyclen aromatischen Charakters, wie Amide mit Imidazolen, z. B. Imidazol (erhältlich z. B. durch Behandeln der entsprechenden Säure mit N,N'-Carbonyldiimidazol; Imidazol-Methode), oder Pyrazol, z. B. 3,5-Dimethylpyrazol (erhältlich z. B. über das Säurehydrazid durch Behandeln mit Acetylaceton; Pyrazolid-Methode).

Wie erwähnt, können Derivate von Carbonsäuren, die als Acylierungsmittel verwendet werden, auch in situ gebildet werden. So kann man z. B. N,N'-disubstituierte Amidinoester in situ bilden, indem man das Gemisch des Ausgangsmaterials der Formel m und der als Acylierungsmittel verwendeten Säure der Formel II in Gegenwart eines geeigneten N,N'-disubstituierten Carbodiimids, z. B. N,N'-Cyclohexylcarbodiimid, zur Reaktion bringt, beispielsweise in Gegenwart einer geeigneten Base, wie Triethylamin. Weiter kann man Amino- oder Amidoester der als Acylierungsmittel verwendeten Säuren in Gegenwart des zu acylierenden Ausgangsmaterials der Formel m bilden, indem man das Gemisch der entsprechenden Säure- und Amino-Ausgangsmaterialien in Gegenwart eines N,N'-disubstituierten Carbodiimids, z. B. N,N'-Dicyclohexylcarbodiimid, und eines N-Hydroxyamins oder N-Hydroxy-amids, z. B. N-Hydroxysuccinimid, gegebenenfalls in Anwesenheit einer geeigneten Base, z. B. 4-Dimethylamino-pyridin, umsetzt. Ferner kann man durch Umsetzung mit N,N,N',N'-Tetraalkyluroniumverbindungen, wie O-Benztriazol-1-yl-N,N,N',N'-tetra-methyl-uronium-hexafluorphosphat, Aktivierung in situ erreichen. Schliesslich können Phosphorsäureanhydride der Carbonsäuren der Formel II in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart eines racemisierungssenkenden Additives, wie N-Hydroxybenztriazol, umsetzt.

Die Umsetzung zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, für Kondensationsreaktionen beschrieben.

Bevorzugt ist die Umsetzung einer Verbindung der Formel II' entsprechend der an sich bekannten Aminolyse von Phthalsäurediestern mit Ammoniak oder primären Aminen. Die Umsetzung verläuft mit aktivierten Phthalsäurediarylestern, z.B. dem Di(p-nitrophenyl)ester, normalerweise bei Raumtemperatur, mit Diniederalkylestern dagegen meist nur bei hohen Temperaturen. Besonders bevorzugt ist die Umsetzung von Diniederalkylestern in Lösungsmitteln, insbesondere einem hochsiedenden Alkohol, z. B. einem Diol, wie Ethylenglykol, oder auch Glycerin, bei Normaldruck und Temperaturen von 100 bis 150 °C, z.B. von etwa 120 °C, oder die Umsetzung der Niederalkylester mit Ammoniak oder dem betreffenden Amin der Formel III bei denselben Temperaturen in Anwesenheit eines Lösungsmittels, beispielsweise eines Alkoholes, z.B. eines Niederalkanoles, wie Methanol oder Ethanol, oder ferner in Abwesenheit eines Lösungsmittels, in einem Autoklaven bei erhöhtem Druck. Vorzugsweise wird als Phthalsäurediester der Dimethylester verwendet.

Sind ausser den Resten -COOR₃ und -COOR₄ in den Verbindungen der Formel II' noch weitere veresterte Carboxyreste als Substituenten vorhanden, z.B. in einem Niederalkoxycarbonyl-niederalkyl als A₁ und/oder A₂ oder in einem durch Niederalkoxycarbonyl und/oder Niederalkoxycarbonylniederalkyl substituierten Niederalkylen, welches aus A₁ und A₂ zusammen gebildet wird, vorhanden, so können diese bei der Umsetzung mit Ammoniak, Niederalkylaminen, Diniederalkylaminen, Hydroxylamin oder Phenylamin (vorzugsweise im selben Ansatz praktisch gleichzeitig mit den Resten -COOR₃ und -COOR₄) zu den entsprechenden Amiden umgesetzt werden.

Bevorzugt ist auch die Umsetzung entsprechend der an sich bekannten Aminolyse von Phthalsäureanhydriden der Formel IV mit Verbindungen der Formel III [Ammoniak oder primäre Amine (Niederalkylamin, Arylniederalkylamin, Arylamin, Hydrazin (an einem N-Atom geschützt), Hydroxylamin oder Niederalkyloxyamin)], welche vorzugsweise bei höheren Temperaturen oder mit Hexamethyldisilazan und Methanol bei Raumtemperatur [Davis, Peter D. and Bit, Rino A., Tetrahedron Lett. 31, 5201-5204 (1990)] erfolgt.

Zu den Schutzgruppen für funktionelle Gruppen in Ausgangsmaterialien, deren Umsetzung vermieden werden soll, insbesondere Carboxy-, Amino-, Hydroxy-, Mercapto- und Sulfogruppen, zählen insbesondere diejenigen Schutzgruppen (conventional protecting groups), die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen. Charakteristisch für Schutzgruppen ist auch, dass sie in den Endstoffen nicht vorliegen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z. B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1 -Methylthiomethoxycarbonyl oder 1 -Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Iodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe wird auch als organische Silyloxycarbonylgruppe geschützt. Eine organische Silyloxycarbonylgruppe ist beispielsweise eine Triniederalkylsilyloxycarbonylgruppe, z. B. Trimethylsilyloxycarbonyl. Das Siliciumatom der Silyloxycarbonylgruppe kann auch durch zwei Niederalkyl-, z. B. Methylgruppen, und eine Amino- oder Carboxygruppe eines zweiten Moleküls der Formel I substituiert sein. Verbindungen mit solchen Schutzgruppen lassen sich z. B. mit Dimethylchlorsilan als Silylierungsmittel herstellen.

Eine Carboxygruppe wird auch in Form eines inneren Esters mit einer in geeignetem Abstand, z. B. in γ-Stellung, zur Carboxygruppe im Molekül vorliegenden Hydroxygruppe, d. h. in Form eines Lactons, vorzugsweise eines γ-Lactons, geschützt.

Eine geschützte Carboxygruppe ist bevorzugt tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl, oder eine in Form eines Lactons, insbesondere eines γ-Lactons, geschützte Carboxygruppe.

Die Freisetzung von geschütztem Carboxy erfolgt nach üblichen, z. B. den in den oben genannten Standardwerken über Schutzgruppen genannten Verfahren.

So kann geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall-, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z. B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin. Als innerer Ester, wie als γ-Lacton, geschütztes Carboxy kann durch Hydrolyse in Gegenwart eines Hydroxid-haltigen Base, wie Erdalkalihydroxides oder insbesondere eines Alkalimetallhydroxides, z. B. NaOH, KOH oder LiOH, besonders LiOH, freigesetzt werden, wobei gleichzeitig die entsprechend geschützte Hydroxygruppe freigesetzt wird.

Eine geschützte Aminogruppe ist durch eine Aminoschutzgruppe geschützt, z. B. in Form einer Acylamino-, Arylmethylamino-, veretherten Mercaptoamino-,2-Acyl-niederalk-1-enylamino oder Silylaminogruppe oder als Azidogruppe.

In einer Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind vorzugsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Iod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung substituiertes Niederalkoxycarbonyl, z. B. tert-Niederalkoxycarbonyl, wie tert-Butoxycarbonyl, Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z.B. Benzyloxycarbonyl, 4-Nitrobenzyloxycarbonyl, Diphenylmethoxycarbonyl, 9-Fluorenylmethoxycarbonyl oder Di-(4-methoxyphenyl)methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Iodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, z. B. einer Mono-, Di- oder insbesondere Triarylmethylaminogruppe, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonyl-prop- 1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als Säureanionen kommen vor allem diejenigen von starken anorganischen Säuren, wie von Schwefelsäure, Phosphorsäure oder Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl, z.B. tert-Butoxycarbonyl oder Benzyloxycarbonyl.

Bevorzugt sind auch bivalente Aminoschutzgruppen, wie mono- oder disubstituierte Methylidengruppen, wie 1-Niederalkoxy (beispielsweise Methoxy oder Ethoxy)-niederalkyliden (beispielsweise Ethyliden oder 1-n-Butyliden), z.B. =C(CH₃)(OC₂H₅), ferner z.B. =C(CH₃)₂ oder =CH-Phenyl, und insbesondere Bisacylreste, z.B. der Phthalylrest, welcher zusammen mit dem zu schützenden Stickstoffatom ein 1H-isoindol-1,3(2H)-dion (Phthalimidogruppe) bildet.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, insbesondere Bromwasserstoff, oder von Schwefel- oder Phosphorsäure, vorzugsweise von Chlorwasserstoff, in polaren Lösungsmitteln, wie Wasser oder einer Carbonsäure, wie Essigsäure, oder Ethern, bevorzugt cyclischen Ethem, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisen- oder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, bevorzugt in polaren Lösungsmitteln, wie Diniederalkylniederalkanoylamiden, z. B. Dimethylformamid, Ethern, wie cyclischen Ethern, z. B. Dioxan, oder Alkoholen, wie Methanol, Ethanol oder Propanol, wobei Methanol besonders bevorzugt ist, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig- oder Trifluoressigsäure, gegebenenfalls in Gegenwart von Wasser, gespalten werden, und eine als Silylamino geschützten Aminogruppe kann z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten. Die Abspaltung der Phthalylgruppe kann z.B. mittels Hydrazinhydrat geschehen oder mittels einer Säure, z.B. einer Mineralsäure, wie Chlorwasserstoffsäure, oder einer organischen Säure, wie Essigsäure, gegebenenfalls in Gegenwart von organischen Lösungsmitteln, z.B. Methanol oder Tetrahydrofuran.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine Hydroxygruppe kann beispielsweise durch eine Acylgruppe, z. B. unsubstituiertes oder durch Halogen, wie Chlor, substituiertes Niederalkanoyl, wie Acetyl oder 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Di- oder Triphenylmethoxycarbonyl. Eine Hydroxygruppe kann ferner durch Triniederalkylsilyl, z. B. Trimethylsilyl, Triisopropylsilyl oder tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, ein Thiaanaloges davon oder durch 1-Phenylniederalkyl, wie Benzyl, Diphenylmethyl oder Trityl, wobei die Phenylreste beispielsweise durch Halogen, z.B. Chlor, Niederalkoxy, z. B. Methoxy, und/oder Nitro substituiert sein können, geschützt sein.

Zwei in einem Molekül vorkommende, insbesondere benachbarte Hydroxygruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine in Nachbarstellung zu einer Carboxygruppe stehende Hydroxygruppe kann durch Bildung eines inneren Esters (Lacton), insbesondere eines γ-Lactones, geschützt sein.

Bevorzugt ist eine geschützte Hydroxygruppe durch Triniederalkylsilyl oder als Lacton geschützt, insbesondere durch tert-Butyl-dimethylsilyl.

Eine Mercaptogruppe kann insbesondere durch S-Alkylierung mit gegebenenfalls substituierten Alkylresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylrest, z. B. durch Methoxy, substituiertes Diphenylmethyl, wie Di-(4-methoxyphenyl)-methyl, Triphenylmethyl, Pyridyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thia-aliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifiuoressigsäure, freigesetzt. Durch Pyridyldiphenylmethyl geschütztes Mercapto kann z. B. durch Quecksilber-II-salze bei pH 2-6 oder durch Zink/Essigsäure oder elektrolytische Reduktion, Acetamidomethyl und iso-Butyrylamidomethyl z. B. durch Reaktion mit Quecksilber-II-salzen bei pH 2-6, 2-Chloracetamidomethyl z. B. durch 1-Piperidinothiocarboxamid, S-Ethylthio, S-tert-Butylthio und S-Sulfo z. B. durch Thiolyse mit Thiophenol, Thioglycolsäure, Natrium-thiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z. B. einer durch Niederalkyl ein- oder zweifach substituierten Methylengruppe, wie durch Niederalkyliden, z. B. Isopropyliden, Cycloalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt. Veresterte Hydroxygruppen, z. B. Niederalkanoyloxy, wie Acetyloxy, können auch durch Esterasen freigesetzt werden, acyliertes Amino beispielsweise durch geeignete Peptidasen.

Eine Sulfogruppe kann beispielsweise durch Niederalkyl, z. B. Methyl oder Ethyl, durch Phenyl oder als Sulfonamid, beispielsweise als Imidazolid, geschützt sein.

Eine als Sulfonsäureester oder Sulfonamid geschützte Sulfogruppe wird beispielsweise durch saure Hydrolyse, z. B. in Gegenwart von Mineralsäure, oder bevorzugt durch basische Hydrolyse, z. B. mit Alkalimetallhydroxid oder Alkalimetallcarbonat, beispielsweise Natriumcarbonat, freigesetzt.

Oxo wird erforderlichenfalls geschützt durch Acetalbildung, z. B. mit Niederalkanolen, insbesondere mit Ethan-1,2-diol, wobei die Schutzgruppe auf der gewünschten Stufe durch Hydrolyse in Gegenwart einer Säure, wie Essigsäure oder Schwefelsäure, abgespalten werden kann, Thioxoniederalkyl wird erforderlichenfalls geschützt durch Thioacetalbildung, z. B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann.

Die Temperaturen für die Freisetzung der geschützten funktionellen Gruppen liegen vorzugsweise zwischen -80 und 100 °C, vor allem zwischen -20 und 50 °C, z.B. zwischen 10 und 35 °C, wie im Bereich der Raumtemperatur oder bei Rückflusstemperatur.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

Die Ausgangsmaterialien der Formel II' werden z.B. dadurch hergestellt, dass man ein Cyclohexadien der Formel V 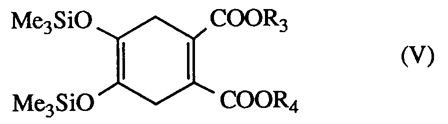
worin Me für Methyl steht (alternativ könnten auch andere Niederalkylreste vorliegen) und worin R₃ und R₄ die für Verbindungen der Formel II' genannten Bedeutungen haben, mit einem Anilin der Formel VI

AHN-Ar (VI)

worin A insbesondere Wasserstoff oder substituiertes Niederalkyl, substituiertes Niederalkenyl, substituiertes Niederalkinyl oder Heterocyclylniederalkyl bedeutet, wie oben für A₁ oder A₂ definiert, und worin Ar Aryl, Heteroaryl oder unsubstituiertes oder substituiertes Cycloalkyl bedeutet, wie oben für Ar₁ oder Ar₂ definiert, unter Säurekatalyse umsetzt [s. Matlin, Stephen A. and Barron, Kenneth, J. Chem. Res. Synop. 8, 246-247 (1990)]. In den Ausgangsmaterialien sind funktionelle Gruppen, die nicht an der Reaktion teilnehmen sollen, erforderlichenfalls geschützt. Vorhandene Schutzgruppen werden erforderlichenfalls auf einer geeigneten Reaktionsstufe entfernt. Schutzgruppen, ihre Einführung und ihre Abspaltung sind oben bereits beschrieben. Substituenten an den Resten Ar können auch nach der Kondensation durch Methoden der klassischen Aromaten- bzw. Heterocyclenchemie oder enzymatische Methoden (z.B. 4-Hydroxylierungen) eingeführt werden.

Die Herstellung der Verbindungen der Formel V erfolgt z.B. via einer Diels-Alder-Reaktion aus einem 2,3-Bis(triniederalkylsilyloxy)butadien und einem Acetylendicarbonsäure-diniederalkylester, ist ebenfalls in der angegebenen Literatur (Matlin et al.) beschrieben und analog wie dort durchführbar.

Zur Herstellung von unsymmetrischen Verbindungen der Formel II', worin A₁ und A₂ und/oder Ar₁ und Ar₂ verschieden sind, können beispielsweise Verbindungen der Formel V mit zwei unterschiedlichen Verbindungen der Formel VI - z.B. stufenweise - umgesetzt und die gewünschten Verbindungen der Formel II' durch eine chromatographische Trennung, z.B. an Kieselgel, isoliert werden.

Weiterhin können zur Herstellung von Verbindungen der Formel II', worin die Reste die genannten Bedeutungen ausser aus A₁ und A₂ zusammen gebildetem und durch Substituenten ausser Niederalkyl oder Hydroxy substituiertes Niederalkylen haben, beispielsweise Verbindungen der Formel II', worin anstelle von A₁ und A₂ Wasserstoff steht, mit einem Reagens der Formel VII,

Alk-L (VII)

worin Alk substituiertes Niederalkyl, substituiertes Niederalkenyl, substituiertes Niederalkinyl oder Heterocyclylniederalkyl, wie oben bei der Definition von A₁ und/oder A₂ genannt, bedeutet, wobei L im Falle von substituiertem Niederalkenyl oder substituiertem Niederalkinyl vorzugsweise an ein Kohlenstoffatom gebunden ist, von dem keine Mehrfachbindung ausgeht, und so gewählt ist, dass die Umsetzung durch Substitution schneller erfolgt als die Addition an die Mehrfachbindung; und L eine nukleofuge Gruppe, vorzugsweise aliphatisch - oder aromatisch - substituiertes Sulfonyloxy, z.B. Methansulfonyloxy oder p-Toluolsulfonyloxy (Tosyloxy), Halogen, insbesondere Chlor, Brom oder Jod, oder Cyano, oder im Falle dass der Rest Alk- 2 oder mehr Kohlenstoffatome enthält, Oxa (-O-) oder Thia (-S-), welches an zwei vizinale Kohlenstoffatome gebunden ist (wobei ein Oxiran oder Thiiran gebildet wird, welches bei Alkylierung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, oder einem Harnstoffderivat, wie 1,3-Dimethyl-3,4,5,6-tetrahydro-2(1H)-pyrimidinon (DMPU), erfolgt, und bei anschliessender Hydrolyse unter Entstehung einer 2-Hydroxy- oder 2-Mercaptoniederalkylgruppe reagiert), oder Aza (-NH-), welches an zwei vizinale Kohlenstoffatome gebunden ist (wobei ein Aziran gebildet wird, welches bei Umsetzung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, oder einem Harnstoffderivat, wie 1,3-Dimethyl-3,4,5,6-tetrahydro-2(1H)-pyrimidinon (DMPU), erfolgt, und bei anschliessender Hydrolyse unter Bildung einer 2-Aminoniederalkylgruppe reagiert), umgesetzt werden, wobei funktionelle Gruppen in Ausgangsmaterialien, die nicht an der Reaktion teilnehmen sollen, frei oder in geschützter Form vorliegen, und vorhandene Schutzgruppen auf geeigneten Reaktionsstufen erforderlichenfalls abgespalten werden.

Die Reaktion findet vorzugsweise in Gegenwart einer starken Base statt, wie einem Alkalimetallhydrid, z. B. Natriumhydrid, einem Alkalimetallamid, wie Natriumamid, oder einem Alkalimetall-diniederalkylamid, wie Lithiumdiispropylamid, insbesondere von Natriumhydrid oder Natriumamid, welches z.B. als Dispersion in Öl zugesetzt werden kann, insbesondere unter Verwendung eines gegenüber der molaren Menge der Verbindung der Formel II', worin anstelle von A₁ und A₂ je ein Wasserstoffatom steht, äquimolaren Menge oder eines Überschusses der Base, beispielsweise der 1- bis 5-fachen molaren Menge, vor allem der 1- bis 2-fachen molaren Menge, bei Temperaturen zwischen Raumtemperatur und der Rückflusstemperatur des Reaktionsgemisches, insbesondere zwischen etwa 80 und etwa 100 °C, in aprotischen, insbesondere polaren Lösungsmitteln, wie Säureamiden, z.B. Dimethylformamid, 1,3-Dimethyl-3,4,5,6-tetrahydro-2(1H)-pyrimidinon (DMPU), oder Hexamethylphosphorsäuretriamid, oder Gemischen von solchen Lösungsmitteln, in An- oder Abwesenheit von Schutzgas, wie Argon oder Stickstoff, wobei im Falle der Verwendung von Alkalimetallamiden als Basen entstehender Ammoniak durch Anlegen eines Vakuums, z. B. von 0,1 bis 100, insbesondere von 0,5 bis 10 Torr, entfernt wird.

Sollen Verbindungen der Formel I hergestellt werden, worin nur einer der Reste A₁ und A₂ die oben genannten Bedeutungen ausser Wasserstoff hat, während der andere Wasserstoff bedeutet, wird die Reaktion mit einer molaren Menge der Verbindung der Formel VII durchgeführt, welche vorzugsweise der 0,2- bis 2-fachen molaren Menge, z. B. der 1- bis 1,6-fachen molaren Menge, gegenüber der molaren Menge der Verbindung der Formel II', worin anstelle von A₁ und A₂ Wasserstoff steht, entspricht. Sollen beide Wasserstoffatome in der Verbindung der Formel II', worin anstelle von A₁ und A₂ Wasserstoff steht, durch die bei der Definition von A₁ und A₂ genannten Reste ausser Wasserstoff ersetzt werden, so wird vorzugsweise ein Überschuss, z. B. ein 2- bis 10-, insbesondere ein etwa zwei- bis dreifacher Überschuss der Verbindung der Formel VII eingesetzt.

Die Verbindungen der Formeln V, VI und VII sind bekannt, kommerziell erhältlich oder nach an sich bekannten Verfahren herstellbar.

In den Verbindungen der Formel VII ist Alk vorzugsweise substituiert durch geschütztes Amino, insbesondere als geschütztes Aminoniederalkyl, z.B. Phthalimidoniederalkyl, wie Phthalimidopropyl, oder Niederalkanoylaminoniederalkyl, wie 3-Acetylaminopropyl, beispielsweise durch Niederalkoxycarbonyl, wie tert-Butyloxycarbonyl, geschütztes und im Mononiederalkylrest unsubstituiertes oder wie oben substituiertes (und dann erforderlichenfalls an den Substituenten geschütztes) Mononiederalkylamino, insbesondere in entsprechend geschütztem und substituiertem Mononiederalkylaminoniederalkyl; in den beiden N-Niederalkylresten unsubstituiertes oder wie oben definiert substituiertes (und dann erforderlichenfalls an Substituenten geschütztes) Diniederalkylamino, insbesondere in entsprechendem unsubstituiertem oder substituiertem Diniederalkylaminoniederalkyl; N-geschütztes Cycloalkylamino, insbesondere in N-geschütztem Cycloalkylaminoniederalkyl; N-geschütztes Phenylniederalkylamino, insbesondere in N-geschütztem Phenylniederalkylaminoniederalkyl; N-geschütztes Phenylamino, insbesondere in N-geschütztem Phenylaminoniederalkyl; Acylamino, wie in Acylaminoniederalkyl; geschütztes Hydroxy, insbesondere in Hydroxyniederalkyl, worin die Hydroxygruppe geschützt vorliegt; Niederalkoxy, worin der endständige Niederalkylrest unsubstituiert oder wie oben substituiert (und dann erforderlichenfalls mit geschützten Substituenten) vorliegt, wie in entsprechendem substituiertem geschütztem oder unsubstituiertem Niederalkoxyniederalkyl; Phenylniederalkoxy, insbesondere in Phenylniederalkoxyniederalkyl; Acyloxy, insbesondere in Acylniederalkoxyniederalkyl; geschütztes Mercapto, insbesondere in Mercaptoniederalkyl, worin die Mercaptogruppe in geschützter Form vorliegt; Niederalkylthio, worin der endständige Niederalkylrest unsubstituiert oder wie oben substituiert (und dann erforderlichenfalls mit geschützten Substituenten) vorliegt, insbesondere in entsprechend substituiertem geschütztem oder unsubstituiertem Niederalkylthioniederalkyl; Phenylniederalkylthio, wie in Phenylniederalkylthioniederalkyl; Acylthio, wie in Acylthioniederalkyl; geschütztes Carboxy, wie in geschütztem Carboxyniederalkyl; verestertes Carboxy, wie in verestertem Carboxyniederalkyl; Cyano, wie in Cyanoniederalkyl; Oxo, wie in Oxoniederalkyl (erforderlichenfalls geschützt durch Acetalbildung, z. B. mit Niederalkanolen, insbesondere mit Ethan-1,2-diol, wobei die Schutzgruppe auf der gewünschten Stufe durch Hydrolyse in Gegenwart einer Säure, wie Essigsäure oder Schwefelsäure, abgespalten werden kann); Thioxo, insbesondere in Thioxoniederalkyl (erforderlichenfalls geschützt durch Thioacetalbildung, z. B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann); und/oder Heterocyclyl, wie in erforderlichenfalls geschütztem Heterocyclylniederalkyl.

Benutzt man anstelle einer Verbindung der Formel VII eine entsprechende Verbindung, worin am Rest Alk, welcher hier wie oben für A₁ und A₂ definiertes substituiertes Niederalkyl bedeutet, zusätzlich zu einem der genannten nukleofugen Reste L ein Halogenatom vorliegt, so kann man durch Eliminierung von Halogenwasserstoff in Gegenwart der zu Reaktion verwendeten Base, z. B. von Natriumamid, welche z.B. im Überschuss eingesetzt wird, unter den oben genannten Reaktionsbedingungen die entsprechende substituierte Niederalkenylverbindung erhalten. Analog kann man entsprechende substituierte Niederalkinylreste aus Analogen der Verbindung der Formel VII erhalten, worin zusätzlich zu einem der genannten nukleofugen Reste L zwei weitere Halogenatome vorliegen.

In erhältlichen Verbindungen der Formel II' können Carboxygruppen, die in A₁ und/oder A₂ als Substituent vorliegen, in Carbamoyl-, N-Mono- oder N,N-Diniederalkylcarbamoyl-, N-Hydroxycarbamoyl- oder N-Phenylcarbamoylgruppen (auch in N-Aryl- und N-Arylniederalkylcarbamoylgruppen) umgewandelt werden, beispielsweise durch Umsetzung mit Ammoniak, einem Niederalkylamin oder einem Diniederalkylamin, Hydroxylamin oder Phenylamin, in Gegenwart eines Kondensationsmittels, z.B. einem Carbodiimid, wie Dicyclohexylcarbodiimid oder einem polaren Derivat davon, in polaren organischen Lösungsmitteln, wie Ethanol, oder in Gegenwart von einem N.N'-Carbonyldiazolid, wie N.N'-Dicarbonylimidazol (vgl. H. A. Staab, Angew. Chem. 74, 407-423 (1962)) in inerten organischen Lösungsmitteln, z.B. chlorierten Kohlenwasserstoffen, wie Methylenchlorid oder Chloroform, oder in Ethern, wie Diethylether, Tetrahydrofuran oder Dioxan, über das entsprechende Carbonsäureazolid. Man erhält so die entsprechenden Verbindungen der Formel II', worin A₁ und/oder A₂ Carbamoyl, N-Mono- oder N,N-Diniederalkylcarbamoyl, N-Hydroxycarbamoyl oder N-Phenylcarbamoyl enthält. Entsprechende Thiocarbamoylsubstituenten in A₁ und A₂ können aus Carboxy durch Umwandlung in ein Carbonylhalogenid, z. B. mit einem anorganischen Säurehalogenid, wie Phosphortrichlorid, Phosphorpentachlorid oder Thionylchlorid, oder einem organischen Säurehalogenid, wie Oxalyldichlorid, und anschliessende Umsetzung z.B. mit Phosphorpentachlorid, Schwefelwasserstoff und Ammoniak oder den genannten Aminen erhalten werden.

In Verbindungen der Formel II', worin A₁ und/oder A₂ z.B. ein Hydroxyniederalkyl bedeuten, kann durch nukleophile Substitution die Hydroxygruppe in eine Hydrazino-, durch Niederalkyl, Aryl und/oder Arylniederalkyl N-substituierte Hydrazinogruppe, eine Guanidinogruppe oder eine durch Niederalkyl, Aryl und/oder Arylniederalkyl N-substituierte Guanidinogruppe umgewandelt werden. Beispielsweise kann eine Hydroxyverbindung durch Umsetzung mit aromatischen Sulfonsäuren oder aktivierten Derivaten davon, wie den entsprechenden aromatischen Sulfonsäurehalogeniden, z. B. Toluolsulfonsäurehalogeniden, wie Toluolsulfonsäurechlorid, in Ab- oder vorzugsweise Anwesenheit von geeigneten Basen, z.B. tertiären Stickstoffbasen, wie Triethylamin oder N-Methylmorpholin, in den entsprechenden Ester der aromatischen Sulfonsäure überführt werden und dann dieser Ester mit Hydrazin, Guanidin, den entsprechend substituierten Derivaten, oder Salzen davon, wobei auch Schutzgruppen vorliegen können, unter den Bedingungen der nukleophilen Substitution umgesetzt werden, vorzugsweise in Gegenwart von organischen Lösungsmitteln, z.B. Alkoholen, wie Methanol, Ethanol oder Trifluorethanol, Ketonen, wie Aceton, Nitrilen, wie Acetonitril, Estern, wie Essigsäureethylester, Ethern, wie Diethylether, Ethylenglykoldimethylether, Tetrahydrofuran oder Dioxan, Säureamiden, wie Dimethylformamid, Bisalkansulfinen, wie Dimethylsulfoxid, Arylalkoholen, wie Phenol, oder auch von Wasser, oder Gemischen dieser Lösungsmittel, gegebenenfalls (z.B. zur Umsetzung von an Aryl gebundenem Stickstoff) in inerten organischen Lösungsmitteln, wie Dimethylformamid oder 1,3-Dimethyl-3,4,5,6-tetrahydro-2(1H)-pyrimidinon, unter Zugabe starker Basen, wie Natriumamid oder Natriumhydrid. Erforderlichenfalls werden vorhandene Schutzgruppen abgespalten. Man erhält Verbindungen der Formel II', worin A₁ und/oder A₂ Substituenten ausgewählt aus Hydrazino, an einem oder beiden Stickstoffatomen durch Niederalkyl, Aryl oder Arylniederalkyl substituiertes Hydrazino, Guanidino oder an einem, zwei oder allen drei Stickstoffatomen durch Niederalkyl, Aryl oder Arylniederalkyl substituiertes Guanidino enthält. Diejenigen Verbindungen der Formel II', worin Substituenten ausgewählt aus Hydrazino und am endständigen N-Atom durch Niederalkyl, Aryl und/oder Arylniederalkyl substituiertem Hydrazino vorliegen, kann man auch erhalten ausgehend von einer entsprechenden Oxoverbindung der Formel II' durch deren Umsetzung mit Stickstoffbasen wie Hydrazin oder durch Niederalkyl, Aryl und/oder Arylniederalkyl an einem der beiden N-Atome bis zu zweifach substituiertes Hydrazin, wie weiter unten für die Umsetzung einer Oxoverbindung mit Stickstoffbasen beschrieben, und anschliessende Reduktion einer erhaltenen entsprechenden Iminoverbindung, vorzugsweise durch katalytische Hydrierung mit selektiven Hydrierkatalysatoren, insbesondere in Gegenwart von Palladium auf festen Trägermaterialien, z.B. auf Kohle, in polaren organischen oder organisch-wässrigen Lösungsmitteln oder Lösungsmittelgemischen, insbesondere in Ethern, z.B. cyclischen Ethern, wie Tetrahydrofuran oder Dioxan, oder in Alkoholen, wie Niederalkanolen, z.B. Methanol oder Ethanol, oder Gemischen davon, z.B. Methano/Tetrahydrofuran-Gemischen, bei Temperaturen zwischen -20 und 60 °C, vorzugsweise zwischen 0 und 40 °C, z.B. etwa bei Raumtemperatur.

In Verbindungen der Formel II', worin A₁ und/oder A₂ z.B. ein Cyanoniederalkyl bedeuten, können Cyanogruppen z.B. durch partielle Hydrolyse, im Sinne einer Graf-Ritter-Reaktion, oder über Imino-niederalkylester-Salze in Carbamoyl- oder N-Niederalkylcarbamoylgruppen in Verbindungen der Formel II' überführt werden. Die Bedingungen bei der Hydrolyse des Cyano-Zwischenproduktes können so gewählt werden, dass die Reaktion auf der Stufe des Amids abgebrochen wird. Zu diesem Zweck ist insbesondere die Hydrolyse mit Säuren geeignet, wobei z.B. 80%-ige Schwefelsäure (unter Erwärmen), Polyphosphorsäure (bei 110-150 °C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen. Auch mit Hilfe der Graf-Ritter-Reaktion gelingt die Herstellung N-monoalkylierter Amide der Formel II' aus den entsprechenden Nitrilen. Hierzu setzt man die Nitrile in Gegenwart einer starken Säure, vornehmlich 85-90%-iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen oder Alkoholen. Die Imino-niederalkylester der Formel II' erhält man z.B. durch säurekatalysierte Anlagerung von Alkoholen an die Nitrilvorläufer. Diese Anlagerung kann alternativ durch Basen, z.B. Alkoholate wie Natriummethoxid, katalysiert werden. Setzt man anstelle von Alkoholen entsprechende Mercaptane ein, beispielsweise in Gegenwart von Stickstoffbasen, wie Triethylamin oder N-Methylmorpholin, so erhält man die entsprechenden Imino-niederalkylthioester. Aus den Iminoniederalkylestern erhält man die Carbamoylderivate, aus den Imino-niederalkylthioestern die entsprechenden Thiocarbamoylderivate im Sinne einer Pinner-Spaltung durch thermischen Zerfall der Iminoester-Salze bei Temperaturen oberhalb von etwa 80 °C. Die Thiocarbamoylverbindungen können auch direkt erhalten werden durch Umsetzung von Cyanogruppen mit Schwefelwasserstoff analog der partiellen Hydrolyse, beispielsweise in Gegenwart von tertiären Aminen, wie Triethylamin.

Verbindungen der Formel II', worin A₁ und/oder A₂ z.B. ein Amidinoniederalkyl oder ein an einem Stickstoffatom durch bis zu zwei Reste ausgewählt aus Niederalkyl, Aryl und Arylniederalkyl substituiertes Amidinoniederalkyl bedeuten, lassen sich durch Umsetzung einer entsprechenden Imino-niederalkylester- oder Imino-niederalkylthiolestervorstufe (als Säureadditionssalz, z.B. -(C=NH)-OC₂H₅ . HCl bzw. -C(=NH)-SC₂H₅ . HI, wie oben beschrieben hergestellt aus einer entsprechenden Cyanoverbindung) herstellen, indem mit Ammoniak oder den entsprechenden Aminen umgesetzt wird. Die Cyanovorstufen können beispielsweise auch durch Umsetzung mit einem Alkalimetallamid, oder durch Reaktion mit einem primären oder sekundären Ammoniumsalz, z.B. einem primären oder sekundären Ammoniumhalogenid, in die entsprechenden freien, mono- oder disubstituierten Amidine überführt werden. Verbindungen der Formel II', worin A₁ und/oder A₂ ein an allen beiden Stickstoffatomen durch Aryl, Arylniederalkyl oder Niederalkyl substituiertes Amidino enthalten, lassen sich aus den (analog wie oben für Niederalkylcarbamoyl beschrieben herstellbaren) Verbindungen herstellen, worin in Formel II' anstelle von Carbamoyl in A₁ und/oder A₂ durch Niederalkyl, Aryl oder Arylniederalkyl N-substituiertes Carbamoyl als Substituent enthalten ist, z. B. durch Umsetzung mit POCl₃ oder PCl₅ in die entsprechenden Imidsäurechloride (z.B. -(C=NH-niederalkyl)-Cl), welche nach Reaktion mit Ammoniak bzw. einem primären oder sekundären Amin substituierte Amidine der Formel II' ergeben (vgl. Chem. Abstr. 81, 91186a (1974)).

In erhältlichen Verbindungen der Formel II' können Aminogruppen, die in A₁ und/oder A₂ als Substituent vorliegen, in Ureido- oder an einem oder beiden Stickstoffatomen durch je bis zu einen Rest ausgewählt aus Niederalkyl, Aryl und Arylniederalkyl substituiertes Ureido umgewandelt werden, indem man Verbindungen der Formel II', in denen z.B. A₁ und/oder A₂ Aminoniederalkyl oder N-Mononiederalkylaminoniederalkyl bedeuten, oder in denen anstelle von einem oder beiden der Reste A₁ und A₂ ein Arylaminoniederalkyl und/oder ein Arylniederalkylaminoniederalkyl stehen (herstellbar z. B. durch Umsetzung von Verbindungen der Formel II', worin anstelle von A₁ oder A₂ Wasserstoff steht, mit Analogen der Verbindungen der Formel VII, worin ausser der nukleofugen Gruppe L noch eine weitere nukleofuge Gruppe, z.B. Halogen, enthalten ist, unter Bedingungen analog der Umsetzung von Verbindungen der Formel II', worin anstelle von A₁ und/oder A₂ Wasserstoff steht, unter erneuter Anwendung analoger Bedingungen und Substitution des zweiten nukleofugen Restes entweder mit einem Arylamin oder einem Arylniederalkylamin, oder aus Verbindungen der Formel II', worin A₁ und/oder A₂ ein Hydroxyniederalkyl bedeuten, indem man die Hydroxygruppe in einen nukleofugen Rest, z.B. durch Behandlung mit einem aromatischen Sulfonsäurehalogenid, wie Toluolsulfonsäurechlorid, überführt und diesen dann mit einem Arylamin oder einem Arylniederalkylamin unter Bedingungen analog der Umsetzung von Verbindungen der Formel II', worin anstelle von A₁ und/oder A₂ Wasserstoff steht, mit Verbindungen der Formel VII umsetzt), mit einem Niederalkyl-, Aryl- oder Arylniederalkylisocyanat oder N-geschütztem Isocyanat (z. B. Benzylisocyanat) umsetzt, vorzugsweise in einem Ether, z.B. einem cyclischen Ether, wie Tetrahydrofuran, bei bevorzugten Temperaturen zwischen -20 und 60 °C, insbesondere etwa bei Raumtemperatur, wobei funktionelle Gruppen, die nicht an der Reaktion teilnehmen sollen, erforderlichenfalls geschützt sind, und vorhandene Schutzgruppen auf einer geeigneten Reaktionsstufe abspaltet.

Analog lassen sich in Verbindungen der Formel II' Aminogruppen, die in A₁ und/oder A₂ als Substituent vorliegen, in Thioureido- oder an einem oder beiden Stickstoffatomen durch je bis zu einen Rest ausgewählt aus Niederalkyl, Aryl oder Arylniederalkyl substituiertes Thioureido umwandeln, indem man anstelle der Isocyanate entsprechende Thioisocyanate verwendet.

Verbindungen der Formel II', worin A₁ und/oder A₂ z.B. ein am endständigen Stickstoff durch 2 Reste ausgewählt aus Niederalkyl, Aryl und Arylniederalkyl substituiertes Ureidoniederalkyl bedeuten, können beispielsweise hergestellt werden, indem man entsprechende Aminoniederalkylverbindungen der Formel II' mit Phosgen oder Analogen davon, z. B. N.N'-Carbonyldiazoliden, wie N.N'-Carbonyldiimidazol (vgl. H. A. Staab, Angew. Chem. 74, 407-423 (1962)), umsetzt und die entstehenden Chlorocarbonyl- oder Azolidocarbonyl-aminoverbindungen dann mit durch 2 Reste ausgewählt aus Niederalkyl, Aryl und Arylniederalkyl substituiertem Ammoniak umsetzt, oder umgekehrt die entsprechenden Aminoniederalkylverbindungen der Formel II' mit dem Reaktionsprodukt von durch 2 Reste ausgewählt aus Niederalkyl, Aryl und Arylniederalkyl substituiertem Ammoniak mit Phosgen oder Analogen davon, z.B. den N.N'-Carbonyldiazoliden, wie N.N'-Carbonyldiimidazol, umsetzt unter Erhalt der entsprechend substituierten Ureidoverbindungen. Die Reaktionen werden vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und der Rückflusstemperatur, insbesondere zwischen 0 und 30 °C, durchgeführt.

Verbindungen der Formel II', worin A₁ und/oder A₂ z.B. ein am endständigen Stickstoff durch 2 Reste ausgewählt aus Niederalkyl, Aryl und Arylniederalkyl substituiertes Thioureidoniederalkyl enthalten, können auf analoge Weise beispielsweise hergestellt werden, indem man entsprechende Aminoniederalkylverbindungen der Formel II' mit Thiophosgen oder Analogen davon, z. B. N.N'-Thiocarbonyldiazoliden, wie N.N'-Thiocarbonyldiimidazol (vgl. H. A. Staab, Angew. Chem. 74, 407-423 (1962), umsetzt und die entstehenden Chlorothiocarbonyl- oder Azolido-thiocarbonylaminoverbindungen dann mit durch 2 Reste ausgewählt aus Niederalkyl, Aryl und Arylniederalkyl substituiertem Ammoniak umsetzt, oder umgekehrt die entsprechenden Aminoniederalkylverbindungen der Formel II' mit dem Reaktionsprodukt von durch 2 Reste ausgewählt aus Niederalkyl, Aryl und Arylniederalkyl substituiertem Ammoniak mit Thiophosgen oder Analogen davon, z.B. den N.N'-Thiocarbonyldiazoliden, wie N.N'-Thiocarbonyldiimidazol, umsetzt.

In Verbindungen der Formel II' kann z.B. ein Hydroxyniederalkyl A₁ und/oder A₂ zur entsprechenden Oxo-Verbindung oxidiert werden. Im Falle von primären Alkoholen ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle von sekundären Alkoholen kann ausserdem mit weniger selektiven Oxidationsmitteln, wie Chromsäure, Dichromat/Schwefelsäure, Dichromat/Eisessig, Salpetersäure, Braunstein, Selendioxid oder Dimethylsulfoxid in Gegenwart von Oxalylchlorid, in Wasser, wässrigen oder organischen Lösungsmitteln, wie halogenierten Kohlenwasserstoffen, z.B. Methylenchlorid, oder Carbonsäureamiden, wie Dimethylformamid, oxidiert werden, vorzugsweise bei Temperaturen zwischen - 50 °C und Rückflusstemperatur, insbesondere zwischen - 10 und 50 °C. Man erhält Verbindungen der Formel II', worin der Rest A₁ und/oder A₂ Oxo trägt.

Verbindungen der Formel II', worin A₁ und/oder A₂ Imino, Niederalkylimino, Acylimino, Hydroxyimino, Niederalkoxyimino, Hydrazono, N-Mono- oder N,N-Diniederalkylhydrazono und/oder N-Acylhydrazono enthalten, z.B. als Subsituenten in einem substituierten Niederalkyl, können aus entsprechenden Oxo-Verbindungen der Formel II' hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in welchem die (vorzugsweise wie zuletzt beschrieben durchgeführte) Oxidation einer Hydroxy- zu einer Oxoverbindung durchgeführt wird.

So kann eine Oxoverbindung durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in ein entsprechendes Iminoderivat überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; wobei in Wasser (in An- oder Abwesenheit von Tensiden), einem wässrigen Lösungsmittelgemisch, wie einem Gemisch von Wasser mit einem oder mehreren Alkoholen, z. B. Methanol, Ethanol oder Isopropanol, Diniederalkylsulfoxiden, wie Dimethylsulfoxid, oder Diniederalkylniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen - 20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur; umgesetzt wird.

Solchermassen erhältliche Oxoverbindungen der Formel II' können in die entsprechenden Thioxoverbindungen überführt werden, beispielsweise durch Umsetzung mit Phosphorpentasulfid oder vorzugsweise Phosphorpentasulfid-Ersatzstoffen, wie Laweson's Reagenz (= 2,4-Bis-(4-methoxyphenyl)-2,4-dithioxo-1,3,2,4-dithiaphosphetan) wobei die Reaktion in inerten organischen Lösungsmitteln, beispielsweise in einem halogenierten Kohlenwasserstoff, wie Dichlormethan, bei Temperaturen von 30 °C bis zur Rückflusstemperatur, insbesondere unter Rückfluss, durchgeführt wird.

Verbindungen der Formel II', worin A₁ und/oder A₂ Acyliminosubstituenten enthalten, z.B. als Substituenten in einem substituierten Niederalkyl, können aus entsprechenden Iminoausgangsmaterialien erhalten werden durch deren Umsetzung mit einer freien Säure, welche den einzuführenden Acylrest enthält, beispielsweise in Gegenwart von Kondensationsmitteln, wie Carbodiimiden, z. B. Dicyclohexylcarbodiimid, oder mit einem aktivierten Säurederivat davon, z. B. einem Carbonsäurehalogenid, gegebenenfalls in Gegenwart einer geeigneten Base, z.B. eines tertiären Amins, wie bereits definiert, vorzugsweise unter Feuchtigkeitsausschluss.

Verbindungen der Formel II', worin A₁ und/oder A₂ Niederalkylthioiminosubstituenten tragen, z.B. an substituiertem Niederalkyl, können vorzugsweise hergestellt werden, indem man entsprechende Iminoausgangsmaterialien der Formel II' mit Niederalkylsulfenylhalogeniden (herstellbar z. B. aus Sulfensäuren mit Halogenwasserstoff oder durch Chlorolyse, Bromolyse oder Jodolyse von entsprechenden Organo-Schwefel-Verbindungen, wobei die Herstellung auch in situ erfolgen kann), insbesondere Niederalkylsulfenyl-halogeniden, wie Methylsulfenylchlorid, umsetzt, vorzugsweise unter Verwendung der Salze der Iminoverbindungen oder in Gegenwart von Alkalihydroxiden, wie Natrium- oder Kaliumhydroxid, vorzugsweise in organischen Lösungsmitteln, z.B. Kohlenwasserstoffen, wie Heptan, Ethern, wie Diethylether, Dioxan oder Tetrahydrofuran, oder Carbonsäureamiden, wie Dimethylformamid, bei bevorzugten Temperaturen zwischen 0 °C und der Rückflusstemperatur, insbesondere zwischen 0 und 30 °C.

An dieser Stelle sei ausdrücklich vermerkt, dass Verbindungen der Formel II', worin A₁ und/oder A₂ zusätzlich zu oder anstelle von substituiertem Niederalkyl aus substituiertem Niederalkenyl und substituiertem Niederalkinyl ausgewählt sind, auch wenn diese in den Abschnitten für die Herstellung von Verbindungen der Formel II' nicht explizit erwähnt sind, in sinnvoller und zweckmässig Weise mit analogen Herstellungsverfahren dargestellt werden können, wie für die Verbindungen der Formel II' mit substituierten Niederalkylresten beschrieben.

Verbindungen der Formel II', worin A₁ und/oder A₂ z.B. Heterocyclylniederalkyl bedeuten, können aus entsprechenden Verbindungen der Formel II', worin wenigstens einer der Substituenten A₁ und A₂ Wasserstoff bedeutet oder aus entsprechenden Analogen, worin anstelle von A₁ und A₂ jeweils Wasserstoff steht, erhalten werden, vorzugsweise durch Umsetzung mit Verbindungen der Formel VIII,

Heterocyclylniederalkyl-L' (VIII)

worin Heterocyclylniederalkyl wie oben definiert ist und L' eine nukleofuge Gruppe bedeutet, wie oben für L bei Verbindungen der Formel VII definiert, unter nukleophiler Substitution der nukleofugen Gruppe L'. Die Reaktionsbedingungen entsprechen dabei vorzugsweise den bei der Alkylierungsreaktion mit Verbindungen der Formel VII genannten Bedingungen in Gegenwart einer starken Base.

Zur Herstellung von Verbindungen der Formel II', worin A₁ und A₂ zusammen durch Substituenten ausser Niederalkyl oder Hydroxy substituiertes Niederalkylen bedeuten, werden beispielsweise Verbindungen der Formel II', worin anstelle von A₁ und A₂ Wasserstoff steht, mit einem alkylierenden Reagens der Formel IX,

L₁-B-L₂ (IX)

worin B ein substituiertes Niederalkylen als bivalentes Radikal (beispielsweise substituiert durch die bei der Definition von aus A₁ und A₂ zusammen gebildetem substituiertem Niederalkylen genannten Substituenten und zusätzlich Niederalkyl und/oder Hydroxy, welche als Substituenten in Zwischenprodukten möglich sind) bedeutet, und worin L₁ und L₂ unabhängig voneinander eine nukleofuge Gruppe bedeuten, wie oben für L bei der Definition von Verbindungen der Formel VII beschrieben, umgesetzt, wobei erforderlichenfalls solche funktionelle Gruppen in den Ausgangsmaterialien, die nicht an der Reaktion teilnehmen sollen, durch Schutzgruppen geschützt sind, die auf einer geeigneten Reaktionsstufe entfernt werden. Die bevorzugten Schutzgruppen, ihre Einführung und ihre Abspaltung wurden oben erwähnt.

L₁ ist vorzugsweise eine nukleofuge Gruppe, insbesondere aliphatisch - oder aromatisch - substituiertes Sulfonyloxy, z.B. Methansulfonyloxy oder p-Toluolsulfonyloxy (Tosyloxy), Halogen, wie Chlor, Brom oder Jod, oder Cyano, während L₂, falls ein Niederalkylenrest B zwei oder mehr Kohlenstoffatome enthält, Oxa (-O-) oder Thia (-S-), welches an zwei vizinale Kohlenstoffatome gebunden ist (wobei ein Oxiran oder Thiiran gebildet wird, welches bei Alkylierung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, erfolgt, und bei anschliessender Hydrolyse unter Entstehung einer 1-Hydroxy- oder 1-Mercaptoniederalkylgenruppe reagiert), oder Aza (-NH-), welches an zwei vizinale Kohlenstoffatome gebunden ist (wobei ein Aziran gebildet wird, welches bei Alkylierung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, erfolgt, und bei anschliessender Hydrolyse unter Bildung einer 1-Aminoniederalkylengruppe reagiert), bedeuten kann.

Es ist auch möglich, dass L₁ und L₂ zusammen am endständigen Kohlenstoff von B gebundenes Oxo bedeuten (die Verbindung der Formel IX ist dann ein Aldehyd), oder jeweils Niederalkoxy bedeuten (die Verbindung der Formel IX ist dann ein Acetal). Vorzugsweise ist B in Verbindungen der Formel II dann augewählt aus verestertem Carboxymethylen, wie Niederalkoxycarbonylmethylen, oder Niederalkanoylmethylen. Die Umsetzung findet beispielsweise in Gegenwart von Säuren, z.B. Halogenwasserstoffsäuren, wie Chlorwasserstoffsäure, oder vorzugsweise in Gegenwart von Lewis-Säuren, insbesondere SnCl₂ (z.B. als Hydrat), in geeigneten Lösungsmitteln oder Lösungsmittelgemischen, z.B. Ethern, wie Diniederalkoxyniederalkanen, insbesondere in 1,2-Dimethoxyethan, bei bevorzugten Temperaturen zwischen 0 und 50 °C, z.B. etwa bei Raumtemperatur, statt, erforderlichenfalls unter Wasserzusatz.

Die Umsetzung erfolgt vorzugsweise unter den Reaktionsbedingungen, die für die Umsetzung von Verbindungen der Formel II', worin anstelle von A₁ und A₂ jeweils ein Wasserstoffatom steht, mit Verbindungen der Formel VII als bevorzugt beschrieben sind.

Die Verbindung der Formel IX wird vorzugsweise in der äquimolaren Menge oder im Überschuss gegenüber der Verbindung der Formel II' eingesetzt, worin anstelle von A₁ und A₂ je ein Wasserstoffatom steht, insbesondere in der 1- bis 3-fachen molaren Menge, z.B. der 1- bis 1,5-fachen molaren Menge, wie der etwa 1,2-fachen Menge. Die eingesetzte starke Base wird vorzugsweise im Überschuss gegenüber der Verbindung der Formel II' eingesetzt, worin anstelle von A₁ und A₂ je ein Wasserstoffatom steht, insbesondere in der 2- bis 10-fachen molaren Menge, z.B. in der 2- bis 3-fachen molaren Menge.

Die Reaktion kann so geführt werden, dass die nukleofugen Gruppen L₁ und L₂ praktisch gleichzeitig in einem Ansatz substituiert werden, oder die nukleofugen Gruppen L₁ und L₂ können sukzessive substituiert werden.

In den Verbindungen der Formel IX ist B vorzugsweise durch einen oder mehrere der folgenden Substituenten substituiertes Niederalkylen: geschütztes Amino oder Aminoniederalkyl, z.B. Phthalimido oder Phthalimidoniederalkyl, wie Phthalimidopropyl oder, beispielsweise durch Niederalkoxycarbonyl, wie tert-Butyloxycarbonyl, geschütztes und im Mononiederalkylrest unsubstituiertes oder wie oben substituiertes (und dann erforderlichenfalls an den Substituenten geschütztes) Mononiederalkylamino oder Mononiederalkylamino-niederalkyl, in den beiden N-Niederalkylresten unsubstituiertes oder wie oben definiert substituiertes (und dann erforderlichenfalls an Substituenten geschütztes) Diniederalkylamino oder Diniederalkylaminoniederalkyl, N-geschütztes Cycloalkylamino oder Cycloalkylaminoniederalkyl, N-geschütztes Phenylniederalkylamino oder Phenylniederalkylaminoniederalkyl, N-geschütztes Phenylamino oder Phenylaminoniederalkyl, Acylamino oder Acylaminoniederalkyl, Hydroxy (zusätzlich zu der Definition von aus A₁ und A₂ gebildetem substituiertem Niederalkylen in Zwischenprodukten möglich) oder Hydroxyniederalkyl, wobei die Hydroxygruppe geschützt vorliegt, Niederalkoxy oder Niederalkoxyniederalkyl, worin der endständige Niederalkylrest unsubstituiert oder wie oben substituiert (und dann erforderlichenfalls mit geschützten Substituenten) vorliegt, Phenylniederalkoxy oder Phenylniederalkoxyniederalkyl, Acyloxy oder Acyloxyniederalkyl, Mercapto oder Mercaptoniederalkyl, worin die Mercaptogruppe in geschützter Form vorliegt, Niederalkylthio oder Niederalkylthioniederalkyl, worin der endständige Niederalkylrest unsubstituiert oder wie oben substituiert (und dann erforderlichenfalls mit geschützten Substituenten) vorliegt, Phenylniederalkylthio oder Phenylniederalkylthioniederalkyl, Acylthio oder Acylthioniederalkyl, geschütztes Carboxy oder Carboxyniederalkyl, verestertes Carboxy oder Carboxyniederalkyl, Cyano oder Cyanoniederalkyl, Oxo oder Oxoniederalkyl (erforderlichenfalls geschützt durch Acetalbildung, z. B. mit Niederalkanolen, insbesondere mit Ethan-1,2-diol, wobei die Schutzgruppe auf der gewünschten Stufe durch Hydrolyse in Gegenwart einer Säure, wie Essigsäure oder Schwefelsäure, abgespalten werden kann), oder Thioxo oder Thioxoniederalkyl (erforderlichenfalls geschützt durch Thioacetalbildung, z. B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann).

In erhältlichen Verbindungen der Formel II' kann Carboxy (in einer Carboxy- oder Carboxyniederalkylgruppe, die in einem aus A₁ und A₂ zusammen gebildetem substituiertem Niederalkylen vorliegt) in Carbamoyl, N-Mono- oder N,N-Diniederalkylcarbamoyl, N-Hydroxycarbamoyl oder N-Phenylcarbamoyl (auch in N-Aryl- und N-Arylniederalkylcarbamoylgruppen) umgewandelt werden, beispielsweise durch Umsetzung mit Ammoniak, einem Niederalkylamin oder einem Diniederalkylamin, Hydroxylamin oder Phenylamin, oder jeweils einem Salz davon, in Gegenwart eines Kondensationsmittels, z.B. einem Carbodiimid, wie Dicyclohexylcarbodiimid oder einem polaren Derivat davon, in polaren organischen Lösungsmitteln, wie Ethanol, oder einem N.N'-Carbonyldiazolid, wie N.N'-Dicarbonylimidazol (vgl. H. A. Staab, Angew. Chem. 74, 407-423 (1962)) in inerten organischen Lösungsmitteln, z.B. chlorierten Kohlenwasserstoffen, wie Methylenchlorid oder Chloroform, oder in Ethern, wie Diethylether, Tetrahydrofuran oder Dioxan, über das entsprechende Carbonsäureazolid. Man erhält so entsprechende Verbindungen der Formel II', in denen ein aus A₁ und A₂ zusammen gebildetes substituiertes Niederalkylen vorliegt, welches durch Carbamoyl, N-Mono- oder N,N-Diniederalkylcarbamoyl, N-Hydroxycarbamoyl und/oder N-Phenylcarbamoyl substituiert ist. Entsprechende Thiocarbamoylsubstituenten in aus A₁ und A₂ zusammen gebildetem substituiertem Niederalkylen können aus Carboxy durch Umwandlung in ein Carbonylhalogenid, z. B. mit einem anorganischen Säurehalogenid, wie Phosphortrichlorid, Phosphorpentachlorid oder Thionylchlorid, oder einem organischen Säurehalogenid, wie Oxalyldichlorid, und anschliessende Umsetzung z.B. mit Phosphorpentachlorid, Schwefelwasserstoff und Ammoniak, primären oder sekundären Aminen erhalten werden.

In Verbindungen der Formel II', worin ein aus A₁ und A₂ zusammen gebildetes substituiertes Niederalkylen durch Hydroxy und/oder Hydroxyniederalkyl substituiert ist, kann durch nukleophile Substitution die Hydroxygruppe in eine Hydrazino-, durch Niederalkyl, Aryl und/oder Arylniederalkyl N-substituierte Hydrazinogruppe, eine Guanidinogruppe oder eine durch Niederalkyl, Aryl und/oder Arylniederalkyl N-substituierte Guanidinogruppe umgewandelt werden. Beispielsweise kann Hydroxy durch Umsetzung mit aromatische Sulfonsäuren oder aktivierten Derivaten davon, wie den entsprechenden aromatischen Sulfonsäurehalogeniden, z. B. Toluolsulfonsäurehalogeniden, wie Toluolsulfonsäurechlorid, in Ab- oder vorzugsweise Anwesenheit von geeigneten Basen, z.B. tertiären Stickstoffbasen, wie Triethylamin oder N-Methylmorpholin, in durch die entsprechende aromatischen Sulfonsäure verestertes Hydroxy überführt werden und dann dieser Ester mit Hydrazin, Guanidin, den entsprechend substituierten Derivaten, oder Salzen davon, wobei auch Schutzgruppen vorliegen können, unter den Bedingungen der nukleophilen Substitution umgesetzt werden, vorzugsweise in Gegenwart von organischen Lösungsmitteln, z.B. Alkoholen, wie Methanol, Ethanol oder Trifluorethanol, Ketonen, wie Aceton, Nitrilen, wie Acetonitril, Estern, wie Essigsäureethylester, Ethern, wie Diethylether, Ethylenglykoldimethylether, Tetrahydrofuran oder Dioxan, Säureamiden, wie Dimethylformamid, Bisalkansulfinen, wie Dimethylsulfoxid, Arylalkoholen, wie Phenol, oder auch von Wasser, oder Gemischen dieser Lösungsmittel, erforderlichenfalls (z. B. zur Umsetzung von an Aryl gebundenem Stickstoff) in inerten organischen Lösungsmitteln, wie Dimethylformamid oder 1,3-Dimethyl-3,4,5,6-tetrahydro-2(1H)-pyrimidinon, unter Zugabe starker Basen, wie Natriumamid oder Natriumhydrid. Erforderlichenfalls werden vorhandene Schutzgruppen abgespalten. Man erhält Verbindungen der Formel II', worin A₁ und A₂ zusammen ein substituiertes Niederalkylen mit Substituenten ausgewählt aus Hydrazino, an einem oder beiden Stickstoffatomen durch Niederalkyl, Aryl oder Arylniederalkyl substituiertem Hydrazino, Guanidino und an einem, zwei oder allen drei Stickstoffatomen durch Niederalkyl, Aryl oder Arylniederalkyl substituiertem Guanidino bilden.

In Verbindungen der Formel II', worin A₁ und A₂ zusammen ein durch Cyano und/oder Cyanoniederalkyl substituiertes Niederalkylen bilden, können Cyanogruppen z.B. durch partielle Hydrolyse, im Sinne einer Graf-Ritter-Reaktion, oder über Imino-niederalkylester-Salze in Carbamoyl- oder N-Niederalkylcarbamoylgruppen in Verbindungen der Formel II' überführt werden. Die Bedingungen bei der Hydrolyse des Cyano-Zwischenproduktes können so gewählt werden, dass die Reaktion auf der Stufe des Amids abgebrochen wird. Zu diesem Zweck ist insbesondere die Hydrolyse mit Säuren geeignet, wobei z.B. 80%-ige Schwefelsäure (unter Erwärmen), Polyphosphorsäure (bei 110-150 °C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen. Mit Hilfe der Graf-Ritter-Reaktion gelingt die Herstellung N-monoalkylierter Amide der Formel II' aus den entsprechenden Nitrilen. Hierzu setzt man die Nitrile in Gegenwart einer starken Säure, vornehmlich 85-90%-iger Schwefelsäure, oder auch Polyphosphorsäure, Ameisensäure, Bortrifluorid oder anderen Lewis-Säuren, nicht jedoch Aluminiumchlorid, mit Verbindungen um, die im Sauren Carbeniumionen bilden können, also z.B. mit Olefinen oder Alkoholen. Die Imino-niederalkylester der Formel II' erhält man z.B. durch säurekatalysierte Anlagerung von Alkoholen an die Nitrilvorläufer (als Salze). Diese Anlagerung kann alternativ durch Basen, z.B. Alkoholate wie Natriummethoxid, katalysiert werden. Setzt man anstelle von Alkoholen entsprechende Mercaptane ein, beispielsweise in Gegenwart von Stickstoffbasen, wie Triethylamin oder N-Methylmorpholin, so erhält man die entsprechenden Imino-niederalkylthioester. Aus den Imino-niederalkylestern erhält man die Carbamoylderivate, aus den Imino-niederalkylthioestern die entsprechenden Thiocarbamoylderivate im Sinne einer Pinner-Spaltung durch thermischen Zerfall der Iminoester-Salze bei Temperaturen oberhalb von etwa 80 °C. Die Thiocarbamoylverbindungen können auch direkt erhalten werden durch Umsetzung von Cyanogruppen mit Schwefelwasserstoff analog der partiellen Hydrolyse, beispielsweise in Gegenwart von tertiären Stickstoffbasen, wie Triethylamin.

Verbindungen der Formel II', worin ein aus A₁ und A₂ zusammen gebildetes substituiertes Niederalkylen durch Amidino, Amidinoniederalkyl, an einem Stickstoffatom durch bis zu zwei Reste ausgewählt aus Niederalkyl, Aryl und Arylniederalkyl substituiertes Amidino und/oder an einem Stickstoffatom durch bis zu zwei Reste ausgewählt aus Niederalkyl, Aryl und Arylniederalkyl substituiertes Amidinoniederalkyl substituiert ist, lassen sich durch Umsetzung der wie oben aus entsprechenden Cyano- oder Cyanoniederalkylausgangsverbindungen hergestellten Imino-niederalkylester oder Imino-niederalkylthiolester (als Säureadditionssalze, z.B. -(C=NH)-OC₂H₅ ·HCl bzw. -C(=NH)-SC₂H₅ ·HI) herstellen, indem mit Ammoniak, primären oder sekundären Aminen umgesetzt wird. Die entsprechenden Cyanovorstufen können beispielsweise auch durch Umsetzung mit einem Alkalimetallamid, oder durch Reaktion mit einem primären oder sekundären Ammoniumsalz, z.B. einem primären oder sekundären Ammoniumhalogenid, in die entsprechenden freien, mono- oder disubstituierten Amidine überführt werden. Verbindungen der Formel II', worin A₁ und A₂ zusammen ein substituiertes Niederalkylen bedeuten, welches ein an allen beiden Stickstoffatomen durch Aryl, Arylniederalkyl oder Niederalkyl substituiertes Amidino oder Amidinoniederalkyl als Substituenten trägt, lassen sich auch aus den (wie oben für Niederalkylcarbamoyl beschrieben herstellbaren) entsprechenden Verbindungen herstellen, worin in Formel II' durch Niederalkyl, Aryl oder Arylniederalkyl N-substituiertes Carbamoyl steht, z. B. durch Umsetzung mit POCl₃ oder PCl₅ in die entsprechenden Imidsäurechloride (z.B. -(C=NH-niederalkyl)-Cl), welche nach Reaktion mit Ammoniak bzw. einem primären oder sekundären Amin substituierte Amidine der Formel II ergeben (vgl. Chem. Abstr. 81, 91186a (1974)).

In erhältlichen Verbindungen der Formel II' können in Amino- und/oder Aminoniederalkylresten (die als Substituent in einem aus A₁ und A₂ zusammen gebildeten substituierten Niederalkylen vorliegen) vorkommende Aminoreste in Ureido, Ureidoniederalkyl oder an einem oder beiden Stickstoffatomen durch je bis zu einen Rest ausgewählt aus Niederalkyl, Aryl oder Arylniederalkyl substituiertes Ureido oder Ureidoniederalkyl umgewandelt werden, indem man entsprechende Aminoverbindungen der Formel II', in denen Aminoreste vorliegen, solche, in denen N-Mononiederalkylaminoreste vorliegen oder solche, in denen anstelle von Amino Arylamino oder Arylniederalkylamino steht (herstellbar z. B. durch Umsetzung von Verbindungen der Formel II', worin das aus A₁ und A₂ zusammen gebildete substituierte Niederalkylen Hydroxy enthält, welches durch Veresterung, z. B. durch Umsetzung mit einem aromatischen Sulfonsäurehalogenid, wie Toluolsulfonsäurechlorid, in einen nukleofugen Rest, z.B. aromatisches Sulfonyloxy, überführt wird, analog der Umsetzung von Verbindungen der Formel II', worin anstelle von A₁ und A₂ Wasserstoff steht, mit Verbindungen der Formel VII unter nukleophiler Substitution des nukleofugen aromatischen Sulfonyloxy entweder mit einem Arylamin oder einem Arylniederalkylamin), mit einem Niederalkyl-, Aryl- oder Arylniederalkylisocyanat oder einem N-geschützten Isocyanat (z. B. Benzylisocyanat) umsetzt, vorzugsweise in einem Ether, z.B. einem cyclischen Ether, wie Tetrahydrofuran, bei bevorzugten Temperaturen zwischen -20 und 60 °C, insbesondere etwa bei Raumtemperatur, wobei funktionelle Gruppen, die nicht an der Reaktion teilnehmen sollen, erforderlichenfalls geschützt sind, und vorhandene Schutzgruppen gewünschtenfalls abspaltet.

Analog lassen sich in Verbindungen der Formel II' Aminogruppen, die als Substituent in einem aus A₁ und A₂ zusammen gebildeten substituierten Niederalkylen vorliegen, in Thioureido oder an einem oder beiden Stickstoffatomen durch je bis zu einen Rest ausgewählt aus Niederalkyl, Aryl oder Arylniederalkyl substituiertes Thioureido umwandeln, indem man anstelle der Isocyanate entsprechende Thioisocyanate verwendet.

Verbindungen der Formel II', worin als Substituent in einem aus A₁ und A₂ zusammen gebildeten substituierten Niederalkylen am endständigen Stickstoff durch 2 Reste ausgewählt aus Niederalkyl, Aryl und Arylniederalkyl substituiertes Ureido und/oder Ureidoniederalkyl vorliegt, können beispielsweise hergestellt werden, indem man entsprechende Aminoverbindungen der Formel II' mit Phosgen oder Analogen davon, z. B. N.N'-Carbonyldiazoliden, wie N.N'-Carbonyldiimidazol (vgl. H. A. Staab, Angew. Chem. 74, 407-423 (1962)), umsetzt und die entstehenden Chlorocarbonyl- oder Azolidocarbonylaminoverbindungen dann mit durch 2 Reste ausgewählt aus Niederalkyl, Aryl und Arylniederalkyl substituiertem Ammoniak umsetzt, oder umgekehrt die entsprechenden Aminonverbindungen der Formel II' mit dem Reaktionsprodukt von durch 2 Reste ausgewählt aus Niederalkyl, Aryl und Arylniederalkyl substituiertem Ammoniak mit Phosgen oder Analogen davon, z.B. den N.N'-Carbonyldiazoliden, wie N.N'-Carbonyldiimidazol, umsetzt unter Erhalt der entsprechend substituierten Ureidoverbindungen. Die Reaktionen werden vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und Rückflusstemperatur, insbesondere zwischen 0 und 30 °C, durchgeführt.

Verbindungen der Formel II', worin als Substituent in einem aus A₁ und A₂ gebildeten substituierte Niederalkylen am endständigen Stickstoff durch 2 Reste ausgewählt aus Niederalkyl, Aryl und Arylniederalkyl substituiertes Thioureidoniederalkyl vorliegt, können auf analoge Weise beispielsweise hergestellt werden, indem man entsprechende Aminoverbindungen der Formel II' mit Thiophosgen oder Analogen davon, z. B. N.N'-Thiocarbonyldiazoliden, wie N.N'-Thiocarbonyldiimidazol (vgl. H. A. Staab, Angew. Chem. 74, 407-423 (1962)), umsetzt und die entstehenden Chlorothiocarbonyl- oder Azolido-thiocarbonylaminoverbindungen dann mit durch 2 Reste ausgewählt aus Niederalkyl, Aryl und Arylniederalkyl substituiertem Ammoniak umsetzt, oder umgekehrt die entsprechenden Aminoverbindungen der Formel II' mit dem Reaktionsprodukt von durch 2 Reste ausgewählt aus Niederalkyl, Aryl und Arylniederalkyl substituiertem Ammoniak mit Thiophosgen oder Analogen davon, z.B. den N.N'-Thiocarbonyldiazoliden, wie N.N'-Thiocarbonyldiimidazol, umsetzt.

Eine Verbindung der Formel II', worin als Substituent in einem aus A₁ und A₂ zusammen gebildeten substituierten Niederalkylen Hydroxy und/oder Hydroxyniederalkyl vorliegt, kann zu einer entsprechenden Oxo-Verbindung oxidiert werden. Im Falle von primären Alkoholen ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle von sekundären Alkoholen kann ausserdem mit weniger selektiven Oxidationsmitteln, wie Chromsäure, Dichromat/Schwefelsäure, Dichromat/Eisessig, Salpetersäure, Braunstein und Selendioxid oxidiert werden. Man erhält Verbindungen der Formel II', in denen ein aus A₁ und A₂ zusammen gebildetes substituiertes Niederalkylen durch Oxo substituiert ist.

Verbindungen der Formel II', worin in einem aus A₁ und A₂ zusammen gebildeten substituierten Niederalkylen ein oder mehrere Substituenten ausgewählt aus Imino, Niederalkylimino, Acylimino, Hydroxyimino, Niederalkoxyimino, Hydrazono, N-Mono- oder N,N-Diniederalkylhydrazono, N-Acylhydrazono und einem durch Imino, Niederalkylimino, Acylimino, Hydroxyimino, Niederalkoxyimino, Hydrazono, N-Mono- oder N,N-Diniederalkylhydrazono und/oder N-Acylhydrazono substituierten Niederalkyl vorliegen, können aus entsprechenden Oxo-Verbindungen der Formel II' hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in dem die Oxidation einer Hydroxy- zur Oxoverbindung durchgeführt wird, welche beispielsweise wie zuletzt beschrieben erfolgt.

So können die Oxoverbindungen durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in die entsprechenden Iminoderivate überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; wobei in Wasser, einem wässrigen Lösungsmittelgemisch, wie einem Gemisch von Wasser mit einem oder mehreren Alkoholen, z. B. Methanol, Ethanol oder Isopropanol, Diniederalkylsulfoxiden, wie Dimethylsulfoxid, oder Diniederalkylniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen - 20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur; umgesetzt wird.

Verbindungen der Formel II', worin als Substituent in einem aus A₁ und A₂ gebildeten substituierten Niederalkylen Acylimino und/oder Acyliminoniederalkyl vorliegt, können aus den entsprechenden Iminoverbindungen erhalten werden durch Umsetzung mit den entsprechenden freien Säuren, welche den Acylrest enthalten, beispielsweise in Gegenwart von Kondensationsmitteln, wie Carbodiimiden, z. B. Dicyclohexylcarbodiimid, oder aktivierten Säurederivaten davon, z. B. Carbonsäurehalogeniden, gegebenenfalls in Gegenwart einer geeigneten Base, z.B. eines tertiären Amins, wie bereits definiert, vorzugsweise unter Feuchtigkeitsausschluss.

Verbindungen der Formel II', worin aus A₁ und A₂ zusammen gebildetes Niederalkylen Niederalkylthioiminosubstituenten trägt, können vorzugsweise hergestellt werden, indem man entsprechende Iminoausgangsmaterialien der Formel II' mit Niederalkylsulfenylhalogeniden (herstellbar z. B. aus Sulfensäuren mit Halogenwasserstoff oder durch Chlorolyse, Bromolyse oder Jodolyse von entsprechenden Organo-Schwefel-Verbindungen, wobei die Herstellung auch in situ erfolgen kann), insbesondere Niederalkylsulfenylhalogeniden, wie Methylsulfenylchlorid, umsetzt, vorzugsweise unter Verwendung der Salze der Iminoverbindungen oder in Gegenwart von Alkalihydroxiden, wie Natriumoder Kaliumhydroxid, vorzugsweise in organischen Lösungsmitteln, z.B. Kohlenwasserstoffen, wie Heptan, Ethern, wie Diethylether, Dioxan oder Tetrahydrofuran, oder Carbonsäureamiden, wie Dimethylformamid, bei bevorzugten Temperaturen zwischen 0 °C und der Rückflusstemperatur, insbesondere zwischen 0 und 30 °C.

Aus den erhältlichen Verbindungen der Formel II' werden die Verbindungen der Formel II vorzugsweise durch Hydrolyse, z.B. im sauren oder alkalischen Medium, erhalten. Vorzugsweise erfolgt die Hydrolyse der Verbindung der Formel II' in einer wässrigen alkoholischen Lösung einer Hydroxybase, z.B. mit einer Lösung eines Alkalimetallhydroxides, wie Natrium- oder Kaliumhydroxid, in einem Gemisch aus Wasser und Ethanol oder Methanol, bei bevorzugten Temperaturen von 0 °C bis zur Rückflusstemperatur des entsprechenden Reaktionsgemisches, insbesondere zwischen etwa 60 °C und der Rückflusstemperatur. Besonders bevorzugt ist die Reaktionsführung unter Sauerstoffausschluss, beispielsweise unter Schutzgas, wie Argon oder Stickstoff.

Die Ausgangsmaterialien der Formel IV werden vorzugsweise dadurch hergestellt, dass man eine Verbindung der Formel II, wie oben definiert, mit einem Säureanhydrid der Formel X,

R₅-(C=O)-O-(C=O)-R₅' (X)

umsetzt, worin R₅ und R₅' unabhängig voneinander für Wasserstoff oder Niederalkyl, nicht jedoch beide für Wasserstoff, stehen, beispielsweise in An- oder vorzugsweise Abwesenheit weiterer geeigneter Lösungsmittel, bei bevorzugten Temperaturen zwischen 0 °C und Rückflusstemperatur, z.B. bei 40 bis 60 °C.

Die übrigen Ausgangsmaterialien sind bekannt, kommerziell erhältlich oder nach bekannten Verfahren herstellbar.

### Verfahren b): Bildung von Aminalen

In Verbindungen der Formeln XI und XII sind, falls funktionelle Gruppen vorliegen, die nicht an der Reaktion teilnehmen sollen, diese Gruppen erforderlichenfalls geschützt, vorzugsweise durch die unter Verfahren a) angegebenen Schutzgruppen. Die Abspaltung der Schutzgruppen in den resultierenden Verbindungen erfolgt erforderlichenfalls nach den ebenfalls unter Verfahren a) genannten Methoden.

Ein reaktionsfähiges Derivat eines Aldehyds der Formel XII ist insbesondere ein entsprechendes Acetal der Formel XIIa,

R₀-CH(-O-G)₂ (XIIa)

worin R₀ die genannten Bedeutungen hat und G Niederalkoxy, wie Methoxy oder Ethoxy, bedeutet, oder worin die beiden Reste -O-G zusammen Niederalkylendioxy, wie Ethylen-1,2-dioxy, bedeuten.

Die Umsetzung findet vorzugsweise in Gegenwart von Säuren, z.B. Halogenwasserstoffsäuren, wie Chlorwasserstoffsäure, oder Schwefelsäure, oder insbesondere in Gegenwart von Lewis-Säuren, vor allem SnCl₂ (z.B. als Hydrat), in geeigneten Lösungsmitteln oder Lösungsmittelgemischen, z.B. Ethern, wie Diniederalkoxyniederalkanen, insbesondere in 1,2-Dimethoxyethan, bei bevorzugten Temperaturen zwischen 0 und 50 °C, z.B. etwa bei Raumtemperatur, statt, erforderlichenfalls unter Wasserzusatz.

Die Ausgangsverbindungen der Formel XII oder XIIa sind bekannt, kommerziell erhältlich oder nach an sich bekannten Verfahren herstellbar.

Die Ausgangsmaterialien der Formel XI sind analog den unter Verfahren a) beschriebenen Methoden aus Analogen von Verbindungen der Formel II' oder IV, worin anstelle von A₁ und A₂ jeweils ein Wasserstoffatom steht (herstellbar z.B. aus Verbindungen der Formel V und der Formel VI, worin A Wasserstoff bedeutet und die übrigen Reste die angegebenen Bedeutungen haben, nach den genannten Methoden und gegebenenfalls über die entsprechenden Zwischenprodukte), durch Umsetzung mit Verbindungen der Formel III herstellbar. Beispielsweise sind sie herstellbar, wie in EP 0 516 588 (Anmeldenummer 92 810385) beschrieben.

### Umwandlungen:

Verbindungen der Formel I können in an sich bekannter Weise in andere Verbindungen der Formel I umgewandelt werden.

Es ist möglich, einzelne der genannten Umwandlungen durchzuführen, oder aber auch geeignete Kombinationen zu wählen, also zwei oder mehr Umwandlungen mit einer Verbindung der Formel I durchzuführen. Funktionelle Gruppen in Ausgangsmaterialien der Formel I und anderen Ausgangsmaterialien, die nicht an der jeweiligen Umsetzung teilnehmen sollen, liegen erforderlichenfalls in geschützter Form vor. Die Schutzgruppen werden zu geeigneten Zeitpunkten abgespalten. Die Einführung der Schutzgruppen, die Schutzgruppen selbst und ihre Abspaltung sind wie oben beschrieben.

Zum Beispiel kann eine Verbindung der Formel I, worin die Gruppe -C(=X)- für -C(=O)- steht, mit einem geeigneten Reagens umgesetzt werden, so dass eine andere Verbindung der Formel I, worin die Gruppe -C(=X)- für -C(=S)-, -CH₂- oder -C(=CR₁R₂)- steht, erhalten wird. Ein geeignetes Reagens für die Ueberführung von -C(=O)- in -C(=S)- ist z.B. das Lawesson-Reagens (= 2,4-Bis-(4-methoxyphenyl)-2,4-dithioxo-1,3,2,4-dithiaphosphetan), wobei die Reaktion beispielsweise in einem halogenierten Kohlenwasserstoff, wie Dichlormethan, bei Temperaturen von 30 °C bis zur Rückflusstemperatur, insbesondere bei Rückflusstemperatur, durchgeführt wird. Zur Umwandlung von -C(=O)- in -CH₂- sind beispielsweise die Systeme LiA1H₄/Tetrahydrofuran oder Zinkamalgam/HCl/Ethanol geeignet. Die Ueberführung von -C(=O)- in -C(=CR₁R₂)- gelingt z.B. durch die Umsetzung mit einer starken Base, z.B. LDA (Lithiumdiisopropylamid) und dann einem Grignard-Reagens der Formel HCR₁R₂MgHal (Hal = Halogen, z.B. Iod).

Weiterhin können z.B. Verbindungen der Formel I, worin R Wasserstoff bedeutet, durch Alkylierung, z.B. mit Niederalkyl- oder Arylniederalkylhalogeniden nach Behandlung mit geeigneten Basen, etwa Natriumhydrid oder Kalium-tert.-butoxid, in andere Verbindungen der Formel I, worin R für Niederalkyl oder Arylniederalkyl steht, überführt werden.

Verbindungen der Formel I, worin einer der Reste A₁ und A₂ Wasserstoff bedeutet, können durch Umsetzung mit geeigneten Reagentien in andere Verbindungen der Formel I, worin keiner der Reste A₁ und A₂ mehr Wasserstoff bedeutet, überführt werden.

Zur Einführung von A₁ oder A₂ = substituiertes Niederalkyl kommt z.B. die Behandlung mit der Base LDA und anschliessende Umsetzung mit einem substituierten Diniederalkylether oder einem substituierten Niederalkylhalogenid in Frage. Unter diesen Bedingungen wird eine gegebenenfalls im Molekül vorhandene Gruppe -N(-R)- = -NH- nur wenig oder gar nicht alkyliert. Substituiertes Niederalkyl ist dabei wie für den entsprechenden Rest A₁ und/oder A₂ in Verbindungen der Formel I definiert.

Verbindungen der Formel I, worin Ar₁ und/oder Ar₂ durch Halogen, vorzugsweise Brom, substituiertes Aryl, insbesondere Phenyl oder Naphthyl, bedeutet, können in die entsprechenden Derivate umgewandelt werden, in denen eines oder alle der in Aryl Ar₁ und/oder Ar₂ vorhandenen Halogenatome durch Cyano ersetzt sind, beispielsweise durch Umsetzung mit einem Cyanidsalz eines Übergangsmetalles, insbesondere CuCN, bei Temperaturen zwischen 50 und 150 °C, vorzugsweise zwischen 60 und 140 °C, in einem inerten polaren Lösungsmittel, wie einem N,N-Diniederalkyl-niederalkancarbonsäureamid, z. B. Dimethylformamid, ohne oder mit anschliessender Zugabe eines Katalysators, z. B. eines Übergangsmetallhalogenides, wie Eisen(III)chlorid, in wässriger Lösung, (siehe auch Rosenmund et al., Ber. 52, 1749 (1916); von Braun et al., Ann. 488, 111 (1931)).

In Verbindungen der Formel I können die Reste Ar₁ und/oder Ar₂, welche unsubstituiertes oder substituiertes Aryl bedeuten, vorzugsweise unsubstituiertes Phenyl oder Naphthyl, unabhängig voneinander unter Einführung einer oder mehrerer Nitrogruppen nitriert werden, beispielsweise unter üblichen Bedingungen zur Einführung einer Nitrogruppe in Aromaten, z. B. mit konzentrierter oder 100%-iger Salpetersäure bei Temperaturen zwischen 0 und 100 °C, vorzugsweise zwischen 10 und 40 °C, in einem inerten Lösungsmittel, z. B. einem organischen Säureanhydrid, wie Acetanhydrid. Entstehen dabei mehrere verschiedene Produkte mit unterschiedlicher Position und Anzahl der Nitrogruppen, so können diese nach üblichen Methoden, beispielsweise säulenchromatographisch, getrennt werden.

Ein Nitrosubstituent der Reste Ar₁ und/oder Ar₂ kann zu Amino reduziert werden, z.B. durch Hydrierung unter üblichen Bedingungen, z. B. durch Hydrierung in Gegenwart eines zur selektiven Reduktion der Nitrogruppen geeigneten Hydrierkatalysators, wie Raney-Nickel, in einem inerten Lösungsmittel, z. B. einem cyclischen oder acyclischen Ether, wie Tetrahydrofuran, unter Normaldruck oder bei bis zu 5 bar erhöhtem Druck.

Verbindungen der Formel I mit veretherten Hydroxygruppen, beispielsweise mit Niederalkoxyresten als Substituenten innerhalb von Ar₁ und/oder Ar₂ oder an A₁ und/oder A₂, können durch Etherspaltung in die entsprechenden Hydroxy-substituierten Verbindungen der Formel I überführt werden. Die Etherspaltung erfolgt unter an sich bekannten Bedingungen, beispielsweise in Gegenwart von Halogenwasserstoffsäuren, wie Bromwasserstoff oder Iodwasserstoff, in An- oder Abwesenheit von Lösungsmitteln, wie Carbonsäuren, z. B. Niederalkancarbonsäuren, wie Essigsäure, bei Temperaturen zwischen 20 °C und der Rückflusstemperatur des Reaktionsgemisches, oder vorzugsweise unter schonenden Bedingungen mit Borhalogeniden, insbesondere Bortribromid, in einem inerten Lösungsmittel, wie einem chlorierten Kohlenwasserstoff, z. B. Methylenchlorid oder Chloroform, bei Temperaturen zwischen -80 und 0 °C, insbesondere zwischen -50 und -20 °C.

Verbindungen der Formel I, in denen A₁ und/oder A₂ oder aus diesen beiden Resten zusammen gebildetes substituiertes Niederalkylen Hydroxyreste enthalten, z.B. in Hydroxyniederalkyl A₁ und/oder A₂ oder aus A₁ und A₂ zusammen gebildetem 1-Hydroxyniederalkyl-ethylen, können durch Oxidation zu den entsprechenden Carbonylverbindungen und direkt anschliessende oder erst nach der Isolierung der Carbonylverbindung erfolgende Umsetzung mit Hydroxylamin oder einem Salz davon oder weiteren Aminoverbindungen in die entsprechenden Iminoverbindungen, wie Hydroxyiminoverbindungen, überführt werden. Die weiteren Substituenten, Reagentien und bevorzugten Reaktionsbedingungen finden sich bei der Beschreibung der Herstellung von Verbindungen der Formel II, worin als Substituent in aus A₁ und A₂ zusammen gebildetem substituiertem Niederalkylen Imino, Niederalkylimino, Acylimino, Hydroxyimino, Niederalkoxyimino, Hydrazono, N-Mono- oder N,N-Diniederalkylhydrazono und/oder N-Acylhydrazono vorliegt, aus entsprechenden Oxo-Verbindungen der Formel II, wobei an Stelle der Oxoverbindungen der Formel II solche der Formel I einzusetzen sind.

Verbindungen der Formel I, in denen Hydroxyimino als Substituent in A₁ und/oder A₂ oder einem aus diesen beiden Resten zusammen gebildetem Niederalkylen vorliegt, können unter Hydrierung zu den entsprechenden Aminoverbindungen umgesetzt werden. Die Hydrierung erfolgt dabei vorzugsweise katalytisch mit selektiven Hydrierkatalysatoren, insbesondere in Gegenwart von Palladium auf festen Trägermaterialien, z.B. auf Kohle, in polaren organischen oder organisch-wässrigen Lösungsmitteln oder Lösungsmittelgemischen, insbesondere in Ethern, z.B. cyclischen Ethern, wie Tetrahydrofuran oder Dioxan, oder in Alkoholen, z.B. Niederalkanolen, wie Methanol oder Ethanol, oder Gemischen davon, z.B. in Methanol/Tetrahydrofuran-Gemischen, bei Temperaturen zwischen -20 und 60 °C, vorzugsweise zwischen 0 und 40 °C, z.B. etwa bei Raumtemperatur.

In Verbindungen der Formel I mit primären Hydroxygruppen, beispielsweise solchen Verbindungen der Formel I, in denen substituiertes Niederalkyl, Niederalkenyl oder Niederalkinyl A₁ und/oder A₂ primäre Hydroxygruppen enthalten, wie in Hydroxyethyl, oder worin ein aus A₁ und A₂ zusammen gebildetes substituiertes Niederalkylen vorliegt, welches als Substituent Hydroxyniederalkyl, wie Hydroxymethyl, enthält, können primäre Hydroxygruppen zu entsprechenden Carboxy- oder Carboxyniederalkylresten oxidiert werden, beispielsweise durch Oxidation mit Chromsäure, Dichromat/Schwefelsäure, Salpetersäure, Braunstein oder Kaliumpermanganat, vorzugsweise mit Kaliumpermanganat in neutralem oder alkalischem Medium, z.B. in wässriger alkoholischer Lösung, bei bevorzugten Temperaturen zwischen -20 und 50 °C, insbesondere zwischen 0 °C und Raumtemperatur. Man erhält entsprechende Carboxy-substituierte Verbindungen der Formel I.

In Verbindungen der Formel I, worin ein substituiertes Niederalkyl A₁ und/oder A₂ Carboxy enthält, wie in Carboxymethyl A₁ und/oder A₂, oder worin ein aus A₁ und A₂ zusammen gebildetes substituiertes Niederalkylen vorliegt, welches als Substituent Carboxy oder Carboxyniederalkyl enthält, beispielsweise in solchen Verbindungen, die nach dem zuletzt beschriebenen Verfahren aus Verbindungen der Formel I mit primären Hydroxygruppen hergestellt sind, können Carboxygruppen durch Reaktion mit Diazomethan (ergibt Methyloxycarbonyl) oder mit Niederalkanolen, wie Methanol oder Ethanol, in die entsprechenden Niederalkoxycarbonylgruppen überführt werden. Die Reaktion mit Diazomethan erfolgt beispielsweise in wässrig-alkoholischer Lösung, z.B. in Wasser/Methanol, oder vorzugsweise in Ether, in Gegenwart einer etherischer Lösung von Diazomethan, z.B. Diazomethan in Diethylether, bei Temperaturen zwischen -20 und 30 °C, z.B. zwischen 0 °C und Raumtemperatur. Die Reaktion mit Niederalkanolen erfolgt vorzugsweise in Gegenwart von Kondensationsmitteln, wie Carbodiimiden, z.B. Dicyclohexylcarbodiimid, in dem betreffenden Niederalkanol, dem ein weiteres inertes organisches Lösungsmittel, vorzugsweise Dimethylformamid oder Dimethylacetamid, zugesetzt sein kann, bei Temperaturen zwischen 0 ° und der Rückflusstemperatur, vorzugsweise zwischen 10 und 40 °C. Man erhält entsprechende Niederalkoxycarbonylverbindungen der Formel I. Umgekehrt kann in einer Verbindung der Formel I verestertes Carboxy (insbesondere Niederalkoxycarbonyl, wie Ethoxycarbonyl, beispielsweise als Substituent von aus A₁ und A₂ in Formel I gemeinsam gebildetem Niederalkylen), in freies Carboxy umgewandelt werden, beispielsweise durch Hydrolyse in Gegenwart von Wasser und Säuren, wie Halogenwasserstoffsäuren, z.B. Chlorwasserstoffsäure, oder Schwefelsäure, oder geeigneten Lewis-Säuren, wie SnCl₂, auch in Gegenwart von geeigneten Lösungsmitteln, insbesondere Ethern, z.B. Diniederalkoxyniederalkanen, wie 1,2-Dimethoxyethan, bei bevorzugten Temperaturen von zwischen 0 und 50 °C, z.B. etwa bei Raumtemperatur, erforderlichenfalls unter Schutzgas, wie Argon oder Stickstoff. Möglich ist auch die Freisetzung von Carboxy durch biochemische Methoden wie die Verseifung mit Esterasen in geeigneten Puffersystemen.

Verbindungen der Formel I, worin als Substituent in einem aus A₁ und A₂ zusammen gebildeten substituierten Niederalkylen Hydroxy und/oder Hydroxyniederalkyl vorliegt, können zu einer entsprechenden Oxo-Verbindung oxidiert werden. Im Falle von primären Alkoholen ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle von sekundären Alkoholen kann ausserdem mit weniger selektiven Oxidationsmitteln, wie Chromsäure, Dichromat/Schwefelsäure, Dichromat/Eisessig, Salpetersäure, Braunstein, Selendioxid oder Dimethylsulfoxid in Gegenwart von Oxalylchlorid, in Wasser, wässrigen oder organischen Lösungsmitteln, wie halogenierten Kohlenwasserstoffen, z.B. Methylenchlorid, oder Carbonsäureamiden, wie Dimethylformamid, oxidiert werden, vorzugsweise bei Temperaturen zwischen - 50 °C und Rückflusstemperatur, insbesondere zwischen - 10 und 50 °C. Man erhält Verbindungen der Formel I, in denen aus A₁ und A₂ zusammen gebildetes substituiertes Niederalkylen durch Oxo substituiert ist.

Verbindungen der Formel I, worin in einem aus A₁ und A₂ zusammen gebildeten substituierten Niederalkylen ein oder mehrere Substituenten ausgewählt aus Imino, Niederalkylimino, Acylimino, Hydroxyimino, Niederalkoxyimino, Hydrazono, N-Mono- oder N,N-Diniederalkylhydrazono, N-Acylhydrazono und durch Imino, Niederalkylimino, Acylimino, Hydroxyimino, Niederalkoxyimino, Hydrazono, N-Mono- oder N,N-Diniederalkylhydrazono und/oder N-Acylhydrazono substituiertem Niederalkyl vorliegt, können aus entsprechenden Oxo-Verbindungen der Formel I hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in dem die Oxidation einer Hydroxy- zur Oxoverbindung durchgeführt wird, welche beispielsweise wie zuletzt beschrieben erfolgt.

So können die Oxoverbindungen durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in die entsprechenden Iminoderivate überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt wird; wobei in Wasser (in An- oder Abwesenheit von Tensiden), einem wässrigen Lösungsmittelgemisch, wie einem Gemisch von Wasser mit einem oder mehreren Alkoholen, z. B. Methanol, Ethanol oder Isopropanol, Diniederalansulfinen, wie Dimethylsulfoxid, oder Diniederalkylniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen - 20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur; umgesetzt wird.

Verfahrensgemäss erhältliche freie Verbindungen der Formel I mit salzbildenden Gruppen können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Diastereomere z.B. durch Verteilung zwischen mehrphasigen Lösungsmittelgemischen, Umkristallisieren und/oder chromatographische Trennung, beispielsweise an Kieselgel, Racemate z.B. durch Bilden von Salzen mit optisch reinen salzbildenden Reagentien, Auftrennen des so erhältlichen Diastereomerengemisches, z.B. mittels fraktionierter Kristallisation, und anschliessende Freisetzung des gewünschten reinen Enantiomeren, oder durch Chromatographie an optisch aktiven Säulenmaterialien.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 200°C, vorzugsweise von etwa -20°C bis etwa 150°C, z.B. bei Raumtemperatur bis zu der Rückflusstemperatur, bei Schmelzen bei bis zu 220 °C, unter atmosphärischem Druck oder in einem geschlossenen Gefäss, gegebenenfalls unter Druck, z.B. bei dem Druck, der im Reaktionsgemisch unter den Reaktionsbedingungen in einem geschlossenen Rohr entsteht, und/oder in einer inerten Atmosphäre, z.B. unter Argon oder Stickstoff. Bevorzugt sind die jeweils spezifisch genannten Reaktionsbedingungen.

Lösungs- und Verdünnungsmittel sind beispielsweise Wasser, Alkohole, z.B. Niederalkanole, wie Methanol, Ethanol oder Propanol, Diole, wie Ethylenglykol, Triole, wie Glycerin, oder Arylalkohole, wie Phenol, Säureamide, z.B. Carbonsäuramide, wie Dimethylformamid, Dimethylacetamid oder 1,3-Dimethyl-3,4,5,6-tetrahydro-2(1H)-pyrimidinon (DMPU), oder Amide anorganischer Säuren, wie Hexamethylphosphorsäuretriamid, Ether, z.B. cyclische Ether, wie Tetrahydrofuran oder Dioxan, oder acyclische Ether, wie Diethylether oder Ethylenglykoldimethylether, halogenierte Kohlenwasserstoffe, wie Haloniederalkane, z.B. Methylenchlorid oder Chloroform, Ketone, wie Aceton, Nitrile, wie Acetonitril, Säureanhydride, wie Acetanhydrid, Ester, wie Essigsäureethylester, Bisalkansulfine, wie Dimethylsulfoxid, Stickstoffheterocyclen, wie Pyridin, Kohlenwasserstoffe, z.B. Niederalkane, wie Heptan, oder Aromaten, wie Benzol oder Toluol, oder Gemische dieser Lösungsmittel, wobei die jeweils geeigneten Lösungsmittel für die oben genannten Reaktionen ausgewählt werden können.

Infolge der engen Beziehung zwischen den Verbindungen der Formel I und ihren Vorstufen in freier Form und in Form von Salzen und/oder Tautomeren sind vor- und nachstehend unter den freien Verbindungen und Ausgangsmaterialien sinn- und zweckmässig gegebenenfalls auch die entsprechenden Salze bzw. freien Verbindungen und/oder Tautomeren zu verstehen, sofern die Verbindungen eine oder mehrere salzbildende Gruppen, z.B. basische Gruppen, wie Amino- oder Iminogruppen, auch solche, die an nicht mehr als ein ungesättigtes Kohlenstoffatom, wie die Gruppen -NA₁Ar₁ und/oder -NA₂Ar₂ am C-Atom des zentralen Phenylringes Rest, worin Ar₁ und A₁ und/oder Ar₂ und A₂ nicht über ein ungesättigtes Kohlenstoffatom gebunden sind, und/oder saure Gruppen, wie Carboxy oder Sulfo (SO₃H), und/oder tautomerisierbare Gruppen enthalten. Sofern im Zusammenhang mit Ausgangsmaterialien und Verbindungen der Formel I vor- und nachstehend ein Substituent, eine Verbindung, ein Tautomeres, ein Salz, Substituenten, Verbindungen, Tautomere oder Salze erwähnt werden, ist dies, soweit sinnvoll und zweckmässig, unabhängig von der Verwendung des Singulars oder Plurals zu verstehen als "ein oder mehrere". Ausgangsmaterialien können erforderlichenfalls auch in geschützter Form vorliegen, soweit sinnvoll und zweckmässig, wobei Schutzgruppen zu geeigneten Zeitpunkten abgespalten werden. Schutzgruppen und ihre Abspaltung sind insbesondere wie oben definiert.

Die Verbindungen oder ihre Salze können auch als Hydrate erhalten werden, oder ihre Kristalle können z.B. das zur Kristallisation verwendete Lösungsmittel einschliessen.

Im Verfahren der vorliegenden Erfindung werden vorzugsweise solche Ausgangsmaterialien eingesetzt, die zu den als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsmaterial unter den Reaktionsbedingungen gebildet oder in Form eines Derivates, z.B. eines Salzes davon, verwendet wird.

Reihenfolge und Reaktionsbedingungen aller beschriebenen Umsetzungen sind vorzugsweise so zu wählen, wie es für den Fachmann als sinn- und zweckmässig anzusehen ist.

Neue Zwischenprodukte gehören ebenfalls zur vorliegenden Erfindung. Insbesondere gilt dies für die Zwischenverbindungen der Formel II und die der Formel II', welche wie oben definiert sind. Besonders bevorzugt sind die in den Beispielen genannten Zwischenverbindungen der Formel II und der Formel II'.

Die vorliegende Erfindung betrifft auch pharmazeutische Präparate, die als Wirkstoff Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Bevorzugt ist eine pharmazeutische Zusammensetzung, welche geeignet ist zur Verabreichung an einen Warmblüter, insbesondere Menschen, der an einer Erkrankung leidet, die auf eine Hemmung einer Proteinkinase anspricht, beispielsweise Psoriasis oder ein Tumor, umfassend eine zur Hemmung der Proteinkinase wirksame Menge einer Verbindung der Formel I oder eines Salzes davon, falls salzbildende Gruppen vorliegen, zusammen mit mindestens einem pharmazeutisch annehmbaren Trägermaterial.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, oder Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Überzügen versehen werden, wobei man u.a. konzentrierte Zuckerlösungen, die gegebenenfalls Polyvinylpyrrolidon, Talk, arabischen Gummi, Polyethylenglykol und/oder Titandioxid enthalten, Lacklösungen in geeigneten organischen Lösungsmitteln oder Lösungsmittelgemischen oder, zur Herstellung Magensaft-resistenter Überzüge, Lösungen geeigneter Cellulosepräparate, wie Acetylcellulosephthalat oder Hydroxypropylmethylcellulosephthalat, verwendet. Tabletten oder Dragee-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind auch Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, z.B. fetten Ölen, Lauroglycol (Gattefossé S. A., Saint Priest, Frankreich), Gelucir (Gattefossé S. A., Saint Priest, Frankreich) oder Sesamöl, Paraffinöl oder flüssigen Polyethylenglykolen, wie PEG 300 oder 400, oder Fettsäureestern von Niederalkylglykolen gelöst oder suspendiert, gegebenenfalls unter Stabilisator- und/oder Detergens-Zusatz.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände, insbesondere solcher, die auf eine Hemmung von Proteinkinasen ansprechen. Die Verbindungen der vorliegenden Erfindung können prophylaktisch oder therapeutisch verabreicht werden, vorzugsweise in einer gegen die genannten Krankheiten wirksamen Menge an einen Warmblüter, z. B. Menschen, der einer derartigen Behandlung bedarf, wobei man sie vorzugsweise in Form von pharmazeutischen Präparaten verwendet. Dabei wird bei einem Körpergewicht von etwa 70 kg eine tägliche Dosis von 1 mg bis 5000 mg, z.B. von etwa 0,1 g bis etwa 5 g, vorzugsweise von etwa 0,5 g bis etwa 2 g, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: abs. = absolut (wasserfrei); DC = Dünnschichtchromatogramm; DMF = Dimethylformamid; DMPU = 1,3-Dimethyl-3,4,5,6-tetrahydro-2(1H)-pyrimidinon ("Dimethylpyroharnstoff"); Ether = Diethylether; FAB-MS = Fast Atom Bombardment Mass Spectroscopy; gesätt. = gesättigt; ¹H-NMR = Protonen-Kernmagnetische Resonanz; HV = Hochvakuum; konz. = konzentriert; krist. = kristallin; RT = Raumtemperatur, Smp. = Schmelzpunkt; THF = Tetrahydrofuran; Torr = Druckeinheit (1 Torr entspricht 1 mm Quecksilbersäule). Mengenverhältnisse von Lösungsmittel-/Elutionsmittelgemischen beziehen sich, wenn nicht anders angegeben, auf Volumenverhältnisse (v/v).

### Beispiel 1: 5,8-Diphenyl-5,8-diaza-6-hydroxymethyl-5,6,7,8-tetrahydro-naphthalin-2,3-dicarboximid

Eine Suspension von 230 mg (0,7 mmol) 5,8-Diphenyl-5,8-diaza-6-hydroxymethyl-5,6,7,8-tetrahydro-naphthalin-2,3-dicarbonsäuredimethylester in 8 ml Ethylenglykol wird auf 120° erwärmt; unter Rühren wird während 24 h Ammoniak-Gas durchgeleitet. Das Reaktionsgemisch wird abgekühlt, mit gesättigter Kochsalzlösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan auf Kieselgel aufgetragen und chromatographiert. Mit Dichlormethan werden zunächst die Nebenprodukte abgetrennt, die Produktfraktionen werden mit Hexan/Ethylacetat 2:1 eluiert, vereinigt und eingedampft. Der Eindampfrückstand wird aus Ethylacetat/Hexan kristallisiert. Man erhält die Titelverbindung in Form oranger Kristalle, Smp. 268°, FAB-MS: 386 [M⁺+H].

Die Ausgangsmaterialien werden folgendermassen hergestellt:
a) 5,8-Diphenyl-5,8-diaza-6-hydroxymethyl-5,6,7,8-tetrahydro-naphthalin-2,3-dicarbonsäuredimethylester
   Unter Argon werden zu einer Lösung von 377 mg (1 mmol) 4,5-Dianilinophthalsäuredimethylester in 10 ml DMF abs. 96 mg (2.2 mmol) Natriumhydrid (Dispersion 60% in Oel) gegeben. Bei RT wird eine Lösung von 96 µl (1.2 mmol) Epichlorhydrin in 2 ml DMF abs. zugetropft, das Reaktionsgemisch auf 100°C erwärmt und 12 h gerührt. Das Reaktionsgemisch wird abgekühlt, das Lösungsmittel abgedampft, der schwarze Rückstand in Dichlormethan gelöst und die Lösung mit Wasser gewaschen, mit Natriumsulfat getrocknet und eingedampft. Der Eindampfrückstand wird mit Ethylacetat/Hexan 1:2 an Kieselgel chromatographiert, die Produktfraktionen vereinigt und eingedampft. So wird die Titelverbindung als schwach gelbes Pulver erhalten, Smp. 68°, FAB-MS: 433 [M⁺+H].
b) 4,5-Dianilinophthalsäuredimethylester
   Eine Lösung von 5,6 g (15 mmol) 4,5-Bis(trimethylsilyloxy)cyclohexa-1,4-dien-1,2-dicarbonsäuredimethylester und 5,5 ml (60 mmol) Anilin in 60 ml Eisessig wird während 4 h unter Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃-Lösung und zweimal 20 ml Wasser gewaschen, mit Natriumsulfat getrocknet und eingedampft. Das Rohprodukt wird aus Ethanol umkristallisiert. Auf diese Weise wird die Titelverbindung in Form gelber Kristalle erhalten, Smp. 178°, FAB-MS: 377 [M⁺+H].
c) 4,5-Bis(trimethylsilyloxy)cyclohexa-1,4-dien-1,2-dicarbonsäuredimethylester
   Unter Argon wird eine Lösung von 7,1 g (50 mmol) Acetylendicarbonsäuredimethylester in 30 ml Toluol zu 12,5 g (50 mmol) 2,3-Bis(trimethylsilyloxy)-1,3-butadien (95%) getropft und anschliessend 19 h am Rückfluss gekocht. Das Reaktionsgemisch wird abgekühlt, das Lösungsmittel abgedampft und der Rückstand im Hochvakuum (0,1 mbar, 124-127°) destilliert. Auf diese Weise wird die Titelverbindung als gelbes, hochviskoses Oel erhalten, ¹H-NMR (CDCl₃): δ = 0.18 (s, 18H), 3.09 (s, 4H), 3.78 (s, 6H).

### Beispiel 2: 4-(3-Aminopropyl)-4,5-dianilinophthalimid

Analog zu Beispiel 1 werden 44 mg (0.1 mmol) 4-(3-Aminopropyl)-4,5-dianilinophthalsäuredimethylester in 4 ml Ethylenglykol auf 120° erwärmt, wobei unter Rühren während 24 h Ammoniak-Gas durchgeleitet wird. Das Reaktionsgemisch wird abgekühlt, mit konz. Kochsalzlösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 10:1 an Kieselgel chromatographiert, die Produktfraktionen vereinigt und eingedampft. Auf diese Weise wird die Titelverbindung in Form gelber Kristalle erhalten, FAB-MS: 387 [M⁺+H].

Die Ausgangsmaterialien werden folgendermassen hergestellt:
a) 4-(3-Aminopropyl)-4,5-dianilinophthalsäuredimethylester
   Zu einer Lösung von 186 mg (0.33 mmol) 4,5-Dianilino-4-(3-[N-phthalimidopropyl])phthalsäuredimethylester in 15 ml THF werden 39 mg (0.78 mmol) Hydrazinhydrat auf einmal unter Argon zugegeben und das Reaktionsgemisch 22 h unter Rückfluss erhitzt. Das Reaktionsgemisch wird auf 40° abgekühlt, mit 200 µl konz. Salzsäure versetzt und für zwei Minuten auf 70° erwärmt, auf Raumtemperatur abgekühlt und filtriert. Das Filtrat wird eingedampft, der Rückstand zwischen Ethylacetat und Wasser verteilt. Die Wasserphase wird abgetrennt, mit 2N Natronlauge basisch gestellt und noch zweimal mit Ethylacetat extrahiert. Die organischen Phasen werden vereinigt, mit Natriumsulfat getrocknet und eingedampft. Der Rückstand wird mit Dichlormethan/Methanol 10:1 an Kieselgel chromatographiert und die Produktfraktionen eingedampft und am HV getrocknet. Auf diese Weise wird die Titelverbindung in Form eines blassgelben Schaums erhalten, FAB-MS: 434 [M⁺+H], ¹H-NMR (CD₃OD): 7.6 (s, 1H), 7.3 (t, 2H), 7.15 (m, 6H), 6.75 (m, 3H), 3.8 (d, 6H), 3.65 (t, 2H), 2.7 (t, 2H), 1.85 (m, 2H).
b) 4,5-Dianilino-4-(3-[N-phthalimidopropyl])-phthalsäuredimethylester
   Unter Stickstoff werden zu einer Lösung von 377 mg (1 mmol) 4,5-Dianilinophthalsäuredimethylester (Beispiel 1 b) in 15 ml DMPU abs. 68 mg (1.5 mmol) Natriumamid (krist.) gegeben. Das Reaktionsgemisch wird auf 40° erwärmt und 30 min ein Vakuum von 1 Torr angelegt. Darauf wird mit Stickstoff belüftet und es werden 402 mg (1.5 mmol) N-(3-Brompropyl)phthalimid zugegeben. Das Reaktionsgemisch wird auf 100°C erwärmt und 2 h gerührt. Das Reaktionsgemisch wird abgekühlt, mit Wasser versetzt und dreimal mit Ethylacetat extrahiert. Die organischen Phasen werden vereinigt, mit Wasser gewaschen, mit Natriumsulfat getrocknet und eingedampft. Der Eindampfrückstand wird mit Ethylacetat/Hexan 1:1 an Kieselgel chromatographiert, die Produktfraktionen vereinigt und eingedampft. Auf diese Weise wird die Titelverbindung in Form blassgelber Kristalle erhalten, Smp. 182-183°, FAB-MS: 564 [M⁺+H].

### Beispiel 3: 4-(3-[3-Methylureido]propyl)-4,5-dianilinophthalimid

Analog zu Beispiel 1 werden 50 mg (0.1 mmol) 4-(3-[3-Methylureido]propyl)-4,5-dianilinophthalsäuredimethylester in 4 ml Ethylenglykol auf 120° erwärmt, wobei unter Rühren während 24 h Ammoniak-Gas durchgeleitet wird. Das Reaktionsgemisch wird abgekühlt, mit konz. Kochsalzlösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Ethylacetat/Methanol 50:1 in Kieselgel chromatographiert, die Produktfraktionen vereinigt und eingedampft. Auf diese Weise wird die Titelverbindung in Form eines gelben Schaums erhalten, FAB-MS: 444 [M⁺+H], DC (Kieselgel, Ethylacetat/Methanol 20:1) R_{f}=0.40.

Das Ausgangsmaterial wird folgendermassen hergestellt:
a) 4-(3-[3-Methylureido]propyl)-4,5-dianilinophthalsäuredimethylester

Eine Lösung von 43 mg (0.1 mmol) 4-(3-Aminopropyl)-4,5-dianilinophthalsäuredimethylester (Beispiel 2a) in 15 ml THF wird mit 9 µl (0.15 mmol) Methylisocyanat versetzt und 2 h bei RT gerührt. Das Reaktionsgemisch wird eingedampft, in Ethylacetat aufgenommen und die Lösung nacheinander mit 1N HCl und mit Wasser gewaschen, mit Natriumsulfat getrocknet und eingedampft. Auf diese Weise wird die Titelverbindung in Form gelber Kristalle erhalten, FAB-MS: 490 [M⁺+H], DC (Kieselgel, Dichlormethan/Methanol 7:3) R_{f}=0.85.

### Beispiel 4: 4-(2-Aminoethyl)-4,5-dianilinophthalimid

Analog zu Beispiel 1 werden 44 mg (0.1 mmol) 4-(2-Aminoethyl)-4,5-dianilinophthalsäuredimethylester in 4 ml Ethylenglykol auf 120° erwärmt, wobei unter Rühren während 24 h Ammoniak-Gas durchgeleitet wird. Das Reaktionsgemisch wird abgekühlt, mit konz. Kochsalzlösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 10:1 an Kieselgel chromatographiert, die Produktfraktionen vereinigt und eingedampft. Auf diese Weise wird die Titelverbindung in Form gelber Kristalle erhalten, FAB-MS: 373 [M⁺+H].

Das Ausgangsmaterial wird folgendermassen hergestellt:
a) 4-(2-Aminoethyl)-4,5-dianilinophthalsäuredimethylester

Unter Argon werden zu einer Lösung von 377 mg (1 mmol) 4,5-Dianilinophthalsäuredimethylester (Beispiel 1 b) in 10 ml DMF abs. 48 mg (1.1 mmol) Natriumhydrid (Dispersion 60% in Oel, Fluka, Schweiz) gegeben. Bei RT wird eine Lösung von 62 µl (1.2 mmol) Aziridin in 2 ml DMF abs. zugetropft, das Reaktionsgemisch auf 100°C erwärmt und 4 h gerührt. Das Reaktionsgemisch wird abgekühlt, das Lösungsmittel abgedampft, der schwarze Rückstand in Dichlormethan gelöst und die Lösung mit Wasser gewaschen, mit Natriumsulfat getrocknet und eingedampft. Der Eindampfrückstand wird mit Dichlormethan/Methanol 10:1 an Kieselgel chromatographiert, die Produktfraktionen vereinigt, eingedampft und am HV getrocknet. Auf diese Weise wird die Titelverbindung in Form eines schwach gelben Schaums erhalten, FAB-MS: 419 [M⁺+H].

### Beispiel 5: 4-(3-Carbamoylethyl)-4,5-dianilinophthalimid

Analog zu Beispiel 1 werden 46 mg (0.1 mmol) 4-(3-Methyloxycarbonylethyl)-4,5-dianilinophthalsäuredimethylester in 4 ml Ethylenglykol auf 120° erwärmt, wobei unter Rühren während 24 h Ammoniak-Gas durchgeleitet wird. Das Reaktionsgemisch wird abgekühlt, mit konz. Kochsalzlösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Ethylacetat/Hexan 1:1 an Kieselgel chromatographiert, die Produktfraktionen vereinigt und eingedampft. Auf diese Weise wird die Titelverbindung in Form gelber Kristalle erhalten, FAB-MS: 416 [M⁺+H].

Das Ausgangsmaterial wird folgendermassen hergestellt:
a) 4-(3-Methyloxycarbonylethyl)-4,5-dianilinophthalsäuredimethylester

Unter Argon werden zu einer Lösung von 377 mg (1 mmol) 4,5-Dianilinophthalsäuredimethylester (Beispiel 1 b) in 10 ml DMF abs. 48 mg (1.1 mmol) Natriumhydrid (Dispersion 60% in Oel, Fluka, Schweiz) gegeben. Bei RT wird eine Lösung von 134 µl (1.2 mmol) 3-Brompropionsäuremethylester (Fluka, Schweiz) in 2 ml DMF abs. zugetropft, das Reaktionsgemisch auf 100°C erwärmt und 12 h gerührt. Das Reaktionsgemisch wird abgekühlt, das Lösungsmittel abgedampft, der schwarze Rückstand in Dichlormethan gelöst und die Lösung mit Wasser gewaschen, mit Natriumsulfat getrocknet und eingedampft. Der Eindampfrückstand wird mit Dichlormethan/Methanol 10:1 an Kieselgel chromatographiert, die Produktfraktionen vereinigt, eingedampft und am HV getrocknet. Auf diese Weise wird die Titelverbindung in Form von schwach gelben Kristallen erhalten, FAB-MS: 463 [M⁺+H].

### Beispiel 6: 5,8-Diphenyl-5,8-diaza-6-hydroximinomethyl-5,6,7,8-tetrahydronaphthalin-2,3-dicarboximid

Eine Lösung von 386 mg (1 mmol) 5,8-Diphenyl-5,8-diaza-6-hydroxymethyl-5,6,7,8-tetrahydro-naphthalin-2,3-dicarboximid (Beispiel 1) in 20 ml abs. Dichlormethan wird mit 323 mg (1.5 mmol) Pyridiniumchlorochromat (Fluka, Schweiz) in 20 ml abs. Dichlormethan versetzt und 2 h bei RT gerührt. Darauf wird 20 ml Diethylether zugesetzt, durch Celite® (Filterhilfsmittel auf Kieselgurbasis, Fluka, Schweiz) filtriert und mit Dichlormethan/Ether 1:1 nachgewaschen. Das Filtrat wird im kalten Wasserbad auf 20 ml eingeengt, mit 20 ml Methanol versetzt und eine Lösung von 70 mg (1.0 mmol) Hydroxylamin-hydrochlorid in 20 ml Methanol zugegeben. Das Reaktionsgemisch wird 18 h bei RT gerührt, darauf eingedampft und mit Ethylacetat/Hexan 1:1 an Kieselgel chromatographiert. Die Produktfraktionen werden eingedampft und am Hochvakuum getrocknet. Man erhält so die Titelverbindung in Form oranger Kristalle.

### Beispiel 7: 6-Aminomethyl-5,8-diphenyl-5,8-diaza-5,6,7,8-tetrahydronaphthalin-2,3-dicarboximid

Eine Lösung von 40 mg (0.1 mmol) 5,8-Diphenyl-5,8-diaza-6-hydroximinomethyl-5,6,7,8-tetrahydro-naphthalin-2,3-dicarboximid (Beispiel 1) in 5 ml Methanol und 2 ml THF wird an Pd/C mit Wasserstoff hydriert. Das Reaktionsgemisch wird vom Katalysator filtriert, mit Methanol nachgewaschen und eingedampft. Auf diese Weise wird die Titelverbindung in Form gelber Kristalle erhalten, FAB-MS: 385 [M⁺+H].

### Beispiel 8:

Aus der Titelverbindung von Beispiel 1 wird 5,8-Diphenyl-5,8-diaza-5,6,7,8-tetrahydro-naphthalin-2,3-dicarboximid-6-carbonsäure (durch Oxidation mit KMnO₄) hergestellt.

### Beispiel 9:

Ausgehend von Beispiel 8 wird 5,8-Diphenyl-5,8-diaza-5,6,7,8-tetrahydronaphthalin-2,3-dicarboximid-6-carbonsäuremethylester (mit Diazomethan) hergestellt.

### Beispiel 10:

Ausgehend von Beispiel 8 wird 5,8-Diphenyl-5,8-diaza-5,6,7,8-tetrahydronaphthalin-2,3-dicarboximid-6-carbonsäureethylester (mit Ethanol/Dicyclohexylcarbodiimid) hergestellt.

### Beispiel 11:

Analog zu Beispiel 6 wird 5,8-Diphenyl-5,8-diaza-6-hydrazonomethyl-5,6,7,8-tetrahydro-naphthalin-2,3-dicarboximid (durch Umsetzung mit Pyridiniumchlorochromat und anschliessende Reaktion mit Hydrazin) hergestellt.

### Beispiel 12:

Analog zu einem der vorgenannten Beispiele und/oder den genannten Verfahren werden hergestellt:
(a) 5,8-Bis(4-fluorphenyl)-5,8-diaza-6-hydroxymethyl-5,6,7,8-tetra hydro-naphthalin-2,3-dicarboximid
(b) 4-(3-Aminopropyl)-4,5-bis(4-fluoranilino)phthalimid
(c) 4-(3-Methylaminopropyl)-4,5-dianilinophthalimid (aus Beispiel 2a durch Umsetzung mit Methyljodid und Weiterreaktion des 4-(3-Methylaminopropyl)-4,5-dianilinophthalsäuredimethylesters analog Beispiel 2)
(d) 4-(3-Aminoethyl)-4,5-bis(4-fluoranilino)phthalimid
(e) 4-(3-Carbamoylpropyl)-4,5-bis(4-fluoranilino)phthalimid
(f) 5,8-Bis(4-fluorphenyl)-5,8-diaza-6-hydroximinomethyl-5,6,7,8-tetrahydro-naphthalin-2,3-dicarboximid.
(g) 6-Aminomethyl-5,8-bis(4-fluorphenyl)-5,8-diaza-5,6,7,8-tetrahydro-naphthalin-2,3-dicarboximid
(h) 5,8-Bis(4-fluorphenyl)-5,8-diaza-5,6,7,8-tetrahydro-naphthalin-2,3-dicarboximid-6-carbonsäure
(i) 5,8-Bis(4-fluorphenyl)-5,8-diaza-5,6,7,8-tetrahydro-naphthalin-2,3-dicarboximid--6-carbonsäuremethylester
(j) 5,8-Bis(4-fluorphenyl)-5,8-diaza-5,6,7,8-tetrahydro-naphthalin-2,3-dicarboximid-6-carbonsäureethylester
(k) 5,8-Bis(4-fluorphenyl)-5,8-diaza-6-hydrazonomethyl-5,6,7,8-tetrahydro-naphthalin-2,3-dicarboximid
(l) 4-(3-Amidinopropyl)-4,5-dianilinophthalimid
(m) 4-(3-Methylthioiminopropyl)-4,5-dianilinophthalimid
(n) 4-(3-Carbamoylpropyl)-4,5-dianilinophthalimid
(o) 4-(3-Ureidopropyl)-4,5-dianilinophthalimid
(p) 4-(3-[3-Ethylureido]propyl)-4,5-dianilinophthalimid
(q) 4-(3-Aminobutyl)-4,5-dianilinophthalimid
(r) 4-(3-[3-Methylthioureido]ethyl)-4,5-dianilinophthalimid
(s) 4-(3-[N'-Ethylthioureido]ethyl)-4,5-dianilinophthalimid
(t) 4-(3-[3-Methylthioureido]propyl)-4,5-dianilinophthalimid und (u) 4-(3-[Thioureido]propyl)-4,5-dianilinophthalimid

Analog zu Beispiel 1 werden 180 mg (0.36 mmol) 4-(3-[3-Methylthioureido]propyl)-4,5-dianilinophthalsäuredimethylester in 5 ml Ethylenglykol auf 120° erwärmt, wobei unter Rühren während 24 h Ammoniak-Gas durchgeleitet wird. Das Reaktionsgemisch wird abgekühlt, mit konzentrierter Kochsalzlösung versetzt und mit Ethylacetat extrahiert. Die Ethylacetat-Phasen werden nacheinander dreimal mit Wasser und einmal mit gesättigter Kochsalzlösung gewaschen, mit Natriumsulfat getrocknet und eingedampft. Der Eindampfrückstand wird mit Dichlormethan/Methanol 60:1 an Kieselgel chromatographiert, die zuerst eluierenden Produktfraktionen vereinigt und eingedampft. Auf diese Weise wird die Titelverbindung (t) in Form eines gelben Schaums erhalten, FAB-MS: 460 [M⁺+H], DC (Kieselgel, Dichlormethan/Methanol 10:1) R_{f}=0.50. Die darauf eluierenden Fraktionen werden vereinigt und eingedampft. Auf diese Weise wird die Titelverbindung (u) in Form eines gelben Schaums erhalten, FAB-MS: 446 [M⁺+H], DC (Kieselgel, Dichlormethan/Methanol 10:1) R_{f}=0.39.

Das Ausgangsmaterial wird folgendermassen hergestellt:
a) 4-(3-[3-Methylthioureido]propyl)4,5-dianilinophthalsäuredimethylester

Eine Lösung von 220 mg (0.5 mmol) 4-(3-Aminopropyl)-4,5-dianilinophthalsäuredimethylester (Beispiel 2a) in 5 ml Dichlormethan wird mit 70 µl (1 mmol) Methylisothiocyanat versetzt und 4 h bei RT gerührt. Das Reaktionsgemisch wird eingedampft und der Eindampfrückstand mit Ethylacetat an Kieselgel chromatographiert. Die Produktfraktionen werden vereinigt und eingedampft. Auf diese Weise wird die Titelverbindung in Form eines gelben Schaums erhalten, FAB-MS: 507 [M⁺+H], DC (Kieselgel, Dichlormethan/Methanol 10:1) R_{f}=0.68.
(v) 4-(3-[3-Ethylthioureido]propyl)-4,5-dianilinophthalimid
(w) 4-(3-Guanidinylethyl)-4,5-dianilinophthalimid
(x) 4-(3-Guanidinylpropyl)-4,5-dianilinophthalimid
(y) 4-(3-Amidinopropyl)-4,5-bis(4-fluoranilino)phthalimid
(z) 4-(3-Methylthioiminopropyl)-4,5-bis(4-fluoranilino)phthalimid

### Beispiel 13:

Analog zu einem der vorgenannten Beispiele und/oder den genannten Verfahren werden hergestellt:
(a) 4-(3-Carbamoylpropyl)-4,5-bis(4-fluoranilino)phthalimid
(b) 4-(3-Ureidopropyl)-4,5-bis(4-fluoranilino)phthalimid (aus der Titelverbindung von Beispiel 2a) durch Umsetzung mit Carbonyldiimidazol und anschliessend mit Ammoniak)
(c) 4-(3-Thioureidopropyl)4,5-bis(4-fluoranilino)phthalimid
(d) 4-(3-[3-Methylureido]ethyl)-4,5-bis(4-fluoranilino)phthalimid
(e) 4-(3-[3-Ethylureido]ethyl)-4,5-bis(4-fluoranilino)phthalimid
(f) 4-(3-[3-Methylureido]propyl)-4,5-bis(4-fluoranilino)phthalimid
(g) 4-(3-[3-Ethylureido]propyl)-4,5-bis(4-fluoranilino)phthalimid
(h) 4-(3-Aminobutyl)-4,5-bis(4-fluoranilino)phthalimid
(i) 4-(3-[3-Methylthioureido]ethyl)-4,5-bis(4-fluoranilino)phthalimid
(j) 4-(3-[3-Ethylthioureido]ethyl)-4,5-bis(4-fluoranilino)phthalimid
(k) 4-(3-[3-Methylthioureido]propyl)-4,5-bis(4-fluoranilino)phthalimid
(l) 4-(3-[3-Ethylthioureido]propyl)-4,5-bis(4-fluoranilino)phthalimid
(m) 4-(3-Guanidinylethyl)-4,5-bis(4-fluoranilino)phthalimid
(n) 4-(3-Guanidinylpropyl)-4,5-bis(4-fluoranilino)phthalimid.

### Beispiel 14:

Analog einem der genannten Beispiele und/oder den genannten Verfahren wird hergestellt:
(A) 4-(3-Acetylaminopropyl)-4,5-dianilinophthalimid:
   Analog zu Beispiel 1 werden 200 mg (0.46 mmol) 4-(3-Acetylaminopropyl)-4,5-dianilinophthalsäureanhydrid in 10 ml Ethylenglykol auf 120° erwärmt, wobei unter Rühren während 3 h Ammoniak-Gas durchgeleitet wird. Das Reaktionsgemisch wird abgekühlt, mit konzentrierter Kochsalzlösung versetzt und mit Ethylacetat extrahiert. Die Ethylacetat-Phasen werden nacheinander dreimal mit Wasser und einmal mit gesättigter Kochsalzlösung gewaschen, mit Natriumsulfat getrocknet und eingedampft. Der Eindampfrückstand wird mit Ethylacetat an Kieselgel chromatographiert, die Produktfraktionen vereinigt und eingedampft. Auf diese Weise wird die Titelverbindung in Form eines gelben Schaums erhalten, FAB-MS: 429 [M⁺+H], DC (Kieselgel, Ethylacetat) R_{f}=0.30.
   Das Ausgangsmaterial wird folgendermassen hergestellt:
   a) 4-(3-Acetylaminopropyl)-4,5-dianilinophthalsäureanhydrid
      Eine Suspension von 390 mg (0.9 mmol) 4-(3-Aminopropyl)-4,5-dianilinophthalsäuredimethylester (Beispiel 2a), 500 mg Natriumhydroxid, 10 mg Natriumascorbat, 25 ml 2N Natronlauge und 10 ml Methanol wird unter Argon während 16 h am Rückfluss gerührt. Das Reaktionsgemisch wird mit 1N Salzsäure auf pH 1 gestellt und am Rotationsverdampfer eingedampft. Der Rückstand wird mit Aceton digeriert, filtriert und trocken gesaugt, mit 50 ml Acetanhydrid versetzt und während 2 h auf 70°C erhitzt. Die gelbe Suspension wird filtriert und das Filtrat eingedampft. Der Eindampfrückstand wird mit Ethylacetat an Kieselgel chromatographiert, die Produktfraktionen vereinigt und eingedampft. Auf diese Weise wird die Titelverbindung in Form eines gelben Oels erhalten, FAB-MS: 430 [M⁺+H], DC (Kieselgel, Ethylacetat) R_{f}=0.38.
   (B) 4-(3-Acetylaminopropyl)-4,5-bis(4-fluoranilino)phthalimid.

### Beispiel 15: (A) 1,3-Diphenyl-2-ethyloxycarbonyl-2,3-dihydrobenzimidazol-5, 6-dicarboximid und (B)1,3-Diphenyl-2-carboxy-2,3-dihydrobenzimidazol-5, 6-dicarboximid

Zu einer Lösung von 330 mg (1 mmol) 4,5-dianilinophthalimid in 5 ml Dimethoxyethan werden unter Argon 2 mg Zinn(II)chlorid-hydrat und 1000 µl (5.5 mmol) Diethoxyessigsäureethylester (Fluka, Schweiz) zugegeben. Das Reaktionsgemisch wird 16 h bei RT gerührt, filtriert, an Kieselgel adsorbiert und mit Ethylacetat/Hexan 1:2 chromatographiert. Die zuerst eluierten Produktfraktionen werden eingedampft, der Rückstand aus Ethylacetat/Hexan umkristallisiert und am Hochvakuum getrocknet. Auf diese Weise wird die Titelverbindung (A) in Form gold-gelber Kristalle erhalten, FAB-MS: 414 [M⁺+H], DC (Kieselgel, Ethylacetat/Hexan 2:1) R_{f}=0.23. Die danach eluierten Produktfraktionen werden eingedampft, der Rückstand aus Ethylacetat/Hexan umkristallisiert und am Hochvakuum getrocknet. Auf diese Weise wird die Titelverbindung (b) in Form gold-gelber glänzender Kristalle erhalten, FAB-MS: 368 [M⁺+H], DC (Kieselgel, Ethylacetat/Hexan 2:1) R_{f}=0.15.
Das Ausgangsmaterial wird folgendermassen hergestellt:
a) 4,5-Bis(anilino)phthalimid
   Eine Suspension von 230 mg (0,7 mmol) 4,5-Bis(anilino)phthalsäuredimethylester in 23 ml Ethylenglykol wird auf 120° erwärmt; unter Rühren wird während 24 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 40:1 an Kieselgel chromatographiert, die Produktfraktionen vereinigt und eingedampft. Auf diese Weise wird die Titelverbindung in Form gelber Kristalle erhalten, Smp. 215-217°, FAB-MS: 330 [M⁺+H].
b) 4,5-Bis(trimethylsilyloxy)cyclohexa-1,4-dien-1,2-dicarbonsäuredimethylester
   Unter Argon wird eine Lösung von 7,1 g (50 mmol) Acetylendicarbonsäuredimethylester in 30 ml Toluol zu 12,5 g (50 mmol) 95% 2,3-Bis(trimethylsilyloxy)-1,3-butadien getropft und dann 19 h am Rückfluss gekocht. Das Reaktionsgemisch wird abgekühlt, das Lösungsmittel abgedampft und der Rückstand im Hochvakuum (0,1 mbar, 124-127°) destilliert. Auf diese Weise wird die Titelverbindung als gelbes, hochviskoses Oel erhalten, ¹H-NMR (CDCl₃): δ = 0.18 (s, 18H), 3.09 (s, 4H), 3.78 (s, 6H).
c) 4,5-Bis(anilino)phthalsäuredimethylester
   Eine Lösung von 5,6 g (15 mmol) 4,5-Bis(trimethylsilyloxy)cyclohexa-1,4-dien-1,2-dicarbonsäuredimethylester und 5,5 ml (60 mmol) Anilin in 60 ml Eisessig wird während 4 h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Das Rohprodukt wird aus Ethanol umkristallisiert. Auf diese Weise wird die Titelverbindung in Form gelber Kristalle erhalten, Smp. 178°, FAB-MS: 377 [M⁺+H].

### Beispiel 16:

Es werden 5000 Kapseln hergestellt, die je 0,25 g Wirkstoff, z.B. eine der in den Beispielen 1-15 hergestellten Verbindungen, enthalten:

### Zusammensetzung

- Wirkstoff: 1250 g
- Talk: 180 g
- Weizenstärke: 120 g
- Magnesiumstearat: 80 g
- Laktose: 20 g

### Verfahren:

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Patentansprüche

1. Verbindungen der Formel I, 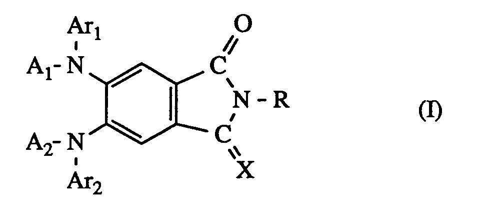 worin A₁ und A₂ unabhängig voneinander substituiertes Niederalkyl, substituiertes Niederalkenyl, substituiertes Niederalkinyl oder Wasserstoff bedeuten, wobei maximal einer der Reste A₁ und A₂ Wasserstoff bedeuten kann, oder A₁ und A₂ zusammen durch Substituenten ausser Niederalkyl und ausser Hydroxy substituiertes Niederalkylen bedeuten; Ar₁ und Ar₂ unabhängig voneinander Aryl, Heteroaryl oder unsubstituiertes oder substituiertes Cycloalkyl bedeuten, die Gruppe -C(=X)- für -C(=O)-, -C(=S)-, -CH₂- oder -C(=CR₁R₂)- steht, wobei R₁ und R₂ unabhängig voneinander Wasserstoff oder Niederalkyl bedeuten, und R Wasserstoff, Niederalkyl, Arylniederalkyl, Aryl, Amino, Hydroxy oder Niederalkoxy bedeutet, Salze davon, sofern salzbildende Gruppen vorliegen, und/oder Tautomere davon, sofern tautomerisierbare Reste vorliegen.

2. Verbindungen der Formel I gemäss Anspruch 1, worin A₁ und A₂ unabhängig voneinander Wasserstoff, Niederalkyl, welches durch bis zu 2 Reste ausgewählt aus Amino, Mono- oder Diniederalkylamino, Cycloalkylamino, Phenylniederalkylamino, Phenylamino, Niederalkanoylamino, Phenylniederalkanoylamino, Phenylcarbonylamino, Hydroxy, Niederalkoxy, Phenylniederalkoxy, Niederalkanoyloxy, Mercapto, Niederalkylthio, Phenylniederalkylthio, Niederalkanoylthio, Carboxy, Niederalkoxycarbonyl, Phenylniederalkoxycarbonyl, Cyano, Carbamoyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, Thioureido, an einem oder beiden Stickstoffatomen durch Niederalkyl, Aryl oder Arylniederalkyl substituiertes Thioureido, Hydrazino, an einem oder beiden Stickstoffatomen durch Niederalkyl, Aryl oder Arylniederalkyl substituiertes Hydrazino, Amidino, an einem oder beiden Stickstoffatomen durch Niederalkyl, Aryl oder Arylniederalkyl substituiertes Amidino, Guanidino, an einem, zwei oder allen drei Stickstoffatomen durch Niederalkyl, Aryl oder Arylniederalkyl substituiertes Guanidino, Oxo, welches nicht am Kohlenstoff gebunden ist, welcher an den A₁ oder A₂ tragenden Stickstoff gebunden ist, Thioxo, Imino, Niederalkylimino, Niederalkanoylimino, Hydroxyimino, Niederalkoxyimino, Hydrazono, N-Mono- oder N,N-Diniederalkylhydrazono, N-Niederalkanoylhydrazono, Niederalkoxycarbonylhydrazono und Niederalkylthioimino substituiert ist, oder Heterocyclylniederalkyl, worin Heterocyclyl unsubstituiertes oder durch Niederalkyl, Niederalkanoyl, Hydroxy, Niederalkoxy, Halogen, Cyano und/oder Trifluormethyl substituiertes Pyrrolyl, 2,5-Dihydropyrrolyl, Pyrrolinyl, Imidazolyl, Imidazolidinyl, Pyrazolinyl, Pyrazolidinyl, Triazolyl, Tetrazolyl, Tetrahydro-oxazolyl, Tetrahydroisoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, welches endständig an den Niederalkylrest gebunden ist, bedeutet, wobei maximal einer der Reste A₁ und A₂ Wasserstoff bedeutet, oder worin A₁ und A₂ gemeinsam Niederalkylen, welches durch bis zu 3 Substituenten ausgewählt aus Amino, Aminoniederalkyl, Mono- oder Diniederalkylamino, Mono- oder Diniederalkylaminoniederalkyl, Cycloalkylamino, Cycloalkylaminoniederalkyl, Phenylniederalkylamino, Phenylniederalkylaminoniederalkyl, Phenylamino, Phenylaminoniederalkyl, Niederalkanoylamino, Phenylniederalkanoylamino, Phenylcarbonylamino, Niederalkanoylaminoniederalkyl, Phenylniederalkanoylaminoniederalkyl, Phenylcarbonylaminoniederalkyl, Hydroxyniederalkyl, Niederalkoxy, Niederalkoxyniederalkyl, Niederalkoxyniederalkoxy, Niederalkoxyniederalkoxyniederalkyl, Phenylniederalkoxy, Phenylniederalkoxyniederalkyl, Niederalkanoyloxy, Niederalkanoyloxyniederalkyl, Mercapto, Mercaptoniederalkyl, Niederalkylthio, Niederalkylthioniederalkyl, Niederalkylthioniederalkylthioniederalkyl, Phenylniederalkylthio, Phenylniederalkylthioniederalkyl, Niederalkanoylthio, Niederalkanoylthioniederalkyl, Carboxy, Carboxyniederalkyl, Niederalkoxycarbonyl, Niederalkoxycarbonylniederalkyl, Phenylniederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylnieder-alkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl, Aryl oder Arylniederalkyl substituiertes Ureido oder Ureidoniederalkyl, Thioureido, Thioureidoniederalkyl, an einem oder beiden Stickstoffatomen durch Niederalkyl, Aryl oder Arylniederalkyl substituiertes Thioureido oder Thioureidoniederalkyl, Hydrazino, Hydrazinoniederalkyl, an einem oder beiden Stickstoffatomen durch Niederalkyl, Aryl oder Arylniederalkyl substituiertes Hydrazino oder Hydrazinoniederalkyl, Amidino, Amidinoniederalkyl, an einem oder beiden Stickstoffatomen durch Niederalkyl, Aryl oder Arylniederalkyl substituiertes Amidino oder Amidinoniederalkyl, Guanidino, Guanidinoniederalkyl, an einem, zwei oder allen drei Stickstoffatomen durch Niederalkyl, Aryl oder Arylniederalkyl substituiertes Guanidino oder Guanidinoniederalkyl, Oxo, Oxoniederalkyl, Thioxo, Thioxoniederalkyl, Imino, Iminoniederalkyl, Niederalkylimino, Niederalkyliminoniederalkyl, Niederalkanoylimino, Niederalkanoyliminoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederalkanoylhydrazononiederalkyl, Niederalkoxycarbonylhydrazononiederalkyl, Niederalkylthioimino und Niederalkylthioiminoniederalkyl substituiert ist, bedeuten; Ar₁ und Ar₂ unabhängig voneinander Aryl, unsubstituiertes oder durch Niederalkyl, Hydroxy, Niederalkoxy, Halogen, Cyano und/oder Trifluormethyl substituiertes Imidazolyl, Triazolyl, Pyridyl, Pyrimidinyl oder Triazinyl oder unsubstituiertes oder durch Niederalkyl oder Hydroxy substituiertes Cycloalkyl bedeuten; die Gruppe -C(=X)- für -(C=O)-, -C(=S)- oder -CH₂- steht; und R Wasserstoff, Niederalkyl, Amino, Hydroxy oder Niederalkoxy bedeutet; wobei
Aryl unsubstituiertes oder substituiertes Phenyl oder Naphthyl bedeutet, worin die Substituenten aus einem oder mehreren Substituenten aus der Gruppe bestehend aus Niederalkyl, Niederalkenyl, Niederalkinyl, Niederalkylen (angeknüpft an zwei benachbarten C-Atomen), Cycloalkyl, Phenylniederalkyl oder Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; Niederalkoxy, Halogenniederalkoxy, Phenylniederalkoxy, Phenyloxy, Niederalkenyloxy, Halogenniederalkenyloxy, Niederalkinyloxy oder Niederalkylendioxy (angeknüpft an zwei benachbarten C-Atomen); Niederalkanoyloxy, Phenylniederalkanoyloxy, Phenylcarbonyloxy; Mercapto; Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl; Halogen, Nitro, Amino; Niederalkylamino, Cycloalkylamino, Phenylniederalkylamino, Phenylamino; Diniederalkylamino, N-Niederalkyl-N-phenylamino, N-Niederalkyl-N-phenylniederalkylamino, Niederalkylenamino, Niederalkanoylamino, Phenylniederalkanoylamino, Phenylcarbonylamino, Niederalkanoyl, Phenylniederalkanoyl; Phenylcarbonyl; Carboxy; Niederalkoxycarbonyl; Carbamoyl, N-Niederalkylcarbamoyl, N,N-Diniederalkylcarbamoyl, N-Hydroxycarbamoyl, N-Phenylcarbamoyl; Cyano, durch zwei unabhängig voneinander aus Hydroxy, Niederalkoxy oder Phenylniederalkoxy, wie Benzyloxy, ausgewählte Reste am Phosphor substituiertes Phosphoryloxy oder durch Phenylen-1,2-dioxy am Phosphor substituiertes Phosphoryloxy, Sulfo; Niederalkoxysulfonyl; Sulfamoyl, N-Niederalkylsulfamoyl, N,N-Diniederalkylsulfamoyl und N-Phenylsulfamoyl, wobei in den Substituenten vorkommende Phenylgruppen jeweils unsubstituiert oder durch Niederalkyl, Niederalkoxy, Hydroxy, Halogen und/oder Trifluormethyl substituiert sind, ausgewählt sind; und worin
Cycloalkyl C₃-C₈-Cycloalkyl bedeutet;
und pharmazeutisch verwendbare Salze dieser Verbindungen, sofern salzbildende Gruppen vorliegen.

3. Verbindungen der Formel I gemäss Anspruch 1, worin A₁ und A₂ unabhängig voneinander aus Wasserstoff und Niederalkyl, welches durch bis zu 2 Reste ausgewählt aus Amino, Mono- oder Diniederalkylamino, Niederalkanoylamino, Hydroxy, Carboxy, Niederalkoxycarbonyl, Phenylniederalkoxycarbonyl, Carbamoyl, N-Niederalkylcarbamoyl, N,N-Diniederalkylcarbamoyl, Ureido, an einem oder beiden Stickstoffatomen durch Niederalkyl substituiertes Ureido, Thioureido, an einem oder beiden Stickstoffatomen durch Niederalkyl substituiertes Thioureido, Amidino, an einem oder beiden Stickstoffatomen durch Niederalkyl substituiertes Amidino, Guanidino, an einem, zwei oder allen drei Stickstoffatomen durch Niederalkyl substituiertes Guanidino, Oxo, welches nicht am Kohlenstoff gebunden ist, welcher an den A₁ oder A₂ tragenden Stickstoff gebunden ist, Hydroxyimino, Niederalkoxyimino, Hydrazono, N-Mono- oder N,N-Diniederalkylhydrazono, N-Niederalkanoylhydrazono, Niederalkoxycarbonylhydrazono und Niederalkylthioimino ausgewählt sind, wobei maximal einer der beiden Reste A₁ und A₂ Wasserstoff bedeuten kann, oder A₁ und A₂ zusammen Niederalkylen, welches durch einen oder bis zu 3 Substituenten ausgewählt aus Amino, Aminoniederalkyl, Mono- oder Diniederalkylamino, Mono- oder Diniederalkylaminoniederalkyl, Hydroxyniederalkyl, Carboxy, Carboxyniederalkyl, Niederalkoxycarbonyl, Niederalkoxycarbonylniederalkyl, Phenylniederalkoxycarbonylniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, Ureido, Ureidoniederalkyl, an einem oder beiden Stickstoffatomen durch Niederalkyl substituiertes Ureido oder Ureidoniederalkyl, Thioureido, Thioureidoniederalkyl, an einem oder beiden Stickstoffatomen durch Niederalkyl substituiertes Thioureido oder Thioureidoniederalkyl, Amidino, Amidinoniederalkyl, an einem oder beiden Stickstoffatomen durch Niederalkyl substituiertes Amidino oder Amidinoniederalkyl, Guanidino, Guanidinoniederalkyl, an einem, zwei oder allen drei Stickstoffatomen durch Niederalkyl substituiertes Guanidino oder Guanidinoniederalkyl, Oxo, Oxoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederalkanoylhydrazononiederalkyl, Niederalkoxycarbonylhydrazononiederalkyl, Niederalkylthioimino und Niederalkylthioiminoniederalkyl substituiert ist, bedeutet; Ar₁ und Ar₂ Phenyl, das unsubstituiert oder durch einen Substituenten ausgewählt aus Trifluormethyl, Hydroxy, Niederalkoxy, Halogenniederalkoxy, Phenylniederalkoxy, Phenyloxy, Niederalkanoyloxy, Phenylniederalkanoyloxy, Phenylcarbonyloxy, Cyano, Sulfo und Sulfamoyl substituiert oder oder bis zu fünfmal durch Halogen substituiert ist, die Gruppe -(C=X)- für -(C=O)- oder -(C=S)- steht, und R Wasserstoff oder Niederalkyl bedeutet, und pharmazeutisch verwendbare Salze davon, sofern salzbildende Gruppen vorliegen.

4. Verbindungen der Formel I gemäss Anspruch 1, worin A₁ und A₂ unabhängig voneinander Wasserstoff oder Niederalkyl, welches durch einen Rest ausgewählt aus Amino, Niederalkanoylamino, Carbamoyl, Ureido, an einem oder beiden Stickstoffatomen durch Niederalkyl substituiertes Ureido, Thioureido, an einem oder beiden Stickstoffatomen durch Niederalkyl substituiertes Thioureido, Amidino, Guanidino und Niederalkylthioimino substituiert ist, bedeuten, wobei maximal einer der Reste A₁ und A₂ Wasserstoff bedeuten kann, oder worin A₁ und A₂ zusammen Niederalkylen, welches durch einen Substituenten ausgewählt aus Amino, Aminoniederalkyl, Hydroxyniederalkyl, Carboxy, Carboxyniederalkyl, Niederalkoxycarbonyl, Niederalkoxycarbonylniederalkyl, Niederalkanoyl, Hydroxyimino, Hydroxyiminoniederalkyl, Hydrazono und Hydrazononiederalkyl substituiert ist, bedeuten; Ar₁ und Ar₂ Phenyl oder 4-Halogenphenyl bedeuten; die Gruppe -(C=X)- für -(C=O)- steht; und R Wasserstoff bedeutet, und pharmazeutisch verwendbare Salze davon, sofern salzbildende Gruppen vorliegen.

5. Verbindungen der Formel I gemäss Anspruch 1, worin A₁ und A₂ unabhängig voneinander 2-Aminoethyl, 3-Aminopropyl, 4-Aminobutyl, 3-Acetylaminopropyl, 2-Carbamoylethyl, 3-Carbamoylpropyl, 2-(3-Methylureido)-ethyl, 2-(3-Ethylureido)-ethyl, 3-Ureidopropyl, 3-Thioureidopropyl, 3-(3-Methylureido)-propyl, 3-(3-Ethylureido)-propyl, 2-(3-Methylthioureido)-ethyl, 2-(3-Ethylthioureido)-ethyl, 3-(3-Methylthioureido)-propyl, 3-(3-Ethylthioureido)-propyl, 2-Amidinoethyl, 3-Amidinopropyl, 2-Guanidinoethyl, 3-Guanidinopropyl, 3-(N-Methylthioimino)-propyl oder Wasserstoff bedeuten, wobei maximal einer der Reste A₁ und A₂ Wasserstoff bedeuten kann, oder worin A₁ und A₂ zusammen in 1-Stellung durch Aminomethyl, 3-Aminopropyl, Hydroxymethyl, Carboxy, Methoxycarbonyl, Ethoxycarbonyl, Propionyl, Hydroxyiminomethyl oder Hydrazonomethyl substituiertes Methylen oder Ethylen bedeuten; Ar₁ und Ar₂ unabhängig voneinander Phenyl oder 4-Fluorphenyl bedeuten; die Gruppe -(C=X)- für -(C=O)- steht; und R Wasserstoff bedeutet, und pharmazeutisch verwendbare Salze davon, sofern salzbildende Gruppen vorliegen.

6. Verbindungen der Formel I gemäss Anspruch 1, worin A₁ und A₂ unabhängig voneinander 2-Aminoethyl, 3-Aminopropyl, 2-Carbamoylethyl, 3-(3-Methylureido)-propyl oder Wasserstoff bedeuten, wobei maximal einer der Reste A₁ und A₂ Wasserstoff bedeuten kann, oder worin A₁ und A₂ zusammen in 1-Stellung durch Aminomethyl, Hydroxymethyl, Carboxy, Methoxycarbonyl, Ethoxycarbonyl, Propionyl, Hydroxyiminomethyl oder Hydrazonomethyl substituiertes Methylen oder Ethylen bedeuten; Ar₁ und Ar₂ Phenyl bedeuten; die Gruppe -(C=X)- für -(C=O)- steht; und R Wasserstoff bedeutet, und pharmazeutisch verwendbare Salze davon, sofern salzbildende Gruppen vorliegen.

7. 5,8-Diphenyl-5,8-diaza-6-hydroxymethyl-5,6,7,8-tetrahydronaphthalin-2,3-di-carboximid der Formel I gemäss Anspruch 1.

8. 4-(3-Aminopropyl)-4,5-dianilinophthalimid der Formel I gemäss Anspruch 1, oder ein pharmazeutisch annehmbares Salz davon.

9. 4-(3-[3-Methylureido]propyl)-4,5-dianilinophthalimid der Formel I gemäss Anspruch 1.

10. 4-(2-Aminoethyl)-4,5-dianilinophthalimid der Formel I gemäss Anspruch 1, oder ein pharmazeutisch annehmbares Salz davon.

11. 4-(3-Carbamoylethyl)-4,5-dianilinophthalimid der Formel I gemäss Anspruch 1.

12. 5,8-Diphenyl-5,8-diaza-6-hydroximinomethyl-5,6,7,8-tetrahydro-naphthalin-2,3-di-carboximid der Formel I gemäss Anspruch 1, oder ein pharmazeutisch annehmbares Salz davon.

13. 6-Aminomethyl-5,8-diphenyl-5,8-diaza-5,6,7,8-tetrahydro-naphthalin-2,3-dicarbox-imid der Formel I gemäss Anspruch 1, oder ein pharmazeutisch annehmbares Salz davon.

14. 5,8-Diphenyl-5,8-diaza-5,6,7,8-tetrahydro-naphthalin-2,3-dicarboximid-6-carbonsäure der Formel I gemäss Anspruch 1, oder ein pharmazeutisch annehmbares Salz davon.

15. 5,8-Diphenyl-5,8-diaza-5,6,7,8-tetrahydro-naphthalin-2,3-dicarboximid-6-carbonsäuremethylester der Formel I gemäss Anspruch 1.

16. 5,8-Diphenyl-5,8-diaza-5,6,7,8-tetrahydro-naphthalin-2,3-dicarboximid-6-carbonsäureethylester der Formel I gemäss Anspruch 1.

17. 5,8-Diphenyl-5,8-diaza-6-hydrazonomethyl-5,6,7,8-tetrahydro-naphthalin-2,3-dicarboximid der Formel I gemäss Anspruch 1, oder ein pharmazeutisch annehmbares Salz davon.

18. 4-(3-Acetylaminopropyl)-4,5-dianilinophthalimid der Formel I gemäss Anspruch 1.

19. 4-(3-[3-Methylthioureido]propyl)-4,5-dianilinophthalimid der Formel I gemäss Anspruch 1.

20. 4-(3-Thioureidopropyl)-4,5-dianilinophthalimid der Formel I gemäss Anspruch 1.

21. 1,3-Diphenyl-2-ethyloxycarbonyl-2,3-dihydrobenzimidazol-5,6-dicarboximid der Formel I gemäss Anspruch 1.

22. 1,3-Diphenyl-2-carboxy-2,3-dihydrobenzimidazol-5,6-dicarboximid der Formel I gemäss Anspruch 1, oder ein pharmazeutisch verwendbares Salz davon

23. Eine Verbindung der Formel I gemäss Anspruch 1 ausgewählt aus 5,8-Bis(4-fluorphenyl)-5,8-diaza-6-hydroxymethyl-5,6,7,8-tetrahydro-naphthalin-2,3-dicarboximid, 4-(3-Aminopropyl)-4,5-bis(4-fluoranilino)phthalimid, 4-(3-Methylaminopropyl)-4,5-dianilinophthalimid, 4-(3-Aminoethyl)-4,5-bis(4-fluoranilino)phthalimid, 4-(3-Carbamoylpropyl)-4,5-bis(4-fluoranilino)phthalimid, 5,8-Bis(4-fluorphenyl)-5,8-diaza-6-hydroxy-2,3-dicarboximid, 6-Aminomethyl-5,8-bis(4-fluorphenyl)-5,8-diaza-5,6,7,8-tetrahydro-naphthalin-2,3-dicarboximid, 5,8-Bis(4-fluorphenyl)-5,8-diaza-5,6,7,8-tetrahydro-naphthalin-2,3-dicarboximid-6-carbonsäure, 5,8-Bis(4-fluorphenyl)-5,8-diaza-5,6,7,8-tetrahydro-naphthalin-2,3-dicarboximid-6-carbonsäuremethylester, 5,8-Bis(4-fluorphenyl)-5,8-diaza-5,6,7,8-tetrahydro-naphthalin-2,3-dicarboximid-6-carbonsäureethylester, 5,8-Bis(4-fluorphenyl)-5,8-diaza-6-hydrazonomethyl-5,6,7,8-tetrahydro-naphthalin-2,3-dicarboximid, 4-(3-Amidinopropyl)-4,5-dianilinophthalimid, 4-(3-Methylthioiminopropyl)-4,5-dianilinophthalimid, 4-(3-Carbamoylpropyl)-4,5-dianilinophthalimid, 4-(3-Ureidopropyl)-4,5-dianilinophthalimid, 4-(3-[3-Ethylureido]propyl)-4,5-dianilinophthalimid, 4-(3-Aminobutyl)-4,5-dianilinophthalimid, 4-(3-[3-Methylthioureido]ethyl)-4,5-dianilinophthalimid, 4-(3-[N'-Ethylthioureido]ethyl)-4,5-d-anilinophthalimid, 4-(3-[3-Ethylthioureido]propyl)4,5-dianilinophthalimid, 4-(3-Guanidinylethyl)-4,5-dianilinophthalimid, 4-(3-Guanidinylpropyl)-4,5-dianilinophthalimid, 4-(3-Amidinopropyl)-4,5-bis(4-fluoranilino)phthalimid, 4-(3-Methylthioiminopropyl)-4,5-bis(4-fluoranilino)phthalimid, 4-(3-Carbamoylpropyl)-4,5-bis(4-fluoranilino)phthalimid, 4-(3-Ureidopropyl)-4,5-bis(4-fluoranilino)phthalimid, 4-(3-Thioureidopropyl)-4,5-bis(4-fluoranilino)phthalimid 4-(3-[3-Methylureido]ethyl)-4,5-bis(4-fluoranilino)phthalimid, 4-(3-[3-Ethylureido]ethyl)-4,5-bis(4-fluoranilino)phthalimid, 4-(3-[3-Methylureido]propyl)-4,5-bis(4-fluoranilino)phthalimid, 4-(3-[3-Ethylureido]propyl)-4,5-bis(4-fluoranilino)phthalimid, 4-(3-Aminobutyl)-4,5-bis(4-fluoranilino)phthalimid, 4-(3-[3-Methylthioureido]ethyl)-4,5-bis(4-fluoranilino)phthalimid, 4-(3-[3-Ethylthioureido]ethyl)-4,5-bis(4-fluoranilino)phthalimid, 4-(3-[3-Methylthioureido]propyl)-4,5-bis(4-fluoranilino)phthalimid, 4-(3-[3-Ethylthioureido]propyl)-4,5-bis-(4-fluoranilino)phthalimid, 4-(3-Guanidinylethyl)4,5-bis(4-fluoranilino)phthalimid, 4-(3-Guanidinylpropyl)-4,5-bis(4-fluoranilino)phthalimid und 4-(3-Acetylaminopropyl)-4,5-bis(4-fluoranilino)phthalimid, und pharmazeutisch annehmbare Salze davon, sofern salzbildende Gruppen vorliegen.

24. Pharmazeutische Präparate enthaltend Verbindungen der Formel I oder ein pharmazeutisch verwendbares Salz einer solchen Verbindung mit mindestens einer salzbildenden Gruppe gemäss einem der Ansprüche 1 bis 23 zusammen mit pharmazeutisch verwendbarem Trägermaterial.

25. Eine Verbindung der Formel I oder ein pharmazeutisch verwendbares Salz einer solchen Verbindung mit mindestens einer salzbildenden Gruppe gemäss einem der Ansprüche 1 bis 23 zur Anwendung in einem Verfahren zur therapeutischen Behandlung des tierischen oder menschlichen Körpers.

26. Verwendung einer Verbindung der Formel I oder eines pharmazeutisch verwendbaren Salzes einer solchen Verbindung mit mindestens einer salzbildenden Gruppe gemäss einem der Ansprüche 1 bis 23 zur Herstellung pharmazeutischer Präparate gegen solche Erkrankungen, die auf eine Hemmung von Protein-Tyrosinkinasen ansprechen.

27. Verfahren zur Herstellung einer Verbindung der Formel I gemäss Anspruch 1, dadurch gekennzeichnet, dass man
a) eine Carbonsäure der Formel II, 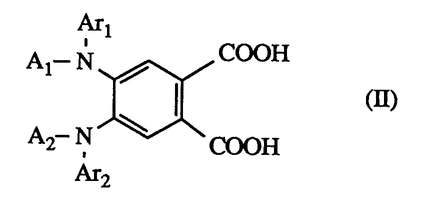 worin Ar₁, Ar₂, A₁ und A₂ die unter Formel I angegebene Bedeutung haben, oder ein reaktionsfähiges Säurederivat davon mit einer Aminoverbindung der Formel III,
H₂N-R (III)
worin R die unter Formel I angegebene Bedeutung hat, umsetzt, wobei in den Ausgangsmaterialien funktionelle Gruppen, die nicht an der Reaktion teilnehmen sollen, erforderlichenfalls in geschützter Form vorliegen, und vorhandene Schutzgruppen abspaltet, oder
b) zur Herstellung von Verbindungen der Formel I, worin A₁ und A₂ zusammen durch verestertes Carboxy oder Niederalkanoyl substituiertes Methylen bedeuten, eine Verbindung der Formel XI, 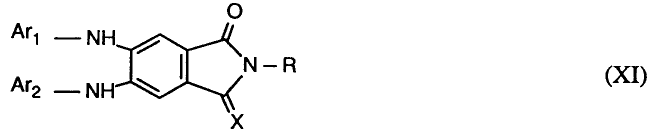 worin Ar₁, Ar₂, X und R die für Verbindungen der Formel I genannten Bedeutungen haben, mit einem Aldehyd der Formel XII,
R₀-CHO (XII)
worin R₀ verestertes Carboxy oder Niederalkanoyl bedeutet, oder einem reaktionsfähigen Derivat davon, umsetzt, wobei in den Ausgangsmaterialien funktionelle Gruppen, die nicht an der Reaktion teilnehmen sollen, erforderlichenfalls in geschützter Form vorliegen, und vorhandene Schutzgruppen abspaltet,
und gewünschtenfalls eine erhältliche Verbindung der Formel I in eine andere Verbindung der Formel I umwandelt, und/oder ein erhältliches Salz in die freie Verbindung oder in ein anderes Salz umwandelt, und/oder eine erhältliche freie Verbindung der Formel I in ein Salz umwandelt und/oder ein erhältliches Gemisch von isomeren Verbindungen der Formel I in die einzelnen Isomeren auftrennt.

28. Eine Verbindung der Formel II, 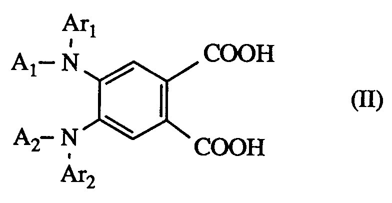 worin A₁ und A₂ unabhängig voneinander substituiertes Niederalkyl, substituiertes Niederalkenyl, substituiertes Niederalkinyl oder Wasserstoff bedeuten, wobei maximal einer der Reste A₁ und A₂ Wasserstoff bedeuten kann, oder A₁ und A₂ zusammen durch Substituenten ausser Niederalkyl und ausser Hydroxy substituiertes Niederalkylen bedeuten; und Ar₁ und Ar₂ unabhängig voneinander Aryl, Heteroaryl oder unsubstituiertes oder substituiertes Cycloalkyl bedeuten, Salze davon, sofern salzbildende Gruppen vorliegen, und Tautomere davon, sofern tautomerisierbare Gruppen vorliegen.

29. Eine Verbindung der Formel II', 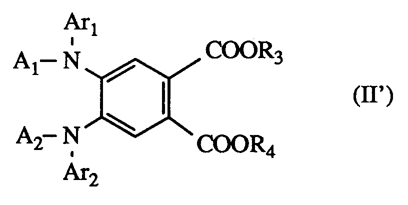 worin A₁ und A₂ unabhängig voneinander substituiertes Niederalkyl, substituiertes Niederalkenyl, substituiertes Niederalkinyl oder Wasserstoff bedeuten, wobei maximal einer der Reste A₁ und A₂ Wasserstoff bedeuten kann, oder A₁ und A₂ zusammen durch Substituenten ausser Niederalkyl und ausser Hydroxy substituiertes Niederalkylen bedeuten; Ar₁ und Ar₂ unabhängig voneinander Aryl, Heteroaryl oder unsubstituiertes oder substituiertes Cycloalkyl bedeuten und R₃ und R₄ unabhängig voneinander Aryl, Arylniederalkyl oder Niederalkyl bedeuten, Salze davon, sofern salzbildende Gruppen vorliegen, und Tautomere davon, sofern tautomerisierbare Gruppen vorliegen
